# EUROPEAN PATENT APPLICATION

(11) **EP 4 692 103 A1**
(43) Date of publication of application: **11.02.2026**
(21) Application number: 24803042.1
(22) Date of filing: 09.05.2024
(51) Int. Cl.: C07H 15/04, A61K 47/54, A61K 39/29, A61K 31/713, A61P 31/14, A61P 31/20, C12N 15/113

(54) **NOVEL TARGETING COMPOUND AND USE THEREOF, AND NUCLEIC ACID CONJUGATE AND USE THEREOF**

(30) Priority: 11.05.2023 CN 202310534961
(71) Applicant: Sunshine Lake Pharma Co., Ltd., Dongguan, Guangdong 523000 (CN)
(72) Inventor: REN, Qingyun, Dongguan, Guangdong 523871 (CN); ZOU, Zhifu, Dongguan, Guangdong 523871 (CN); CHEN, Yunfu, Dongguan, Guangdong 523871 (CN); CHEN, Huimeng, Dongguan, Guangdong 523871 (CN)
(74) Representative: Becker Kurig & Partner Patentanwälte mbB
(86) International application number: PCT/CN2024/091851
(87) International publication number: WO 2024/230763

(57) **Abstract**

The present invention provids a novel targeting compound and nucleic acid conjugate and their uses. In addition, the present invention also relates to a novel conjugated group, which is used for the delivery of small nucleic acid drugs, the conjugated group can be conjugated with nucleotides to form a nucleotide conjugate, wherein the nucleotide conjugate is used to regulate gene expression in hepatocytes to prevent and/or treat related diseases.

## Description

This application claims the priority and benefits of Chinese Patent Application No. 202310534961.X, filed with the State Intellectual Property Office of China on May 11, 2023, which is incorporated herein by reference in its entirety.

### FIELD OF THE INVENTION

The present invention belongs to the field of drug delivery. The purpose of the present invention is to provide a novel targeting compound and nucleic acid conjugate and their uses. In addition, the present invention also relates to a novel conjugated group, which is used for the delivery of small nucleic acid drugs (such as siRNA, ASO, etc.). The conjugated group can be conjugated with nucleotides to form a nucleic acid conjugate. The nucleotide conjugate is used to regulate gene expression in hepatocytes to prevent and/or treat related diseases.

### BACKGROUND OF THE INVENTION

RNA interference (RNAi) is a phenomenon in which double-stranded RNA (dsRNA) molecules shut down or silence the expression of homologous genes at the mRNA level. RNA interference, also known as gene knockdown or gene silencing, is a typical post-transcriptional gene regulation method, also known as post-transcriptional gene silencing (PTGS). The earliest reports of RNA interference appeared in 1990, when two different research groups simultaneously reported RNA interference in transgenic plants. Subsequently, RNA interference was also observed in nearly all eukaryotic organisms, including nematodes, fruit flies, zebrafish, and mice. In 1999, Hamilton and Baulcombe detected RNA fragments of 21-25 nucleotides in length in plants that exhibited RNA interference. These RNA fragments were shown to be essential for RNA interference and were termed small interfering nucleic acids (siRNA). Double-stranded siRNA forms an RNA-induced silencing complex (RISC) together with relevant cell-derived enzymes and proteins. During RNA interference (RNAi), the sense strand of the double-stranded siRNA is excluded from the complex, while the antisense strand guides RISC to bind to the cognate site of the target mRNA. RNase III in the complex then degrades the target mRNA, thereby shutting down target gene expression.

Effective delivery of RNAi agents to cells *in vivo* requires specific targeting and substantial protection from the extracellular environment (particularly serum proteins). One approach to achievespecific targeting is conjugating (also called conjugation) a targeting moiety to the RNAi agent. The targeting moiety helps direct the RNAi agent to the desired target site. One way targeting moieties can improve delivery is through receptor-mediated endocytosis. This mechanism of uptake involves the movement of the RNAi agent bound to a membrane receptor into the interior of a region, either through invagination of the membrane structure or through fusion of the delivery system with the cell membrane surrounding the region. This process is initiated by activation of the cell surface or membrane receptor following binding of a specific ligand to the receptor.

In recent years, the use of N-acetylgalactosamine (GalNAc), a high-affinity ligand for the asialoglycoprotein receptor (ASGPR), as a targeting molecule has achieved promising results in liver-targeted delivery of nucleic acid drugs. Patent application WO2009073809A2 discloses oligonucleotide conjugates containing three clusters of N-acetylgalactosamine (GalNAc). The GalNAc-containing fragment is linked to the oligonucleotide via 3-hydroxy-5-hydroxymethylpyrrolidine. These conjugates have the ability to inhibit the expression of corresponding genes in cells.

### SUMMARY OF THE INVENTION

The present invention provides a novel targeting compound that can be used to prepare a nucleic acid conjugate drug for targeted delivery of oligonucleotides to the liver. The present invention also provides a novel targeting conjugated group (i.e., carrier) that can effectively deliver oligonucleotides to liver cells. The present invention also provides a nucleotide conjugate, a method for its preparation, and its applications. The nucleotide conjugate exhibits high *in vivo* delivery efficiency, excellent stability, high gene expression inhibition activity in liver cells, and/or low toxicity. In another aspect, the present invention further provides an siRNA agent, a method for its preparation, and its applications. This siRNA agent exhibits high *in vivo* delivery efficiency, good stability, high gene expression inhibition activity against HBV, and/or low toxicity.

In one aspect, the present invention relates to a compound having a structure as shown in Formula (I), or a stereoisomer, tautomer, or acceptable salt of the compound of Formula (I). 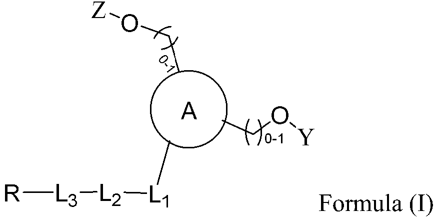 wherein Ring A is a heterocyclic group consisting of 3-12 ring atoms or a cycloalkyl group consisting of 3-12 ring atoms;
Z and Y are each independently H, deuterium, a hydroxyl protecting group, HOC(=O)(CH₂) C(=O)-or M-C(=O)(CH₂)ⱼC(=O)-;
M is a solid support, preferably a hydroxyl- or amino-functionalized solid support, more preferably a resin or CPG, further preferably a macroporous resin or CPG, and even more preferably an aminomethyl resin, hydroxyl resin, or-NHCPG;
each of L₁, L₂ and L₃ is independently C₁₋₁₂ alkylene, C₃₋₁₂ cycloalkyl, heterocyclic group consisting of 3-12 ring atoms, C₁₋₁₂ heteroalkyl, C₂₋₁₂ alkenyl, C₂₋₁₂ alkynyl, -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of the C₁₋₁₂ alkylene, C₃₋₁₂ cycloalkyl, heterocyclyl consisting of 3-12 ring atoms, C₁₋₁₂ heteroalkyl, C₂₋₁₂ alkenyl and C₂₋₁₂ alkynyl groups is independently optionally substituted by 1, 2, 3 or 4 R^{W1}, and 1, 2, 3, 4 or 5 methylene groups in each of the C₁₋₁₂ alkylene, C₁₋₁₂ heteroalkyl, C₂₋₁₂ alkenyl and C₂₋₁₂ alkynyl groups is independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-;
R is a structure represented by the following Formula (a) or Formula (b): 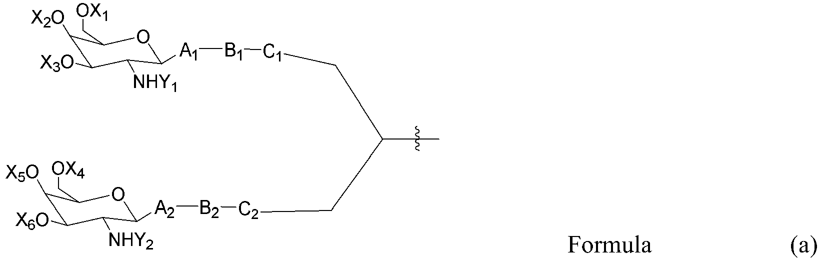 or 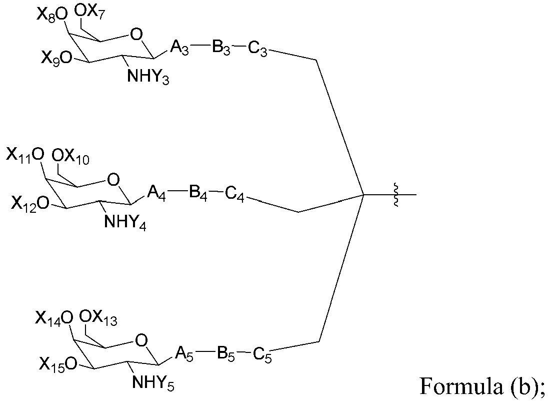
wherein each of X₁, X₂, X₃, X₄, X₅, X₆, X₇, X₈, X₉, X₁₀, X₁₁, X₁₂, X₁₃, X₁₄ and X₁₅ is independently H or a hydroxyl protecting group; each of Y₁, Y₂, Y₃, Y₄ and Y₅ is independently H or an amino protecting group;
each of A₁, A₂, A₃, A₄, A₅, C₁, C₂, C₃, C₄ and C₅ is independently a bond, C₁₋₁₂ alkylene, C₃₋₁₂ cycloalkyl, a heterocyclyl consisting of 3-12 ring atoms, C₁₋₁₂ heteroalkyl, C₂₋₁₂ alkenyl or C₂₋₁₂ alkynyl, wherein each of the C₁₋₁₂ alkylene, C₃₋₁₂ cycloalkyl, heterocyclyl consisting of 3-12 ring atoms, C₁₋₁₂ heteroalkyl, C₂₋₁₂ alkenyl and C₂₋₁₂ alkynyl groups is independently optionally substituted by 1, 2, 3 or 4 R^{W2}, and 1, 2, 3, 4 or 5 methylene groups in each of the C₁₋₁₂ alkylene, C₁₋₁₂ heteroalkyl, C₂₋₁₂ alkenyl and C₂₋₁₂ alkynyl groups are each independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-;
each of B₁, B₂, B₃, B₄ and B₅ is independently a C₃₋₁₂ cycloalkyl, a heterocyclyl consisting of 3-12 ring atoms, a C₁₋₁₂ alkylene, a C₁₋₁₂ heteroalkyl, a C₂₋₁₂ alkenyl or a C₂₋₁₂ alkynyl, wherein at least one of B₁ and B₂ is a C₃₋₁₂ cycloalkyl or a heterocyclyl consisting of 3-12 ring atoms, and at least one of B₃, B₄ and B₅ is a C₃₋₁₂ cycloalkyl or a heterocyclyl consisting of 3-12 ring atoms, each of the C₃₋₁₂ cycloalkyl, heterocyclyl consisting of 3-12 atoms, C₁₋₁₂ alkylene, C₁₋₁₂ heteroalkyl, C₂₋₁₂ alkenyl and C₂₋₁₂ alkynyl groups is independently optionally substituted by 1, 2, 3 or 4 R^{W3}, and 1, 2, 3, 4 or 5 methylene groups in each of the C₁₋₁₂ alkylene, C₁₋₁₂ heteroalkyl, C₂₋₁₂ alkenyl and C₂₋₁₂ alkynyl groups are independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-;
each R¹ is independently H, deuterium or C₁₋₆ alkyl;
each j is independently 1, 2, 3, 4 or 5;
each of R^{W1}, R^{W2} and R^{W3} is independently deuterium, =O, hydroxy, nitro, cyano, F, Cl, Br, I, C₁₋₆ alkyl, C₁₋₆ alkoxy, C₃₋₆ cycloalkyl, C₂₋₆ alkenyl or C₁₋₆ heteroalkyl.

In some embodiments of the compounds of the present invention, each of L₁, L₂ and L₃ is independently C₃₋₁₂ alkylene, C₃₋₆ cycloalkyl, a heterocyclic group consisting of 3-6 ring atoms, C₂₋₁₀ heteroalkyl, C₂₋₁₀ alkenyl, C₂₋₁₀ alkynyl, -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of the C₃₋₁₂ alkylene, C₃₋₆ cycloalkyl, heterocyclyl consisting of 3-6 ring atoms, C₂₋₁₀ heteroalkyl, C₂₋₁₀ alkenyl and C₂₋₁₀ alkynyl groups is independently optionally substituted by 1, 2, 3 or 4 R^{W1}, and 1, 2, 3, 4 or 5 methylene groups in each of the C₃₋₁₂ alkylene, C₂₋₁₀ heteroalkyl, C₂₋₁₀ alkenyl and C₂₋₁₀ alkynyl groups are independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each of R¹ and R^{W1} has the meanings described in the present invention.

In some embodiments of the compounds of the present invention, each of A₁, A₂, A₃, A₄, A₅, C₁, C₂, C₃, C₄ and C₅ is independently a bond, C₃₋₁₂ alkylene, C₃₋₆ cycloalkyl, a heterocyclic group consisting of 3-6 ring atoms, C₂₋₁₀ heteroalkyl, C₂₋₁₀ alkenyl and C₂₋₁₀ alkynyl, wherein each of the C₃₋₁₂ alkylene, C₃₋₆ cycloalkyl, heterocyclyl consisting of 3-6 ring atoms, C₂₋₁₀ heteroalkyl, C₂₋₁₀ alkenyl and C₂₋₁₀ alkynyl groups is independently optionally substituted by 1, 2, 3 or 4 R^{W2}, and 1, 2, 3, 4 or 5 methylene groups in each of the C₃₋₁₂ alkylene, C₂₋₁₀ heteroalkyl, C₂₋₁₀ alkenyl and C₂₋₁₀ alkynyl groups are each independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each of R¹ and R^{W2} has the meanings described in the present invention.

In some embodiments of the compounds of the present invention, each of B₁, B₂, B₃, B₄ and B₅ is independently cyclopropyl, cyclobutyl, cyclopentyl, cyclohexyl, a heterocyclic group consisting of 3 to 6 ring atoms, C₁₋₆ alkylene, C₁₋₆ heteroalkyl, C₂₋₆ alkenyl or C₂₋₆ alkynyl, wherein at least one of B₁ and B₂ is a cyclopropyl, cyclobutyl, cyclopentyl, cyclohexyl or a heterocyclic group consisting of 3-6 ring atoms, at least one of B₃, B₄ and B₅ is a cyclopropyl, cyclobutyl, cyclopentyl, cyclohexyl or a heterocyclic group consisting of 3-6 ring atoms, each of the cyclopropyl, cyclobutyl, cyclopentyl, cyclohexyl, a heterocyclic group consisting of 3-6 ring atoms, C₁₋₆ alkylene, C₁₋₆ heteroalkyl, C₂₋₆ alkenyl and C₂₋₆ alkynyl groups is independently optionally substituted with 1, 2, 3 or 4 R^{W3}, and 1, 2, 3, 4 or 5 methylene groups in each of the C₁₋₆ alkylene, C₁₋₆ heteroalkyl, C₂₋₆ alkenyl and C₂₋₆ alkynyl groups are each independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each of R¹ and R^{W3} has the meanings described in the present invention.

In some embodiments of the compounds of the present invention, Z and Y are each independently H, deuterium, a hydroxyl protecting group, HOC(=O)(CH₂)ⱼC(=O)- or M-C(=O)(CH₂)ⱼC(=O)-, wherein the hydroxyl protecting group is an ester protecting group, an alkoxyalkyl protecting group, a silyl protecting group, a substituted or unsubstituted aryl protecting group, a substituted or unsubstituted arylalkyl protecting group (such as triphenylmethyl, MMTr, DMTr or 4',4',4'-trimethoxytrityl, etc.), wherein each of M and j has the meaning described in the present invention.

In some embodiments of the compounds of the present invention, Z and Y are each independently H, deuterium, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, p-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methylsulfonyl, p-toluenesulfonyl, C₁₋₁₂ alkyl, C₁₋₁₂ alkylC(=O)-, C₁₋₁₂ alkylmethyl, C₁₋₁₂ alkylsilyl, C₆₋₁₀ arylC₁₋₆ alkyl, HOC(=O)(CH₂)ⱼC(=O)-, M-C(=O)(CH₂)ⱼC(=O)-, triphenylmethyl, MMTr, DMTr or 4',4',4'-trimethoxytrityl, wherein each of M and j has the meaning described in the present invention;
the ring A is a heterocyclic group consisting of 3-7 ring atoms or a cycloalkyl group consisting of 3-6 ring atoms, more preferably a cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl.

In some embodiments of the compounds of the present invention, Z and Y are each independently H, deuterium, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, p-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methylsulfonyl, p-toluenesulfonyl, C₁₋₁₀ alkyl, C₁₋₁₀ alkylC(=O)-, C₁₋₁₀ alkylmethyl, C₁₋₁₀ alkylsilyl, C₆₋₁₀ arylC₁₋₄ alkyl, HOC(=O)(CH₂)ⱼC(=O)-, M-C(=O)(CH₂)ⱼC(=O)-, triphenylmethyl, MMTr, DMTr or 4',4',4'-trimethoxytrityl, wherein each of M and j has the meaning as defined herein;
the ring A is a heterocyclic group consisting of 3-7 ring atoms or a cycloalkyl group consisting of 3-6 ring atoms, more preferably a cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl.

In some embodiments of the compounds of the present invention, Z and Y are each independently H, deuterium, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, p-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methylsulfonyl, p-toluenesulfonyl, C₁₋₈ alkyl, C₁₋₈ alkylC(=O)-, C₁₋₈ alkylmethyl, C₁₋₈ alkylsilyl, phenyl C₁₋₃ alkyl, HOC(=O)(CH₂)ⱼC(=O)-, M-C(=O)(CH₂)ⱼC(=O)-, triphenylmethyl, MMTr, DMTr or 4',4',4'-trimethoxytrityl, wherein each of M and j has the meaning as defined herein;
the ring A is a heterocyclic group consisting of 3-7 ring atoms or a cycloalkyl group consisting of 3-6 ring atoms, more preferably a cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl.

In some embodiments of the compounds of the present invention, Z and Y are each independently H, deuterium, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, p-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methylsulfonyl, p-toluenesulfonyl, C₁₋₆ alkyl, C₁₋₆ alkylC(=O)-, C₁₋₆ alkylmethyl, C₁₋₆ alkylsilyl, benzyl, phenethyl, HOC(=O)(CH₂)ⱼC(=O)-, M-C(=O)(CH₂)ⱼC(=O)-, triphenylmethyl, MMTr, DMTr or 4',4',4'-trimethoxytrityl, wherein each of M and j has the meaning as defined herein;
the ring A is a heterocyclic group consisting of 3-7 ring atoms or a cycloalkyl group consisting of 3-6 ring atoms, more preferably a cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl.

In some embodiments of the compounds of the present invention, each of X₁, X₂, X₃, X₄, X₅, X₆, X₇, X₈, X₉, X₁₀, X₁₁, X₁₂, X₁₃, X₁₄ and X₁₅ is independently H, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, methyl, tert-butyl, 2-trimethylsilylethyl, 1,1-dimethyl-2-propenyl, 3-methyl-3-butenyl, allyl, benzyl, p-methoxybenzyldiphenylmethyl, trityl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, p-toluenesulfonyl, trimethylsilyl, triethylsilyl, triisopropylsilyl, C₁₋₆ alkoxy or R²C(=O)-;
each of Y₁, Y₂, Y₃, Y₄, and Y₅ is independently H, 9-fluorenylmethoxycarbonyl or R³C(=O)-;
wherein, each of R² and R³ is independently C₁₋₆ alkyl, halogenated C₁₋₆ alkyl, C₁₋₆ alkoxy, phenyl, halogen-substituted phenyl, C₁₋₆ alkylphenyl or benzyl;
each R¹ is independently H, deuterium or C₁₋₄ alkyl;
each of R^{W1}, R^{W2} and R^{W3} is independently deuterium, =O, hydroxy, nitro, cyano, F, Cl, Br, I, methyl, ethyl, n-propyl, isopropyl, n-butyl, isobutyl, methoxy, ethoxy, 1-propoxy, 2-propoxy, 1-butoxy, 2-methyl-1-propoxy, 2-butoxy, cyclopropyl, cyclobutyl, cyclopentyl, cyclohexyl, C₂₋₄ alkenyl or C₁₋₄ heteroalkyl.

In some embodiments of the compounds of the present invention, each of X₁, X₂, X₃, X₄, X₅, X₆, X₇, X₈, X₉, X₁₀, X₁₁, X₁₂, X₁₃, X₁₄ and X₁₅ is independently H, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, methyl, tert-butyl, 2-trimethylsilylethyl, 1,1-dimethyl-2-propenyl, 3-methyl-3-butenyl, allyl, benzyl, p-methoxybenzyldiphenylmethyl, trityl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, p-toluenesulfonyl, trimethylsilyl, triethylsilyl, triisopropylsilyl, C₁₋₆ alkoxy or R²C(=O)-;
each of Y₁, Y₂, Y₃, Y₄, and Y₅ is independently H, 9-fluorenylmethoxycarbonyl or R³C(=O)-;
wherein, each of R² and R³ is independently C₁₋₄ alkyl, halogenated C₁₋₆ alkyl, C₁₋₄ alkoxy, phenyl, halogen-substituted phenyl, C₁₋₄ alkylphenyl, or benzyl;
each R¹ is independently H, deuterium or C₁₋₄ alkyl;
each of R^{W1}, R^{W2} and R^{W3} is independently deuterium, =O, hydroxy, nitro, cyano, F, Cl, Br, I, methyl, ethyl, n-propyl, isopropyl, n-butyl, isobutyl, methoxy, ethoxy, 1-propoxy, 2-propoxy, 1-butoxy, 2-methyl-1-propoxy, 2-butoxy, tert-butoxy, cyclopropyl, cyclobutyl, cyclopentyl, cyclohexyl, C₂₋₄ alkenyl or C₁₋₄ heteroalkyl.

In some embodiments of the compounds of the present invention, each of X₁, X₂, X₃, X₄, X₅, X₆, X₇, X₈, X₉, X₁₀, X₁₁, X₁₂, X₁₃, X₁₄ and X₁₅ is independently H, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, methyl, tert-butyl, 2-trimethylsilylethyl, 1,1-dimethyl-2-propenyl, 3-methyl-3-butenyl, allyl, benzyl, p-methoxybenzyldiphenylmethyl, trityl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, p-toluenesulfonyl, trimethylsilyl, triethylsilyl, triisopropylsilyl, C₁₋₆ alkoxy or R²C(=O)-;
each of Y₁, Y₂, Y₃, Y₄ and Y₅ is independently H, 9-fluorenylmethoxycarbonyl or R³C(=O)-;
wherein each of R² and R³ is independently methyl, ethyl, n-propyl, isopropyl, trifluoromethyl, difluoromethyl, monofluoromethyl, trichloromethyl, dichloromethyl, monochloromethyl, 2,2,2-trichloroethyl, methoxy, ethoxy, 1-propoxy, 2-propoxy, 1-butoxy, 2-methyl-1-propoxy, 2-butoxy, tert-butoxy, phenyl, halogen-substituted phenyl, C₁₋₃ alkylphenyl or benzyl;
each R¹ is independently H, deuterium or C₁₋₄ alkyl;
each of R^{W1}, R^{W2} and R^{W3} is independently deuterium, =O, hydroxy, nitro, cyano, F, Cl, Br, I, methyl, ethyl, n-propyl, isopropyl, n-butyl, isobutyl, methoxy, ethoxy, 1-propoxy, 2-propoxy, 1-butoxy, 2-methyl-1-propoxy, 2-butoxy, cyclopropyl, cyclobutyl, cyclopentyl, cyclohexyl, C₁₋₄ alkenyl or C₁₋₄ heteroalkyl.

In some embodiments of the compounds of the present invention, R is a structure represented by the following Formula (a-1) or Formula (b-1): 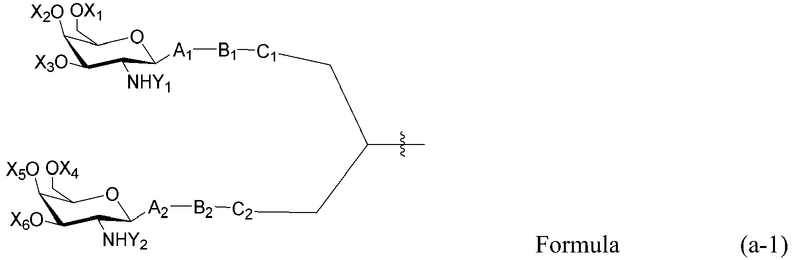 or 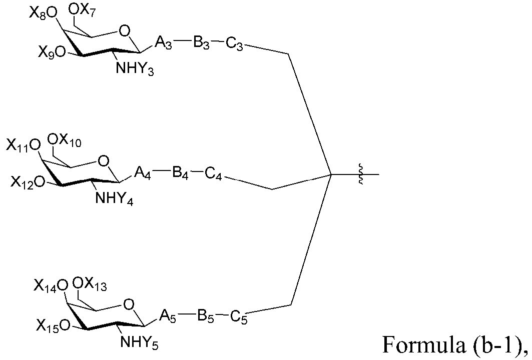 wherein each of X₁, X₂, X₃, X₄, X₅, X₆, X₇, X₈, X₉, X₁₀, X₁₁, X₁₂, X₁₃, X₁₄, X₁₅, Y₁, Y₂, Y₃, Y₄, Y₅, A₁, A₂, A₃, A₄, A₅, B₁, B₂, B₃, B₄, B₅, C₁, C₂, C₃, C₄ and C₅ has the meaning as defined in the present invention.

In some embodiments, the present invention provides a compound having the structure represented by Formula (II), or a stereoisomer, tautomer, or acceptable salt of the compound represented by Formula (II): 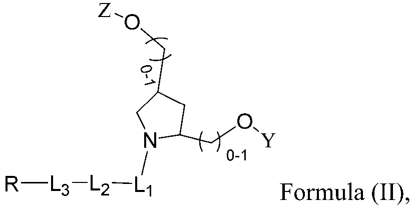 wherein each of Z, Y, L₁, L₂, L₃ and R has the meaning as defined in the present invention.

In some embodiments, the present invention provides a compound having the structure represented by Formula (III), or a stereoisomer, tautomer, or acceptable salt of the compound represented by Formula (III): 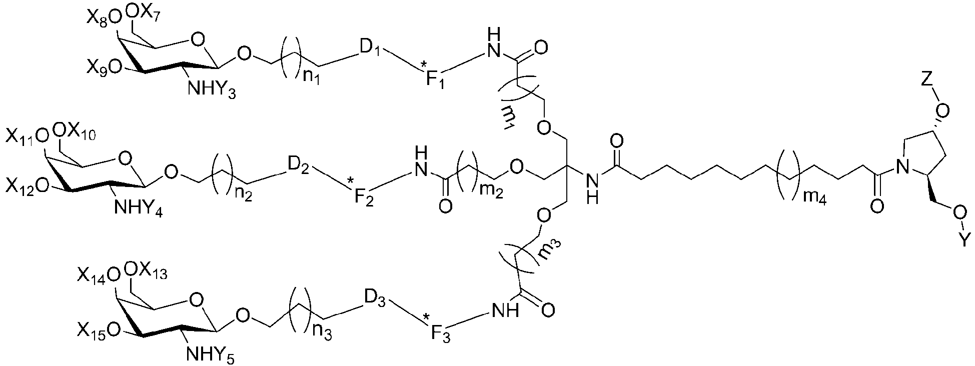
Formula (III) wherein, each of D₁, D₂ and D₃ is independently -C(=O)-*, -C(=O)O-*, -OC(=O)-*, -C(=O)N(R¹)-*, -N(R¹)C(=O)-*, -C(=O)N(R¹)CH₂-* or -N(R¹)C(=O)CH₂-*;
each of F₁, F₂ and F₃ is independently cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl;
each of n₁, n₂, n₃, m₁, m₂, m₃ and m₄ is independently 0, 1, 2, 3 or 4;
wherein each of R¹, X₇, X₈, X₉, X₁₀, X₁₁, X₁₂, X₁₃, X₁₄, X₁₅, Y₃, Y₄, Y₅, Z and Y has the meaning as defined in the present invention.

In another aspect, the present invention relates to a conjugated group comprising a structure represented by Formula (I-a), or a stereoisomer, tautomer, or acceptable salt of the structure represented by Formula (I-a), 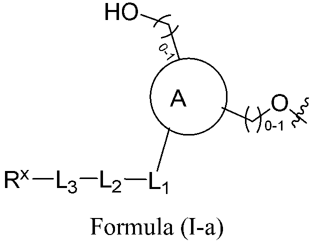 wherein, R^{x} is a structure represented by Formula (c) or Formula (d): 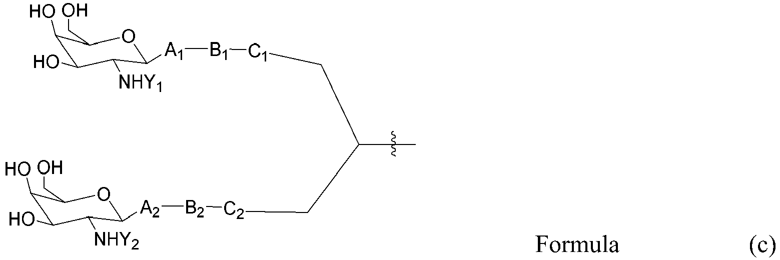 or 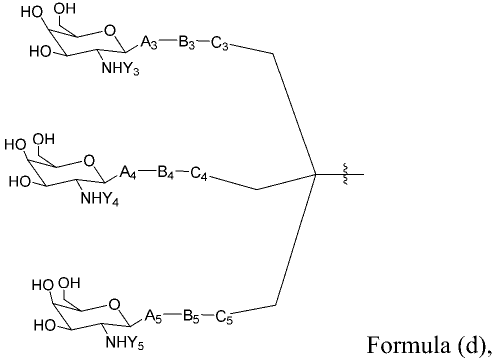 wherein Ring A is a heterocyclic group consisting of 3-12 atoms or a cycloalkyl group consisting of 3-12 atoms;
each of L₁, L₂ and L₃ is independently C₁₋₁₂ alkylene, C₃₋₁₂ cycloalkyl, heterocyclyl consisting of 3-12 ring atoms, C₁₋₁₂ heteroalkyl, C₂₋₁₂ alkenyl, C₂₋₁₂ alkynyl, -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of the C₁₋₁₂ alkylene, C₃₋₁₂ cycloalkyl, heterocyclyl consisting of 3-12 ring atoms, C₁₋₁₂ heteroalkyl, C₂₋₁₂ alkenyl and C₂₋₁₂ alkynyl groups is independently optionally substituted with 1, 2, 3 or 4 R^{W1}, and 1, 2, 3, 4 or 5 methylene groups in each of the C₁₋₁₂ alkylene, C₁₋₁₂ heteroalkyl, C₂₋₁₂ alkenyl and C₂₋₁₂ alkynyl groups is independently optionally replaced with 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-;
each of A₁, A₂, A₃, A₄, A₅, C₁, C₂, C₃, C₄ and C₅ is independently a bond, C₁₋₁₂ alkylene, C₃₋₁₂ cycloalkyl, a heterocyclyl consisting of 3-12 ring atoms, C₁₋₁₂ heteroalkyl, C₂₋₁₂ alkenyl or C₂₋₁₂ alkynyl, wherein each of the C₁₋₁₂ alkylene, C₃₋₁₂ cycloalkyl, a heterocyclyl consisting of 3-12 ring atoms, C₁₋₁₂ heteroalkyl, C₂₋₁₂ alkenyl and C₂₋₁₂ alkynyl is independently optionally substituted with 1, 2, 3 or 4 R^{W2}, and 1, 2, 3, 4 or 5 methylene groups in each of the C₁₋₁₂ alkylene, C₁₋₁₂ heteroalkyl, C₂₋₁₂ alkenyl and C₂₋₁₂ alkynyl are independently optionally replaced with 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-;
each of B₁, B₂, B₃, B₄ and B₅ is independently a C₃₋₁₂ cycloalkyl, a heterocyclyl consisting of 3-12 ring atoms, a C₁₋₁₂ alkylene, a C₁₋₁₂ heteroalkyl, a C₂₋₁₂ alkenyl or a C₂₋₁₂ alkynyl, wherein at least one of B₁ and B₂ is a C₃₋₁₂ cycloalkyl or a heterocyclyl consisting of 3-12 ring atoms, and at least one of B₃, B₄ and B₅ is a C₃₋₁₂ cycloalkyl or a heterocyclyl consisting of 3-12 ring atoms, each of C₃₋₁₂ cycloalkyl, heterocyclyl consisting of 3-12 atoms, C₁₋₁₂ alkylene, C₁₋₁₂ heteroalkyl, C₂₋₁₂ alkenyl and C₂₋₁₂ alkynyl is independently optionally substituted with 1, 2, 3 or 4 R^{W3}, and 1, 2, 3, 4 or 5 methylene groups in each of the C₁₋₁₂ alkylene, C₁₋₁₂ heteroalkyl, C₂₋₁₂ alkenyl and C₂₋₁₂ alkynyl groups are independently optionally replaced with 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-;
each R¹ is independently H, deuterium, or C₁₋₆ alkyl;
each of R^{W1}, R^{W2} and R^{W3} is independently deuterium, =O, hydroxy, nitro, cyano, F, Cl, Br, I, C₁₋₆ alkyl, C₁₋₆ alkoxy, C₃₋₆ cycloalkyl, C₂₋₆ alkenyl or C₁₋₆ heteroalkyl.

In some embodiments of the conjugated groups of the present invention, each of L₁, L₂ and L₃ is independently C₃₋₁₂ alkylene, C₃₋₆ cycloalkyl, a heterocyclyl consisting of 3-6 ring atoms, C₂₋₁₀ heteroalkyl, C₂₋₁₀ alkenyl, C₂₋₁₀ alkynyl, -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of the C₃₋₁₂ alkylene, C₃₋₆ cycloalkyl, a heterocyclyl consisting of 3-6 ring atoms, C₂₋₁₀ heteroalkyl, C₂₋₁₀ alkenyl and C₂₋₁₀ alkynyl is independently optionally substituted with 1, 2, 3 or 4 R^{W1}, and 1, 2, 3, 4 or 5 methylene groups in each of the C₃₋₁₂ alkylene, C₂₋₁₀ heteroalkyl, C₂₋₁₀ alkenyl and C₂₋₁₀ alkynyl are independently optionally is replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each of R¹ and R^{W1} has the meanings described herein.

In some embodiments of the conjugate groups of the present invention, each of A₁, A₂, A₃, A₄, A₅, C₁, C₂, C₃, C₄ and C₅ is independently a bond, a C₃₋₁₂ alkylene, a C₃₋₆ cycloalkyl, a heterocyclyl consisting of 3-6 ring atoms, a C₂₋₁₀ heteroalkyl, a C₂₋₁₀ alkenyl or a C₂₋₁₀ alkynyl, wherein each of C₃₋₁₂ alkylene, a C₃₋₆ cycloalkyl, a heterocyclyl consisting of 3-6 ring atoms, C₂₋₁₀ heteroalkyl, C₂₋₁₀ alkenyl and C₂₋₁₀ alkynyl is independently optionally substituted with 1, 2, 3 or 4 R^{W2}, and 1, 2, 3, 4 or 5 methylene groups in each of the C₃₋₁₂ alkylene, C₂₋₁₀ heteroalkyl, C₂₋₁₀ alkenyl and C₂₋₁₀ alkynyl groups are each independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each of R¹ and R^{W2} has the meanings described in the present invention.

In some embodiments of the conjugate groups of the present invention, each of B₁, B₂, B₃, B₄ and B₅ is independently a cyclopropyl, cyclobutyl, cyclopentyl, cyclohexyl, a heterocyclyl consisting of 3-6 ring atoms, a C₁₋₆ alkylene, a C₁₋₆ heteroalkyl, a C₂₋₆ alkenyl or a C₂₋₆ alkynyl group, wherein at least one of B₁ and B₂ is a cyclopropyl, cyclobutyl, cyclopentyl, cyclohexyl or a heterocyclyl consisting of 3-6 ring atoms, and at least one of B₃, B₄ and B₅ is a cyclopropyl, cyclobutyl, cyclopentyl, cyclohexyl or a heterocyclyl consisting of 3-6 ring atoms, and each of the cyclopropyl, cyclobutyl, cyclopentyl, cyclohexyl, a heterocyclyl consisting of 3-6 ring atoms, a C₁₋₆ alkylene, a C₁₋₆ heteroalkyl, a C₂₋₆ alkenyl and a C₂₋₆ alkynyl group is independently optionally substituted with 1, 2, 3 or 4 R^{W3}, and 1, 2, 3, 4 or 5 methylene groups in each of the C₁₋₆ alkylene, C₁₋₆ heteroalkyl, C₂₋₆ alkenyl and C₂₋₆ alkynyl are each independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each of R¹ and R^{W3} has the meaning described in the present invention.

In some embodiments of the conjugated groups of the present invention, Ring A is a heterocyclyl consisting of 3-7 ring atoms or a cycloalkyl consisting of 3-6 ring atoms, more preferably a cyclopentyl, pyrrolyl, furanyl or piperidinyl.

In some embodiments of the conjugated groups of the present invention, Ring A is a heterocyclyl consisting of 3-7 ring atoms or a cycloalkyl consisting of 3-6 ring atoms, more preferably a cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl. In some embodiments of the conjugated groups of the present invention, each R¹ is independently H, deuterium or C₁₋₄ alkyl;
each of R^{w1}, R^{w2} and R^{w3} is independently deuterium, =O, hydroxy, nitro, cyano, F, Cl, Br, I, methyl, ethyl, n-propyl, isopropyl, n-butyl, isobutyl, methoxy, ethoxy, 1-propoxy, 2-propoxy, 1-butoxy, 2-methyl-1-propoxy, 2-butoxy, cyclopropyl, cyclobutyl, cyclopentyl, cyclohexyl, C₂₋₄ alkenyl or C₁₋₄ heteroalkyl.

In some embodiments of the conjugated groups of the present invention, R^{x} is a structure represented by Formula (c-1) or Formula (d-1): 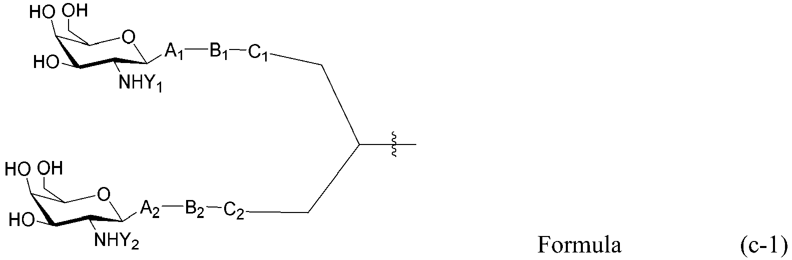 or 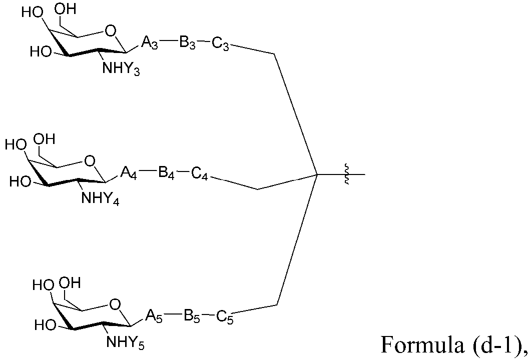 wherein each of A₁, A₂, A₃, A₄, A₅, B₁, B₂, B₃, B₄, B₅, C₁, C₂, C₃, C₄, C₅, Y₁, Y₂, Y₃, Y₄ and Y₅ has the meaning described in the present invention.

In some embodiments of the conjugated groups of the present invention, wherein the conjugated group has a structure represented by Formula (II-a), or a stereoisomer, tautomer, or acceptable salt of the structure represented by Formula (II-a), 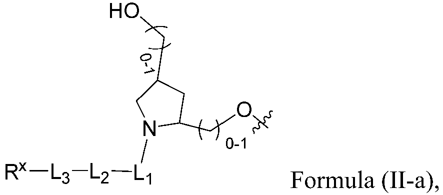 wherein each of R^{x}, L₁, L₂ and L₃ has the meaning described in the present invention.

In some embodiments of the conjugated groups of the present invention, wherein the conjugated group has a structure represented by Formula (III-a), or a stereoisomer, tautomer, or acceptable salt of the structure represented by Formula (III-a), 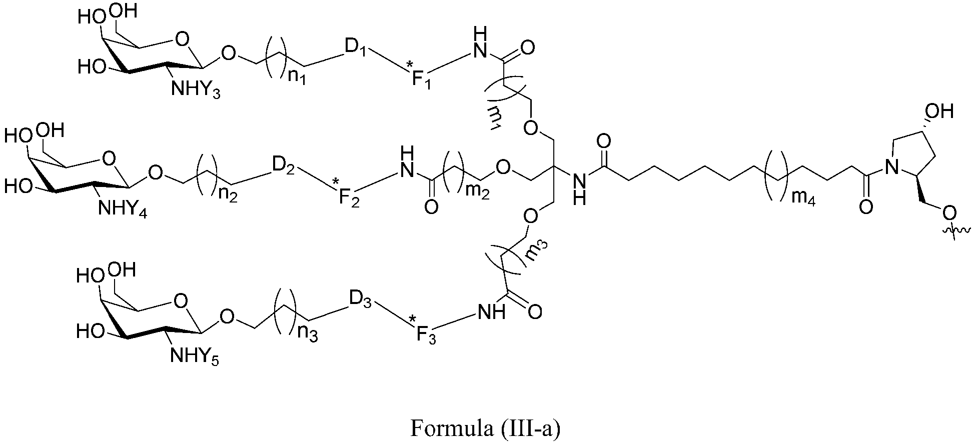 wherein each of D₁, D₂ and D₃ is independently -C(=O)-*, -C(=O)O-*, -OC(=O)-*, -C(=O)N(R¹)-*, -N(R¹)C(=O)-*, -C(=O)N(R¹)CH₂-* or -N(R¹)C(=O)CH₂-*;
each of F₁, F₂, and F₃ is independently cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl;
each of n₁, n₂, n₃, m₁, m₂, m₃ and m₄ is independently 0, 1, 2, 3 or 4;
wherein, each of R₁, Y₃, Y₄ and Y₅ has the meaning described in the present invention.

In another aspect, the present invention relates to a nucleic acid conjugate comprising the conjugated group described herein and a nucleic acid, wherein the nucleic acid may be modified or unmodified.

In some embodiments of the nucleic acid conjugate described herein, the nucleic acid is linked to the conjugated group via a phosphate ester group, a phosphorothioate group, or a phosphate group.

In some embodiments of the nucleic acid conjugate described herein, wherein the nucleic acid is a single-stranded nucleic acid or a double-stranded nucleic acid, preferably siRNA; optionally, the conjugated group is preferably conjugated to the 3' end or 5' end of the sense strand of the siRNA, more preferably conjugated to the 3' end of the sense strand of the siRNA.

In some embodiments of the nucleic acid conjugate described herein, wherein the conjugated group is conjugated to the 3' end or 5' end of the nucleic acid.

In another aspect, the present invention relates to a siRNA conjugate, which is one of DAW30223, DAW30062, DAW30219, DAW30221, DAW30222 and DAW30255,
DAW30223:
   Sense Strand (5'-3'): gs-us-g-u-Gf-c-Af-Cf-Uf-u-c-g-c-u-u-c-a-c-a-DAW40002-4 (SEQ ID NO: 2)
   Antisense Strand (5'-3'): us-Gfs-u-g-a-Agn-g-Cf-Gf-a-a-g-u-Gf-c-Af-c-a-cs-us-u (SEQ ID NO: 7)
DAW30062:
   Sense Strand (5'-3'): gs-us-g-u-Gf-c-Af-Cf-Uf-u-c-g-c-u-u-c-a-c-a-DAW40003-4 (SEQ ID NO: 3)
   Antisense Strand (5'-3'): us-Gfs-u-g-a-Agn-g-Cf-Gf-a-a-g-u-Gf-c-Af-c-a-cs-us-u (SEQ ID NO: 7)
DAW30219:
   Sense Strand (5'-3'): gs-us-g-u-Gf-c-Af-Cf-Uf-u-c-g-c-u-u-c-a-c-a-DAW40004-4 (SEQ ID NO: 4)
   Antisense Strand (5'-3'): us-Gfs-u-g-a-Agn-g-Cf-Gf-a-a-g-u-Gf-c-Af-c-a-cs-us-u (SEQ ID NO: 7)
DAW30221:
   Sense Strand (5'-3'): gs-us-g-u-Gf-c-Af-Cf-Uf-u-c-g- c- u- u-c-a-c-a-DAW40005-4 (SEQ ID NO: 5)
   Antisense Strand (5'-3'): us-Gfs-u-g-a-Agn-g-Cf-Gf-a-a-g-u-Gf-c-Af-c-a-cs-us-u (SEQ ID NO: 7)
DAW30222:
   Sense Strand (5'-3'): gs-us-g-u-Gf-c-Af-Cf-Uf-u-c-g- c- u- u-c-a-c-a-DAW40007-4 (SEQ ID NO: 6)
   Antisense Strand (5'-3'): us-Gfs-u-g-a-Agn-g-Cf-Gf-a-a-g-u-Gf-c-Af-c-a-cs-us-u (SEQ ID NO: 7)
DAW30255:
   Sense Strand (5'-3'): gs-us-c-a-u-c-Cf-a-Cf-Af-Af-u-g-a-g-a-g-u-a-c-a-DAW40007-4 (SEQ ID NO: 8)
   Antisense Strand (5'-3'): us-Gfs-u-a-c-(Tgn)-c-u-c-a-u-u-g-Uf-g-Gf-a-u-g-a-cs-gs-a (SEQ ID NO: 9)
   wherein, the conjugated groups DAW40002-4, DAW40003-4, DAW40004-4, DAW40005-4 and DAW40007-4 are each conjugated to the 3' end of the siRNA sense strand through a phosphate ester group, and their structures are shown below:

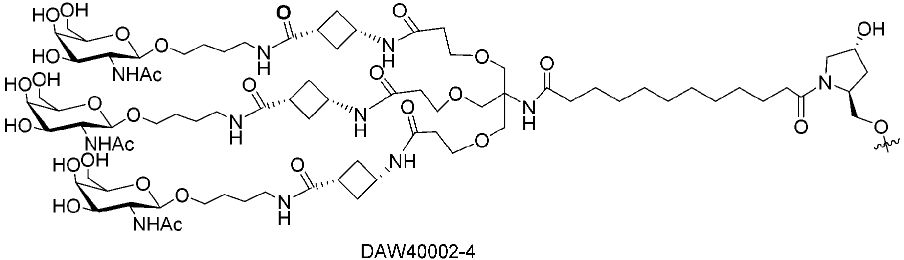

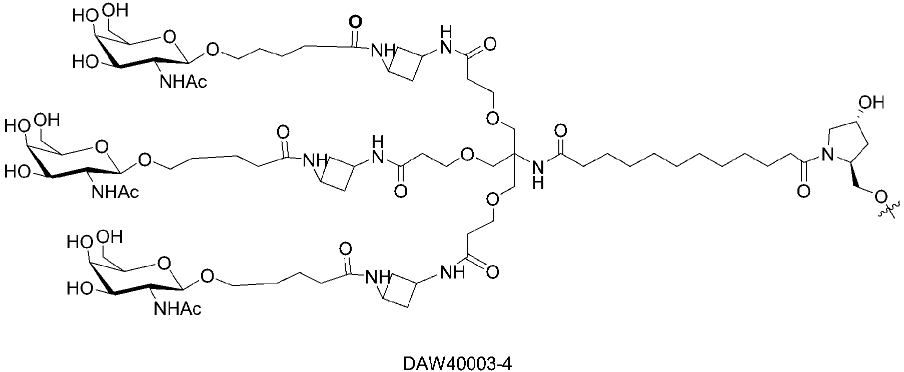

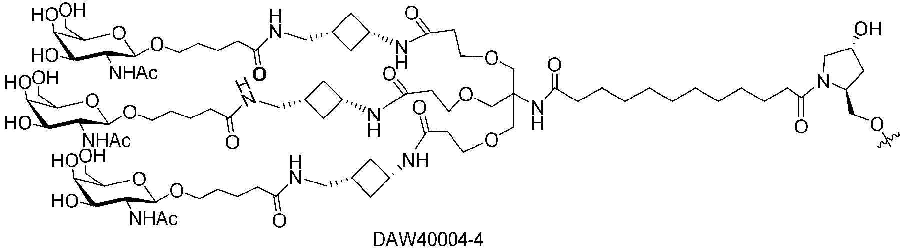

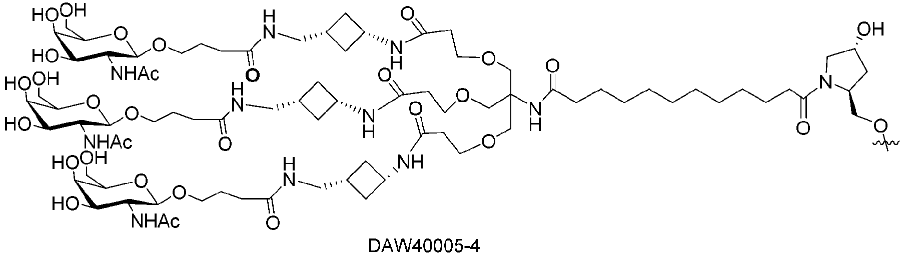

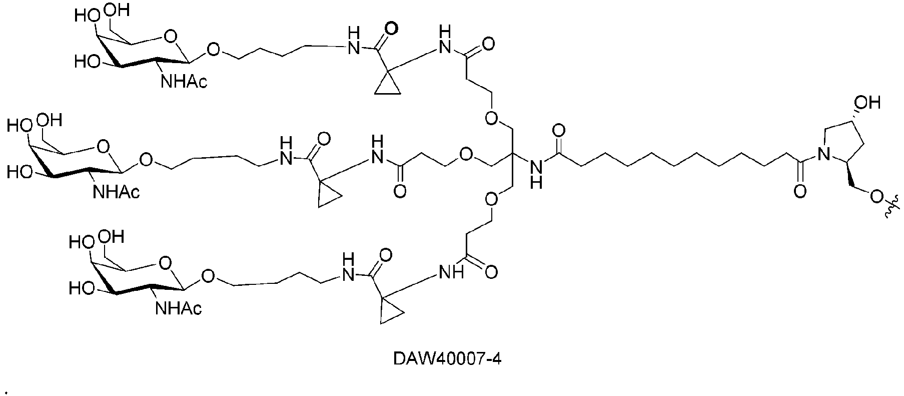

In another aspect, the present invention relates to a pharmaceutical composition comprising the siRNA conjugate herein and a pharmaceutically acceptable carrier.

In another aspect, the present invention also relates to a use of the compound, the conjugated group, or the nucleic acid conjugate herein in the preparation of a medicament for treating and/or preventing a pathological condition or disease caused by the expression of a specific gene in hepatocytes.

In some embodiments, the present invention also relates to the use of the compound or the conjugated group of the present invention in drug delivery for treating and/or preventing pathological conditions or diseases caused by the expression of specific genes in hepatocytes.

In some embodiments of the uses described herein, the compound or conjugated group is used for *in vivo* liver-targeted delivery of a small nucleic acid drug.

In some embodiments of the uses described herein, the compound or conjugated group is used for *in vivo* liver-targeted delivery of an siRNA drug or an ASO drug.

In some embodiments, the present invention further relates to the use of the nucleic acid conjugates described herein in drug delivery for treating and/or preventing pathological conditions or diseases caused by the expression of specific genes in hepatocytes.

In another aspect, the present invention relates to the use of the conjugate groups orthe compounds described herein in drug delivery for treating and/or preventing pathological conditions or diseases caused by the expression of specific genes in hepatocytes.

In some embodiments of the present invention, wherein the specific gene is selected from ApoB, ApoC3, AGT, ANGPTL3, PCSK9, LPA, SCD1, FVII, p53, HBV or HCV.

In another aspect, the present invention relates to the use of the nucleic acid conjugate of the present invention for treating and/or preventing pathological conditions or diseases caused by the expression of specific genes in hepatocytes.

In some embodiments of the present invention, wherein the specific gene is selected from ApoB, ApoC3, AGT, ANGPTL3, PCSK9, LPA, SCD1, FVII, p53, HBV or HCV.

In another aspect, the present invention relates to the siRNA drugs DAW30223, DAW30062, DAW30219, DAW30221, and/or DAW30222, or pharmaceutical compositions thereof, for use in treating and/or preventing hepatitis B.

In another aspect, the present invention relates to DAW30255 or a pharmaceutical composition thereof for use in treating and/or preventing hypertension.

In another aspect, the present invention relates to a method for treating and/or preventing pathological conditions or diseases caused by the expression of specific genes in hepatocytes, which comprises using the compound or the conjugated group of the present invention as a drug delivery system.

In some embodiments of the present invention, wherein the specific gene is selected from ApoB, ApoC3, AGT, ANGPTL3, PCSK9, LPA, SCD1, FVII, p53, HBV or HCV.

In yet another aspect, the present invention relates to a method for treating and/or preventing a pathological condition or disease caused by the expression of a specific gene in hepatocytes, the method comprising administering the nucleic acid conjugate of the present invention.

In some embodiments of the present invention, wherein the specific gene is selected from ApoB, ApoC3, AGT, ANGPTL3, PCSK9, LPA, SCD1, FVII, p53, HBV or HCV.

In another aspect, the present invention relates to a method for preventing, managing, treating or alleviating hepatitis B or hypertension, comprising administering to a patient a therapeutically effective amount of DAW30223, DAW30062, DAW30219, DAW30221, and/or DAW30222, or a pharmaceutical composition thereof.

In another aspect, the present invention relates to a method for preventing, managing, treating or alleviating hepatitis B or hypertension, comprising administering to a patient a therapeutically effective amount of DAW30255, or a pharmaceutical composition thereof.

In some embodiments of the present invention, the nucleic acid (or small nucleic acid) described herein is an siRNA or an ASO.

In some embodiments of the use described herein, the specific gene is selected from, but not limited to, ApoB, ApoC3 (apolipoprotein CIII encoded by the human apolipoprotein CIII gene), AGT (angiotensinogen), ANGPTL3 (angiopoietin-like 3), PCSK9, LPA (lipoprotein(a)), SCD1, FVII, p53, HBV (hepatitis B virus), or HCV (hepatitis C virus). --

In another aspect, The present invention also relates to the use of DAW30223, DAW30062, DAW30219, DAW30221, and/or DAW30222 described herein, and compositions comprising DAW30223, DAW30062, DAW30219, DAW30221, and/or DAW30222 described herein and a pharmaceutically acceptable carrier, in the preparation of a medicament for treating and/or preventing hepatitis B. In another aspect, the present invention also relates to the use of DAW30255 described herein, or a composition thereof, in the preparation of a medicament for treating and/or preventing hypertension.

### DETAILED DESCRIPTION OF THE INVENTION

### DEFINITIONS AND GENERAL TERMINOLOGY

In the present invention, the term "comprising" or "including" is open expression, it means comprising the contents disclosed herein, but don't exclude other contents.

In the present invention, the term "small interfering RNA (siRNA)" refers to a double-stranded RNA of 17 to 30 nucleotides in length, comprising a sense strand and an antisense strand. siRNAs mediate the targeted cleavage of RNA transcripts in the RISC pathway by forming a RNA-induced silencing complex (RISC). Specifically, siRNAs direct the specific degradation of mRNA sequences through a process known as RNA interference (RNAi), inhibiting the translation of mRNA into amino acids and protein. For example, siRNAs can modulate (e.g., inhibit) the expression of hepatitis B virus in cells.

In this invention, the term "antisense strand (or guide strand)" includes the region that is substantially complementary to a target sequence, such as hepatitis B virus mRNA. The "sense strand (or passenger strand)" refers to the strand containing the iRNA that is substantially complementary to the antisense strand. The term "substantially complementary" refers to complete complementarity or at least partial complementarity, e.g., the antisense strand is completely complementary or at least partially complementary to the target sequence. In the case of partial complementarity, mismatches can occur within or in terminal regions of the molecule, with the most tolerated mismatches occurring in the terminal regions, e.g., within 5, 4, 3, or 2 nucleotides of the 5'- and/or 3'- end of the iRNA.

It should be noted that "the antisense strand is substantially complementary to at least a portion of an mRNA" means that the antisense strand comprises a polynucleotide that is substantially complementary to a continuous portion of an mRNA of interest (e.g., an mRNA encoding hepatitis B virus). Alternatively, if a polynucleotide is substantially non-interruptedly complementary to a portion of an mRNA encoding hepatitis B virus, then the antisense strand is complementary to at least a portion of the hepatitis B virus mRNA.

In the present invention, the term "target sequence" refers to a continuous portion of the nucleotide sequence of an mRNA molecule formed during transcription of a gene encoding hepatitis B virus, including mRNA that is an RNA processing product of a primary transcription product.

In the present invention, the term "inhibiting the expression of hepatitis B virus genes" includes any level of inhibition of hepatitis B virus (HBV) genes, for example, at least partial inhibition of HBV gene expression, such as inhibition of at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98% or at least about 99%. HBV gene expression can be assessed based on the level of any variable associated with HBV gene expression, such as HBV mRNA levels or HBV protein levels. Inhibition can be assessed by a decrease in the absolute or relative level of one or more of these variables compared to a control level. The control level can be any type of control level used in the art, for example, a baseline level before administration, or a level measured from a similar subject, cell, or sample, either untreated or treated with a control (e.g., a buffer-only control or an inactive agent-free control).

In the present invention, a "pharmaceutical composition" can refer to a composition used for treating a disease or for use in in vitro cell culture experiments. When used for treating a disease, the term "pharmaceutical composition" generally refers to a unit dosage form and can be prepared by any method well known in the pharmaceutical art. All methods include the step of combining the active ingredient with an excipient which constitutes one or more accessory ingredients. Generally, the compositions are prepared by uniformly and thoroughly combining the active siRNA with a liquid excipient, a finely divided solid excipient, or both.

In the present invention, the term "pharmaceutically acceptable" means that the substance or composition must be chemically and/or toxicologically compatible with the other ingredients comprising the formulation and/or the mammal to be treated therewith. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

In the present invention, the term "pharmaceutically acceptable excipient" encompasses any solvent, solid excipient, diluent, or other liquid excipient, suitable for the specific intended dosage form. Except to the extent that any conventional excipient is incompatible with the RNAi (e.g., siRNA) of the present invention, such as by producing any adverse biological effects or interacting deleteriously with any other component of the pharmaceutically acceptable composition, its use is contemplated by the present invention.

Except to the extent that any conventional excipient is incompatible with the RNAi (e.g., siRNA) of the present invention, such as by producing any adverse biological effects or interacting deleteriously with any other component of the pharmaceutically acceptable composition, its use is contemplated by the present invention.

As used herein, the term "treat" or "treatment" refers to any agent intended to achieve a desired pharmacological and/or physiological effect. The effect may be prophylactic, meaning completely or partially preventing a disease or its symptoms, and/or therapeutic, meaning partially or completely curing a disease and/or its adverse effects. As used herein, "treat" encompasses diseases in mammals, particularly humans, and includes: (a) preventing a disease or condition from occurring in an individual susceptible to the disease but not yet diagnosed with the disease; (b) inhibiting the disease, such as halting its progression; or (c) alleviating the disease, such as alleviating the symptoms associated with the disease. As used herein, "treatment" encompasses any administration of a drug, RNAi agent, or siRNA to a subject to treat, cure, alleviate, ameliorate, mitigate, or inhibit a disease in the subject, including but not limited to administering a drug containing an RNAi agent, siRNA, or siRNA conjugate described herein to a subject in need thereof.

Unless otherwise specified, in the context of the present invention, capital letters C, G, U, A, and T represent the base composition of the nucleotide. In the present invention, a, g, c, and u are 2'-O-methyl (2'-OMe) A, 2'-OMe G, 2'-OMeC, and 2'-OMe U, respectively; Af, Cf, Gf, and Uf are 2'-fluoro A, 2'-fluoro C, 2'-fluoro G, and 2'-fluoro U, respectively; s indicates that the nucleotide has a 5'-phosphorothioate modification; Agn (i.e., GNA) is an adenosine-glycol nucleic acid, and Tgn represents a thymine-glycol nucleotide residue, whose structure is

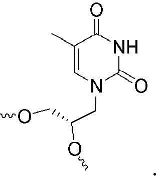

In the context of this invention, Bz represents a benzoyl group; MMTr represents a 4'-methoxytrityl group; and DMTr represents a 4',4'-dimethoxytriphenylmethyl group.

In this invention, "phosphate ester group," "phosphate ester," and "phosphate ester bond" are used interchangeably to include monoesters, phosphodiesters, and phosphotriesters. The term "phosphate ester" in "phosphorothioate group" also has the same meaning. Unless otherwise specified, a natural internucleotide phosphate group is a phosphodiester group.

The term "oxo" refers to a =O group. For example, a carbon atom is double-bonded to an oxygen atom, forming a ketone or aldehyde group.

As used herein, "chemically modified" or "modification" refers to a structure that is chemically different from its naturally occurring counterpart, including all alterations by chemical means, such as the addition or removal of a chemical moiety or the substitution of one chemical moiety for another.

The compounds of the present invention may be asymmetric, e.g., have one or more stereoisomers. Unless otherwise indicated, all stereoisomers are included, including enantiomers and diastereomers. Compounds of the present invention containing asymmetric carbon atoms can be isolated in optically pure or racemic forms. Optically pure forms can be resolved from racemic mixtures or synthesized using chiral starting materials or reagents.

Optically active (*R*)- and (*S*)- isomers, as well as D and L isomers, can be prepared by chiral synthesis or chiral reagents or other conventional techniques. If a single enantiomer of a compound of the present invention is desired, it can be prepared by asymmetric synthesis or derivatization with a chiral auxiliary, wherein the resulting diastereomeric mixture is separated and the auxiliary group is cleaved to provide the pure desired enantiomer. Alternatively, when the molecule contains a basic functional group (e.g., amino) or an acidic functional group (e.g., carboxyl), diastereomeric salts can be formed with an appropriate optically active acid or base, followed by diastereomeric resolution by conventional methods known in the art, and the pure enantiomer can be recovered. Furthermore, separation of enantiomers and diastereomers is typically accomplished by using chromatography employing chiral stationary phases, optionally combined with chemical derivatization (e.g., formation of carbamates from amines).

The present invention also includes isotopically labeled compounds of the present invention that are identical to those described herein, except that one or more atoms are replaced by an atom having an atomic mass or mass number different from that usually found in nature. Examples of isotopes that may be incorporated into the compounds of the present invention include isotopes of hydrogen, carbon, nitrogen, oxygen, phosphorus, sulfur, fluorine, iodine, and chlorine, such as ²H, ³H, ¹¹C, 13C, ¹⁴C, ¹³N, ¹⁵N, ¹⁵O, ¹⁷O, ¹⁸O, ³¹P, ³²P, ³⁵S, ¹⁸F, ¹²³I, ¹²⁵I and ³⁶Cl, respectively.

Unless otherwise indicated, when a position is specifically designated as deuterium (D), that position is understood to have an abundance of deuterium at least 1000 times greater than the natural abundance of deuterium (which is 0.015%) (i.e., at least 10% deuterium incorporation). Example compounds having an abundance greater than the natural abundance of deuterium can include an abundance of deuterium at least 1000 times greater, at least 2000 times greater, at least 3000 times greater, at least 4000 times greater, at least 5000 times greater, at least 6000 times greater, or even greater. The present invention also encompasses various deuterated forms of compounds of Formula (I). Each available hydrogen atom attached to a carbon atom can be independently replaced with a deuterium atom. One skilled in the art will be able to synthesize deuterated forms of compounds of Formula (I) by referring to the relevant literature. In preparing deuterated forms of compounds of formula (I), commercially available deuterated starting materials may be used, or they may be synthesized using conventional techniques using deuterated reagents, including but not limited to deuterated borane, trideuterated borane in tetrahydrofuran, deuterated lithium aluminum hydride, deuterated iodoethane, deuterated iodomethane, and the like.

The conjugated groups described herein can enhance the delivery of therapeutic agents to specific target locations (e.g., specific organs or tissues) within a subject, such as a human or animal. In some embodiments of the present invention, the conjugated groups can enhance the targeted delivery of expression-inhibitory oligonucleotides. In some embodiments of the present invention, the conjugated groups can enhance the delivery of expression-inhibitory oligonucleotides to the liver.

The conjugate groups described herein can be directly or indirectly attached to a compound, such as a therapeutic agent, for example, an expression-suppressing oligonucleotide, for example, to the 3' or 5' end of the expression-suppressing oligonucleotide. In some embodiments of the present invention, the expression-suppressing oligonucleotide comprises one or more modified nucleotides. In some embodiments of the present invention, the expression-suppressing oligonucleotide is an RNAi agent, such as a double-stranded RNAi agent comprising a sense strand and an antisense strand. In some embodiments of the present invention, a conjugated group disclosed herein is attached to the 3' end of the sense strand of a double-stranded RNAi agent. In some embodiments, a phosphate, phosphorothioate, or phosphate group of a conjugated group disclosed herein is attached to a fluorescently expressed oligomer at the 3' end of the sense strand of a double-stranded RNAi agent.

Unless otherwise specified, "conjugation" refers to the covalent attachment of two or more chemical moieties, each with a specific function, to one another; accordingly, "conjugate" refers to a compound formed by covalent attachment of chemical moieties.

The double-stranded siRNA conjugate of the present invention is a compound formed by connecting the double-stranded siRNA and a pharmaceutically acceptable conjugated group, and the double-stranded siRNA and the pharmaceutically acceptable conjugated group are covalently linked.

Stereochemical definitions and conventions used herein generally follow S. P. Parker Ed. , McGraw-Hill Dictionary of Chemical Terms (1984) McGraw-Hill Book Company, New York and Eliel et al., "Stereochemistry of Organic Compounds", John Wiley & Sons, Inc., New York, 1994. The compounds disclosed herein may contain asymmetric or chiral centers, and therefore exist in different stereoisomeric forms. It is intended that all stereoisomeric forms of the compounds disclosed herein, including, but not limited to, diastereomers, enantiomers and atropisomers, as well as mixtures thereof such as racemic mixtures, form part of the present invention. Many organic compounds exist in optically active forms, i.e., they have the ability to rotate the plane of plane-polarized light. In describing an optically active compound, the prefixes *D* and *L,* or *R* and *S*, are used to denote the absolute configuration of the molecule about its chiral center(s). The prefixes *d* and *l* or (+) and (-) are employed to designate the sign of rotation of plane-polarized light by the compound, with (-) or *l* meaning that the compound is levorotatory. A compound prefixed with (+) or *d* is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process. The term "racemic mixture" or "racemate" refers to an equimolar mixture of two enantiomeric species, devoid of optical activity.

The term "tautomer" or "tautomeric form" refers to structural isomers of different energies which are interconvertible via a low energy barrier. For example, proton tautomers (i.e., prototropic tautomers) include interconversions via migration of a proton, such as keto-enol and imine-enamine isomerizations.

The term "composition" refers to a mixture of one or more compounds described herein, or their physiologically acceptable salts or precursors, with other chemical components, as well as other components such as physiologically acceptable carriers and excipients. The purpose of a composition is to facilitate administration to an organism, facilitating absorption of the active ingredient and subsequent biological activity.

The term "pharmaceutical excipient" or "pharmaceutically acceptable excipient" includes, but is not limited to, any adjuvant, carrier, excipient, glidant, sweetener, diluent, preservative, dye/colorant, flavoring agent, surfactant, wetting agent, dispersant, suspending agent, stabilizer, isotonicity agent, solvent, or emulsifier approved by the U.S. Food and Drug Administration for human or domestic animal use.

Unless otherwise specified, the "compound," "ligand," "nucleic acid-ligand conjugate," and "nucleic acid" of the present invention may independently exist in the form of a salt, a mixed salt, or a non-salt form (e.g., a free acid or free base). When present in the form of a salt or mixed salt, it may be a pharmaceutically acceptable salt.

The term "acceptable salt" includes both acceptable acid addition salts and pharmaceutically acceptable base addition salts. "Acceptable acid addition salts" refer to salts formed with inorganic or organic acids that retain the biological effectiveness of the free base without other side effects. Inorganic acid salts include, but are not limited to, hydrochloride, hydrobromide, sulfate, nitrate, phosphate, and the like; organic acid salts include, but are not limited to, formate, acetate, 2,2-dichloroacetate, trifluoroacetate, propionate, caproate, caprylate, decanoate, undecylenate, glycolate, gluconate, lactate, sebacate, adipate, glutarate, malonate, oxalate, maleate, succinate, fumarate, tartrate, citrate, palmitate, stearate, oleate, cinnamate, laurate, malate, glutamate, pyroglutamate, aspartate, benzoate, methanesulfonate, benzenesulfonate, p-toluenesulfonate, alginate, ascorbate, salicylate, 4-aminosalicylate, naphthalene disulfonate, etc. These salts can be prepared by methods known in the art.

"Pharmaceutically acceptable base addition salts" refer to salts formed with inorganic or organic bases that retain the biological effectiveness of the free acid without other adverse effects. Salts derived from inorganic bases include, but are not limited to, sodium, potassium, lithium, ammonium, calcium, magnesium, iron, zinc, copper, manganese, and aluminum salts. Preferred inorganic salts are ammonium, sodium, potassium, calcium, and magnesium salts, with sodium salt being preferred. Salts derived from organic bases include, but are not limited to, salts of primary, secondary, and tertiary amines, substituted amines, including naturally substituted amines, cyclic amines, and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purines, piperazine, piperidine, N-ethylpiperidine, polyamine resins, and the like. Preferred organic bases include isopropylamine, diethylamine, ethanolamine, trimethylamine, dicyclohexylamine, choline, and caffeine. These salts can be prepared by methods known in the art.

As described herein, compounds disclosed herein may optionally be substituted with one or more substituents, such as are illustrated generally below, or as exemplified by particular classes, subclasses, and species of the invention. In general, the term "substituted" refers to the replacement of one or more hydrogen radicals in a given structure with the radical of a specified substituent. Unless otherwise indicated, an optionally substituted group may have a substituent at each substitutable position of the group. When more than one position in a given structure can be substituted with more than one substituent selected from a specified group, the substituent may be either the same or different at each position.

Furthermore, unless otherwise stated, the phrase "each...is independently" is used interchangeably with the phrase "each (of)... and... is independently". It should be understood broadly that the specific options expressed by the same symbol are independently of each other in different radicals; or the specific options expressed by the same symbol are independently of each other in same radicals.

In the present invention, the terms "optionally", "optional" or "option" generally mean that the subsequently described event or circumstance can but need not occur, and that the description includes instances where the event or circumstance occurs and instances where it does not occur.

At various places in the present specification, substituents of compounds disclosed herein are disclosed in groups or in ranges. It is specifically intended that the invention include each and every individual subcombination of the members of such groups and ranges. For example, the term "C₁-₆ alkyl" is specifically intended to individually disclose methyl, ethyl, C₃ alkyl, C₄ alkyl, C₅ alkyl, and C₆ alkyl.

The term "alkyl" or "alkyl group" refers to a saturated linear or branched-chain monovalent hydrocarbon radical of 1 to 20 carbon atoms, wherein the alkyl radical may be optionally and independently substituted with one or more substituents described herein. In some embodiments, the alkyl group contains 1-12 carbon atoms. In other embodiments, the alkyl group contains 1-10 carbon atoms. In other embodiments, the alkyl group contains 1-8 carbon atoms. In still other embodiments, the alkyl group contains 1-6 carbon atoms. In yet other embodiments, the alkyl group contains 1-4 carbon atoms and in still yet other embodiments, the alkyl group contains 1-3 carbon atoms. Further examples of alkyl groups include, but are not limited to, methyl (Me, -CH₃), ethyl (Et, -CH₂CH₃), *n*-propyl (*n*-Pr, -CH₂CH₂CH₃), isopropyl (*i*-Pr, -CH(CH₃)₂), *n*-butyl (*n*-Bu, -CH₂CH₂CH₂CH₃), 2-methylpropyl or isobutyl (*i*-Bu, -CH₂CH(CH₃)₂), 1-methylpropyl or *sec*-butyl (*s*-Bu , -CH(CH₃)CH₂CH₃), *tert*-butyl (*t*-Bu, -C(CH₃)₃), *n*-pentyl (-CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH (CH₃)₂), 3-methyl-1-butyl (-CH₂CH₂CH(CH₃)₂), 2-methyl-1-butyl (-CH₂CH(CH₃)CH₂CH₃), *n*-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), *n*-heptyl, *n*-octyl, *n*-nonyl, *n*-heptyl, and the like.

The term "alkylene" refers to a saturated divalent or multivalent hydrocarbon radical obtained by removing two radicals from a saturated straight-chain or branched hydrocarbon radical. Unless otherwise specified, the alkylene group contains 1-12 carbon atoms. In some embodiments, the alkylene group contains 1-6 carbon atoms. In other embodiments, the alkylene group contains 1-4 carbon atoms. In still other embodiments, the alkylene group contains 1-3 carbon atoms. In other embodiments, the alkylene group contains 1-2 carbon atoms. Examples of alkylene groups include, but are not limited to, methylene (-CH₂-), ethylene (-CH₂CH₂-), isopropylene (-CH(CH₃)CH₂-), *n*-propylene (-CH₂CH₂CH₂-), *n*-butylene (-CH₂CH₂CH₂CH₂-), *sec*-butylene (*s*-Bu, -CH(CH₃)CH₂CH₂-), *n*-pentylene (-CH₂CH₂CH₂CH₂CH₂-), 2-pentylene (-CH(CH₃)CH₂CH₂CH₂-), *n*-hexylene (-CH₂CH₂CH₂CH₂CH₂CH₂-), 2-hexylene (-CH(CH₃)CH₂CH₂CH₂CH₂-), *n*-heptylene (-(CH₂)₇-), *n*-octylene (-(CH₂)₈-), *n*-nonylene (-(CH₂)₉-), decylene (-(CH₂)₁₀-), -(CH₂)₁₁-, -(CH₂)₁₂-, -(CH₂)₁₃-, -(CH₂)₁₄-, and the like.

As used herein, the term "heteroalkyl" refers to an alkyl group having 1, 2, 3, 4, 5, or 6 heteroatoms inserted therein, wherein the heteroatoms are selected from S, N, NH, O or P. The sulfur atom may optionally be oxidized to an S-oxide. In some embodiments, the heteroalkyl group contains 1-12 carbon atoms, in other embodiments, the heteroalkyl group contains 1-10 carbon atoms, in other embodiments, the heteroalkyl group contains 1-8 carbon atoms, in other embodiments, the heteroalkyl group contains 1-6 carbon atoms, in other embodiments, the heteroalkyl group contains 1-4 carbon atoms, and in other embodiments, the heteroalkyl group contains 1-3 carbon atoms. Examples of heteroalkyl groups include, but are not limited to, CH₃O-, CH₃CH₂O-, CH₃CH₂CH₂O-, CH₃CH₂CH₂CH₂O-, CH₃CH₂OCH₂-, CH₃OCH₂CH₂-, CH₃CH₂CH₂OCH₂-, CH₃CH₂OCH₂CH₂-, CH₃OCH₂CH₂CH₂-, CH₃S-, CH₃CH₂S-, CH₃CH₂CH₂S-, CH₃CH₂CH₂CH₂S-, CH₃CH₂SCH₂-, CH₃SCH₂CH₂-, CH₃CH₂CH₂SCH₂-, CH₃CH₂SCH₂CH₂-, CH₃SCH₂CH₂CH₂-, CH₃NH-, CH₃CH₂NH-, CH₃CH₂CH₂NH-, CH₃CH₂CH₂CH₂NH-, CH₃CH₂NHCH₂-, CH₃NHCH₂CH₂-, CH₃CH₂CH₂NHCH₂-, CH₃CH₂NHCH₂CH₂-, CH₃NHCH₂CH₂CH₂-, and the like.

The term "alkoxy" means an alkyl group attached to the rest of the molecule via an oxygen atom, wherein the alkyl group has the meaning as defined herein. Unless otherwise specified, the alkoxy group contains 1-12 carbon atoms. In some embodiments, alkoxy groups contain 1-8 carbon atoms; in other embodiments, alkoxy groups contain 1-6 carbon atoms; in other embodiments, alkoxy groups contain 1-4 carbon atoms; in yet other embodiments, alkoxy groups contain 1-3 carbon atoms. The alkoxy group may be optionally substituted with one or more substituents disclosed herein.

Examples of alkoxy groups include, but are not limited to, methoxy (MeO, -OCH₃), ethoxy (EtO, -OCH₂CH₃), 1-propoxy (*n*-PrO, *n*-propoxy, -OCH₂CH₂CH₃), 2-propoxy (*i*-PrO, *i*-propoxy, -OCH(CH₃)₂), 1-butoxy (*n*-BuO, *n*-butoxy, -OCH₂CH₂CH₂CH₃), 2-methyl-1-propoxy (*i*-BuO, *i*-butoxy, -OCH₂CH(CH₃)₂), 2-butoxy (*s*-BuO, *s*-butoxy, -OCH(CH₃)CH₂CH₃), 2-methyl-2-propoxy (*t*-BuO, *t*-butoxy, -OC(CH₃)₃), 1-pentoxy (*n*-pentoxy, -OCH₂CH₂CH₂CH₂CH₃), 2-pentoxy (-OCH(CH₃)CH₂CH₂CH₃), 3-pentoxy (-OCH(CH₂CH₃)₂), 2-methyl-2-butoxy (-OC(CH₃)₂CH₂CH₃), 3-methyl-2-butoxy (-OCH(CH₃)CH(CH₃)₂), 3-methyl-1-butoxy (-OCH₂CH₂CH(CH₃)₂), 2-methyl-1-butoxy (-OCH₂CH(CH₃)CH₂CH₃), and the like.

The term "alkenyl" refers to a linear or branched monovalent, divalent, or polyvalent hydrocarbon group containing 2-12 carbon atoms, or 2-8 carbon atoms, or 2-6 carbon atoms, or 2-4 carbon atoms, wherein at least one C-C position is an sp2 double bond. The alkenyl group may be independently unsubstituted or substituted with one or more substituents described herein, including "cis" "trans" "*Z*" and "*E*" isomers. Specific examples include, but are not limited to, ethenyl (-CH=CH₂), propenyl (-CH=CHCH₃), allyl (-CH₂CH=CH₂), and the like, wherein the alkenyl group may be independently unsubstituted or substituted with one or more substituents described herein.

The term "alkynyl" refers to a linear or branched monovalent or divalent hydrocarbon group of 2 to 12 carbon atoms, wherein at least one C-C is an sp triple bond, wherein the alkynyl group may be independently and optionally substituted with one or more substituents described herein. In some embodiments, the alkynyl group contains 2 to 12 carbon atoms, in other embodiments, the alkynyl group contains 2 to 8 carbon atoms, in other embodiments, the alkynyl group contains 2 to 6 carbon atoms, and in other embodiments, the alkynyl group contains 2 to 4 carbon atoms. Specific examples include, but are not limited to, ethynyl (-C≡CH), propargyl (-CH₂C≡CH), propynyl (-C≡C-CH₃), butyl alkynyl (-CH₂CH₂C≡CH, -CH₂C≡CCH₃, -C≡CCH₂CH₃ and -CH(CH₃)C≡CH) and pentynyl (-CH₂CH₂CH₂C≡CH, -CH₂CH₂C≡CCH₃, -CH₂C≡CCH₂CH₃, -C≡CCH₂CH₂CH₃, -CH₂CH(CH₃)C≡CH, -CH(CH₃)CH₂C≡CH), -C(CH₃)₂C≡CH, -CH(CH₃)C≡CCH₃, -C≡CCH(CH₃)CH₃ 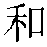 -C≡CCH(CH₃)₂), and the like.

The term "cycloalkyl" refers to a monovalent or multivalent saturated ring having 3 to 12 carbon atoms as a monocyclic, bicyclic, or tricyclic ring system, In one embodiment, the cycloalkyl group contains 3 to 12 carbon atoms; in another embodiment, the cycloalkyl group contains 3 to 8 carbon atoms; in another embodiment, the cycloalkyl group contains 3 to 7 carbon atoms; and in yet other embodiments, the cycloalkyl group contains 3 to 6 carbon atoms. Examples of cycloalkyl groups further include, but are not limited to, cyclopropyl, cyclobutyl, cyclopentyl, cyclohexyl, and the like.

The term "consisting of M-M₁ ring atoms" means that the cyclic group is composed of M-M₁ ring atoms, wherein the ring atoms include carbon atoms and/or heteroatoms such as O, N, S and P. For example, "heterocyclyl consisting of 3-6 ring atoms" refers to a monocyclic heterocyclic group consisting of 3, 4, 5 or 6 ring atoms.

The term "heterocyclyl" refers to a non-aromatic, saturated, or partially unsaturated monocyclic, bicyclic, or tricyclic ring system containing 3-12 ring atoms, wherein at least one ring atom is selected from nitrogen, sulfur or oxygen. Wherein, the heterocyclyl group may be optionally substituted with one or more substituents disclosed herein. Unless otherwise specified, a heterocyclic group can be carbon or nitrogen-based, and a -CH₂- group can be optionally replaced by -C(=O)- or -C(=S)-. A ring sulfur atom can be optionally oxidized to form an S-oxide. A ring nitrogen atom can be optionally oxidized to form an N-oxide. In some embodiments, the heterocyclyl group is a heterocyclyl group consisting of 3-12 ring atoms, in some embodiments, the heterocyclyl group is a heterocyclyl group consisting of 5-10 ring atoms, in some embodiments, the heterocyclyl group is a heterocyclyl group consisting of 3-6 ring atoms, in some embodiments, the heterocyclyl group is a heterocyclyl group consisting of 4-6 ring atoms, in some embodiments, the heterocyclyl group is a heterocyclyl group consisting of 5-6 ring atoms, in other embodiments, the heterocyclyl group is a heterocyclyl group consisting of 4 atoms, which refers to a monovalent or polyvalent, saturated or partially unsaturated, non-aromatic monocyclic ring containing 4 ring atoms, wherein at least one ring atom is selected from nitrogen, sulfur and oxygen atoms. In other embodiments, the heterocyclyl group is a 5-atom heterocyclyl group, which refers to a monovalent or polyvalent, saturated or partially unsaturated, non-aromatic, monocyclic ring containing 5 ring atoms, wherein at least one ring atom is selected from nitrogen, sulfur and oxygen. In other embodiments, the heterocyclyl group is a 6-atom heterocyclyl group, which refers to a monovalent or polyvalent, saturated or partially unsaturated, non-aromatic, monocyclic ring containing 6 ring atoms, wherein at least one ring atom is selected from nitrogen, sulfur and oxygen. "Heterocyclyl" also includes a heterocyclyl group fused with a saturated or partially unsaturated ring or heterocyclic ring. Examples of heterocycles include, but are not limited to, pyrrolidinyl, tetrahydrofuranyl, dihydrofuranyl, tetrahydrothiophenyl, tetrahydropyranyl, dihydropyranyl, tetrahydrothiopyranyl, piperidinyl, morpholinyl, thiomorpholinyl, thioxanyl, piperazinyl, homopiperazinyl, azetidinyl, oxetanyl, thietanyl, homopiperidinyl, glycidyl, azepanyl, oxetanyl, thietanyl, oxazepinyl, diazepinyl, thiazepinyl, 2-pyrrolinyl, 3- pyrrolinyl, indolinyl, 2*H*-pyranyl, 4*H*-pyranyl, dioxanyl, 1,3-dioxolane, pyrazolinyl, dithianyl, dithiolanyl, dihydrothienyl, pyrazolidinylimidazolinyl, imidazolidinyl, 1,2,3,4-tetrahydroisoquinolinyl, 3-azabicyclo[3.1.0]hexyl, 3-azabicyclo[4.1.0]heptyl, azabicyclo[2.2.2]hexyl, 3*H*-indolylquinolizinyl and N-pyridylurea. Examples of heterocyclic groups also include 1,1-dioxothiomorpholinyl, examples of which the carbon atoms on the ring are replaced by oxo (=O) include, but are not limited to, pyrimidinedione, 1,2,4-thiadiazole-5(4*H*)-one, 1,2,4-oxadiazole-5(4*H*)-one, 1H-1,2,4-triazole-5(4*H*)-one, etc., examples of which the carbon atoms on the ring are replaced by a group =S include, but are not limited to, 1,2,4-oxadiazole-5(4*H*)-thione, 1,3,4-oxadiazole-2(3*H*)-thione, and the like.

The term "heteroatom" refers to one or more O, S, N, P and Si, including N, S and P in any oxidation state; in the form of primary, secondary, tertiary amines and quaternary ammonium salts; or in the form of a nitrogen atom in a heterocyclic ring in which the hydrogen is substituted, for example, N (such as N in 3,4-dihydro-2H-pyrrolyl), NH (such as NH in pyrrolidinyl) or NR (such as NR in N-substituted pyrrolidinyl, wherein R represents a substituent as described herein).

The term "aryl" refers to a monocyclic, bicyclic or tricyclic carbon ring system containing 6 to 14 ring atoms, or 6 to 12 ring atoms, or 6 to 10 ring atoms, wherein at least one ring system is aromatic, wherein each ring system comprises 3 to 7 ring atoms and has one or more points of attachment to the rest of the molecule. The term "aryl" and "aromatic ring" can be used interchangeably herein. Examples of aryl groups include phenyl, naphthyl, and anthracenyl. The aryl group may be optionally and independently substituted with one or more substituents disclosed herein.

The terms "alkylsilyl" and "silylalkyl" refer to groups in which the hydrogen atoms in a silyl (-SiH₃) group are independently substituted with one, two, or three alkyl groups, respectively. In some embodiments, the alkylsilyl group is a lower alkylsilyl group formed by attaching one, two, or three C₁₋₁₂ alkyl groups to a silicon atom. In other embodiments, the alkylsilyl group is a lower alkylsilyl group formed by attaching one, two, or three C₁₋₉ alkyl groups to a silicon atom. In other embodiments, the alkylsilyl group is a lower alkylsilyl group formed by attaching one, two, or three C₁₋₆ alkyl groups to a silicon atom. In other embodiments, the alkylsilyl group is a lower alkylsilyl group formed by attaching one, two, or three C₁₋₄ alkyl groups to a silicon atom. In still other embodiments, the alkylsilyl group is a lower alkylsilyl group formed by attaching one, two, or three C₁₋₃ alkyl groups to a silicon atom. Suitable alkylsilyl groups can be monoalkylsilyl, dialkylsilyl, or trialkylsilyl. Examples of alkylsilyl groups include, but are not limited to, trimethylsilyl (-Si(CH₃)₃), triethylsilyl (-Si(CH₂CH₃)₃), tri-n-propylsilyl (-Si(CH₂CH₂CH₃)₃), and the like.

The term "hydroxy-protecting group" refers to a labile chemical moiety that protects a hydroxy group from undesirable reactions during one or more synthetic procedures. After the one or more synthetic procedures, the hydroxy protecting group can be selectively removed. Hydroxy protecting groups known in the art are generally described in T.H. Greene and P.G.M. Wuts, Protective Groups in Organic Synthesis, 3rd ed., John Wiley & Sons, New York (1999). Examples of hydroxy protecting groups of the present invention include, but are not limited to, C₁₋₁₀ alkylmethyl, benzyloxycarbonyl, 4-nitrobenzyloxycarbonyl, 4-bromobenzyloxycarbonyl, 4-methoxybenzyloxycarbonyl, methoxycarbonyl, tert-butoxycarbonyl, isopropyloxycarbonyl, biphenylmethoxycarbonyl, 2,2,2-trichloroethoxycarbonyl, 2-(trimethylsilyl)ethoxycarbonyl, 2-furfuryloxycarbonyl, allyloxycarbonyl, acetyl (Ac or -C(O)CH₃), formyl, chloroacetyl, trifluoroacetyl, methoxyacetyl, phenoxyacetyl, benzoyl (Bz or -C(O)C₆H₅), C₁₋₁₀ alkyl (methyl, tert-butyl, etc.), 2,2,2-trichloroethyl, 2-trimethylsilylethyl , 1,1-dimethyl-2-propenyl, 3-methyl-3-butenyl, allyl, C₆₋₁₀ aryl C₁₋₄ alkyl (such as benzyl, phenethyl, etc.), p-methoxybenzyldiphenylmethyl, triphenylmethyl (triphenylmethyl or trityl), tetrahydrofuranyl, methoxymethyl, methylthiomethyl, benzyloxymethyl, 2,2,2-trichloroethoxymethyl, 2-(trimethylsilyl)ethoxymethyl, methylsulfonyl, p-toluenesulfonyl, C₁₋₁₀ alkylsilyl (such as trimethylsilyl (TMS or -Si(CH₃)₃)), triethylsilyl, triisopropylsilyl, MMTr, DMTr or 4',4',4'-trimethoxytrityl, and the like.

The term "amino-protecting group" refers to a labile chemical moiety that protects an amino group from undesirable reactions during a synthetic procedure. Following the synthetic procedure(s), the amino-protecting group, as described herein, can be selectively removed. Amino-protecting groups known in the art are generally described in T.H. Greene and P.G.M. Wuts, Protective Groups in Organic Synthesis, 3rd ed., John Wiley & Sons, New York (1999). Examples of amino-protecting groups include, but are not limited to, acetyl, tert-butoxycarbonyl, 9-fluorenylmethoxycarbonyl, and benzyloxycarbonyl.

The term "solid support" specifically refers to any particle, bead, or surface on which oligonucleotide synthesis can occur. For example, both inorganic and organic solid supports can be used in embodiments of the present invention. Inorganic solid supports are preferably selected from silica gel and controlled-pore glass (CPG) beads. Organic solid supports are resins, preferably macroporous resins, and more preferably highly cross-linked polystyrene, Tentagel (a graft copolymer composed of a low-cross-linked polystyrene matrix onto which polyethylene glycol (PEG or POE) is grafted), polyvinyl acetate (PVA), Poros-polystyrene/divinylbenzene copolymers, aminopolyethylene glycol, and cellulose, and the like. Preferred embodiments of the present invention utilize a CPG-based solid support. Many other commercially available solid supports are also encompassed by the present invention.

Unless otherwise specified, A₁, A₂, A₃, A₄, A₅, C₁, C₂, C₃, C₄, B₁, B₂, B₃, B₄, B₅, C₅, Y₁, Y₂, Y₃, Y₄, Y₅, L₁, L₂, F₁, F₂, F₃, L₃, D₁, D₂ and D₃ in the general formulas of the present invention are divalent linking groups; Ring A is a trivalent linking group.

When a Markush variable in each general formula of the present invention is required to function as a linking group, the group listed for that Markush variable should be understood as a linking group. For example, A₁, A₂, A₃, A₄, A₅, C₁, C₂, C₃, C₄, B₁, B₂, B₃, B₄, B₅, C₅, Y₁, Y₂, Y₃, Y₄, Y₅, L₁, L₂, F₁, F₂, F₃, L₃, D₁, D₂ and D₃ in the general formula of the present application should be understood as linking groups, and the linking groups involved, such as "cycloalkyl", "heterocyclyl", "heteroalkyl", "alkenyl" and "alkynyl" should respectively represent divalent linking groups such as "cycloalkylene", "heterocyclylene", "heteroalkylene", "alkenylene" and "alkynylene".

### Description of the drawings:

Figure 1 shows a graph illustrating the inhibitory effects of the siRNA conjugates DAW30221, DAW30222, and DAW30223 of the present invention and the control conjugate DAW30025 on HBsAg in a Tg/HBV mouse model.
Figure 2 shows a graph illustrating the AGT knockdown effect of the siRNA conjugate DAW30255 of the present invention in a mouse model.

### DETAILED DESCRIPTION OF COMPOUNDS OF THE INVENTION

The present invention provides a novel targeting compound that can be used to prepare a nucleic acid drug for targeted delivery of oligonucleotides to the liver. The present invention also provides a nucleotide conjugate, a method for its preparation, and its use. This nucleotide conjugate exhibits high *in vivo* delivery efficiency, good stability, high gene expression inhibition activity in hepatocytes, and/or low toxicity. In another aspect, the present invention further provides an siRNA reagent, a method for its preparation, and its use. This siRNA reagent exhibits high in vivo delivery efficiency, good stability, high gene expression inhibition activity in HBV, and/or low toxicity.

In one aspect, the present invention relates to a compound having a structure as shown in Formula (I), or a stereoisomer, tautomer, or acceptable salt of the compound of Formula (I).

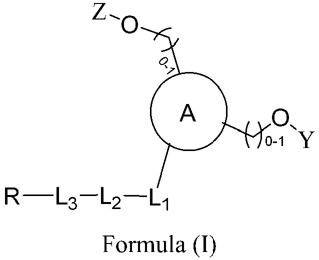

Wherein, each of Z, Y, Ring A, L₁, L₂, L₃ and R has the meanings as defined herein.

In some embodiments of the compounds of the present invention, Ring A is a heterocyclic group consisting of 3-12 ring atoms or a cycloalkyl group consisting of 3-12 ring atoms.

In some embodiments of the compounds of the present invention, Z and Y are each independently H, deuterium, a hydroxyl protecting group, HOC(=O)(CH₂)ⱼC(=O)-, or M-C(=O)(CH₂)ⱼC(=O)-, wherein each of M and j has the meanings as defined herein.

In some embodiments of the compounds of the present invention, Z and Y are each independently H, deuterium, a hydroxyl protecting group, HOC(=O)(CH₂)ⱼC(=O)-, or M-C(=O)(CH₂)C(=O)-, wherein the hydroxyl protecting group is an ester protecting group, an alkoxyalkyl protecting group, a silyl protecting group, a substituted or unsubstituted aryl protecting group, or a substituted or unsubstituted arylalkyl protecting group, and each of M and j has the meanings described herein.

In some embodiments of the compounds of the present invention, each j is independently 1, 2, 3, 4 or 5.

In some embodiments of the compounds of the present invention, M is a solid support.

In some embodiments of the compounds of the present invention, M is a hydroxyl- or amino-functionalized solid support.

In some embodiments of the compounds of the present invention, M is a resin or CPG.

In some embodiments of the compounds of the present invention, M is a macroporous resin or CPG.

In some embodiments of the compounds of the present invention, M is an aminemethyl resin, a hydroxy resin, or CPG.

In some embodiments of the compounds of the present invention, M is an aminemethyl resin, a hydroxy resin, an aminoCPG, or a hydroxyCPG.

In some embodiments of the compounds of the present invention, M is an aminemethyl resin, a hydroxy resin, or a -NHCPG.

In some embodiments of the compounds of the present invention, each of L₁, L₂ and L₃ is independently C₁₋₁₂ alkylene, C₃₋₁₂ cycloalkyl, a heterocyclyl consisting of 3-12 ring atoms, C₁₋₁₂ heteroalkyl, C₂₋₁₂ alkenyl, C₂₋₁₂ alkynyl, -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each of the C₁₋₁₂ alkylene, C₃₋₁₂ cycloalkyl, a heterocyclyl consisting of 3-12 ring atoms, C₁₋₁₂ heteroalkyl, C₂₋₁₂ alkenyl, C₂₋₁₂ alkynyl are independently optionally substituted by 1, 2, 3 or 4 R^{W1}, and 1, 2, 3, 4 or 5 methylene groups in each of the C₁₋₁₂ alkylene, C₁₋₁₂ heteroalkyl, C₂₋₁₂ alkenyl and C₂₋₁₂ alkynyl are independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each R¹ has the meaning described in the present invention.

In some embodiments of the compounds of the present invention, R is a structure represented by the following Formula (a) or Formula (b): 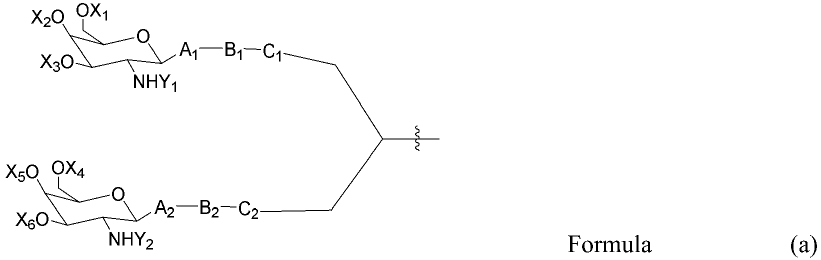 or 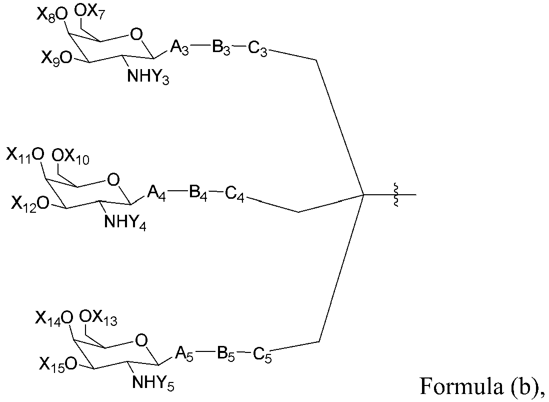 wherein each of X₁, X₂, X₃, X₄, X₅, X₆, X₇, X₈, X₉, X₁₀, X₁₁, X₁₂, X₁₃, X₁₄, X₁₅, Y₁, Y₂, Y₃, Y₄, Y₅, A₁, A₂, A₃, A₄, A₅, C₁, C₂, C₃, C₄ and C₅ has the meaning described in the present invention.

In some embodiments of the compounds of the present invention, wherein each of X₁, X₂, X₃, X₄, X₅, X₆, X₇, X₈, X₉, X₁₀, X₁₁, X₁₂, X₁₃, X₁₄ and X₁₅ is independently H or a hydroxyl protecting group.

In some embodiments of the compounds of the present invention, each of Y₁, Y₂, Y₃, Y₄ and Y₅ is independently H or an amino protecting group.

In some embodiments of the compounds of the present invention, each of A₁, A₂, A₃, A₄, A₅, C₁, C₂, C₃, C₄ and C₅ is independently a bond, C₁₋₁₂ alkylene, C₃₋₁₂ cycloalkyl, a heterocyclyl consisting of 3-12 ring atoms, C₁₋₁₂ heteroalkyl, C₂₋₁₂ alkenyl or C₂₋₁₂ alkynyl, wherein each of the C₁₋₁₂ alkylene, C₃₋₁₂ cycloalkyl, heterocyclyl consisting of 3-12 ring atoms, C₁₋₁₂ heteroalkyl, C₂₋₁₂ alkenyl and C₂₋₁₂ alkynyl groups is independently optionally substituted by 1, 2, 3 or 4 R^{W2}, and 1, 2, 3, 4 or 5 methylene groups in each of the C₁₋₁₂ alkylene, C₁₋₁₂ heteroalkyl, C₂₋₁₂ alkenyl and C₂₋₁₂ alkynyl groups are each independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each R¹ has the meaning described in the present invention.

In some embodiments of the compounds of the present invention, each of B₁, B₂, B₃, B₄ and B₅ is independently a C₃₋₁₂ cycloalkyl, a heterocyclyl consisting of 3-12 ring atoms, a C₁₋₁₂ alkylene, a C₁₋₁₂ heteroalkyl, a C₂₋₁₂ alkenyl or a C₂₋₁₂ alkynyl, wherein at least one of B₁ and B₂ is a C₃₋₁₂ cycloalkyl or a heterocyclyl consisting of 3-12 ring atoms, and at least one of B₃, B₄ and B₅ is a C₃₋₁₂ cycloalkyl or a heterocyclyl consisting of 3-12 ring atoms, each of the C₃₋₁₂ cycloalkyl, heterocyclyl consisting of 3-12 atoms, C₁₋₁₂ alkylene, C₁₋₁₂ heteroalkyl, C₂₋₁₂ alkenyl and C₂₋₁₂ alkynyl groups is independently optionally substituted by 1, 2, 3 or 4 R^{W3}, and 1, 2, 3, 4 or 5 methylene groups in each of the C₁₋₁₂ alkylene, C₁₋₁₂ heteroalkyl, C₂₋₁₂ alkenyl and C₂₋₁₂ alkynyl groups are independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each R¹ has the meaning described in the present invention.

In some embodiments of the compounds of the present invention, each of L₁, L₂ and L₃ is independently C₃₋₁₂ alkylene, C₃₋₆ cycloalkyl, a heterocyclic group consisting of 3-6 ring atoms, C₂₋₁₀ heteroalkyl, C₂₋₁₀ alkenyl, C₂₋₁₀ alkynyl, -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of the C₃₋₁₂ alkylene, C₃₋₆ cycloalkyl, heterocyclyl consisting of 3-6 ring atoms, C₂₋₁₀ heteroalkyl, C₂₋₁₀ alkenyl and C₂₋₁₀ alkynyl groups is independently optionally substituted by 1, 2, 3 or 4 R^{W1}, and 1, 2, 3, 4 or 5 methylene groups in each of the C₃₋₁₂ alkylene, C₂₋₁₀ heteroalkyl, C₂₋₁₀ alkenyl and C₂₋₁₀ alkynyl groups are independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each of R¹ and R^{W1} has the meanings described in the present invention.

In some embodiments of the compounds of the present invention, each of L₁, L₂ and L₃ is independently C₃₋₁₀ alkylene, cyclopropyl, cyclobutyl, cyclopentyl, cyclohexyl, a heterocyclyl consisting of 3-6 ring atoms, C₂₋₈ heteroalkyl, C₂₋₈ alkenyl, C₂₋₈ alkynyl, -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of the C₃₋₁₀ alkylene, cyclopropyl, cyclobutyl, cyclopentyl, cyclohexyl, a heterocyclyl consisting of 3-6 ring atoms, C₂₋₈ heteroalkyl, C₂₋₈ alkenyl and C₂₋₈ alkynyl groups is independently optionally substituted by 1, 2, 3 or 4 R^{W1}, and 1, 2, 3, 4 or 5 methylene groups in each of the C₃₋₁₀ alkylene, C₂₋₈ heteroalkyl, C₂₋₈ alkenyl and C₂₋₈ alkynyl groups are independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each of R¹ and R^{W1} has the meanings described in the present invention.

In some embodiments of the compounds of the present invention, each of L₁, L₂ and L₃ is independently C₃₋₈ alkylene, cyclopropyl, cyclobutyl, cyclopentyl, cyclohexyl, a heterocyclyl consisting of 3-6 ring atoms, C₂₋₆ heteroalkyl, C₂₋₆ alkenyl, C₂₋₆ alkynyl, -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of the C₃₋₈ alkylene, cyclopropyl, cyclobutyl, cyclopentyl, cyclohexyl, a heterocyclyl consisting of 3-6 ring atoms, C₂₋₆ heteroalkyl, C₂₋₆ alkenyl and C₂₋₆ alkynyl groups is independently optionally substituted by 1, 2, 3 or 4 R^{W1}, and 1, 2, 3, 4 or 5 methylene groups in each of the C₃₋₈ alkylene, C₂₋₆ heteroalkyl, C₂₋₆ alkenyl and C₂₋₆ alkynyl groups are independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each R¹ and R^{W1} have the meanings described in the present invention.

In some embodiments of the compounds of the present invention, each of L₁, L₂ and L₃ is independently C₃₋₆ alkylene, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, C₂₋₄ heteroalkyl, C₂₋₄ alkenyl, C₂₋₄ alkynyl, -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of the C₃₋₆ alkylene, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, C₂₋₄ heteroalkyl, C₂₋₄ alkenyl and C₂₋₄ alkynyl groups is independently optionally substituted with 1, 2, 3 or 4 R^{W1}, and 1, 2, 3, 4 or 5 methylene groups in each of the C₃₋₆ alkylene, C₂₋₄ heteroalkyl, C₂₋₄ alkenyl and C₂₋₄ alkynyl groups are independently optionally replaced with 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each R¹ and R^{W1} have the meanings described in the present invention.

In some embodiments of the compounds of the present invention, each of L₁, L₂ and L₃ is independently -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, C₂₋₄ heteroalkyl, C₂₋₄ alkenyl, C₂₋₄ alkynyl, , -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, C₂₋₄ heteroalkyl, C₂₋₄ alkenyl and C₂₋₄ alkynyl groups is independently optionally substituted with 1, 2, 3 or 4 R^{W1}, and 1, 2, 3, 4 or 5 methylene groups in each of -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, C₂₋₄ heteroalkyl, C₂₋₄ alkenyl and C₂₋₄ alkynyl are each independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each of R¹ and R^{W1} has the meanings described in the present invention.

In some embodiments of the compounds of the present invention, each of L₁, L₂ and L₃ is independently -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, C₂₋₁₀ heteroalkyl, C₂₋₁₀ alkenyl, C₂₋₁₀ alkynyl, , -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, C₂₋₁₀ heteroalkyl, C₂₋₁₀ alkenyl and C₂₋₁₀ alkynyl groups is independently optionally substituted with 1, 2, 3 or 4 R^{W1}, and 1, 2, 3, 4 or 5 methylene groups in each of -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, C₂₋₁₀ heteroalkyl, C₂₋₁₀ alkenyl and C₂₋₁₀ alkynyl are each independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each of R¹ and R^{W1} has the meaning described in the present invention.

In some embodiments of the compounds of the present invention, each of A₁, A₂, A₃, A₄, A₅, C₁, C₂, C₃, C₄ and C₅ is independently a bond, C₃₋₁₂ alkylene, C₃₋₆ cycloalkyl, a heterocyclic group consisting of 3-6 ring atoms, C₂₋₁₀ heteroalkyl, C₂₋₁₀ alkenyl or C₂₋₁₀ alkynyl, wherein each of the C₃₋₁₂ alkylene, C₃₋₆ cycloalkyl, heterocyclyl consisting of 3-6 ring atoms, C₂₋₁₀ heteroalkyl, C₂₋₁₀ alkenyl and C₂₋₁₀ alkynyl groups is independently optionally substituted by 1, 2, 3 or 4 R^{W2}, and 1, 2, 3, 4 or 5 methylene groups in each of the C₃₋₁₂ alkylene, C₂₋₁₀ heteroalkyl, C₂₋₁₀ alkenyl and C₂₋₁₀ alkynyl groups are each independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each of R¹ and R^{W2} has the meaning described in the present invention.

In some embodiments of the compounds of the present invention, each of A₁, A₂, A₃, A₄, A₅, C₁, C₂, C₃, C₄ and C₅ is independently a bond, C₃₋₁₀ alkylene, cyclopropyl, cyclobutyl, cyclopentyl, cyclohexyl, a heterocyclic group consisting of 3-6 ring atoms, C₂₋₈ heteroalkyl, C₂₋₈ alkenyl and C₂₋₈ alkynyl, wherein each of the C₃₋₁₀ alkylene, cyclopropyl, cyclobutyl, cyclopentyl, cyclohexyl, a heterocyclic group consisting of 3-6 ring atoms, C₂₋₈ heteroalkyl, C₂₋₈ alkenyl and C₂₋₈ alkynyl groups is independently optionally substituted by 1, 2, 3 or 4 R^{W2}, and 1, 2, 3, 4 or 5 methylene groups in each of the C₃₋₁₀ alkylene, C₂₋₈ heteroalkyl, C₂₋₈ alkenyl and C₂₋₈ alkynyl groups are each independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each of R¹ and R^{W2} has the meaning described in the present invention.

In some embodiments of the compounds of the present invention, each of A₁, A₂, A₃, A₄, A₅, C₁, C₂, C₃, C₄ and C₅ is independently a bond, C₃₋₈ alkylene, cyclopropyl, cyclobutyl, cyclopentyl, cyclohexyl, a heterocyclic group consisting of 3-6 ring atoms, C₂₋₆ heteroalkyl, C₂₋₆ alkenyl, C₂₋₆ alkynyl, -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of the C₃₋₈ alkylene, cyclopropyl, cyclobutyl, cyclopentyl, cyclohexyl, a heterocyclic group consisting of 3-6 ring atoms, C₂₋₆ heteroalkyl, C₂₋₆ alkenyl and C₂₋₆ alkynyl groups is independently optionally substituted by 1, 2, 3 or 4 R^{W2}, and 1, 2, 3, 4 or 5 methylene groups in each of the C₃₋₈ alkylene, C₂₋₆ heteroalkyl, C₂₋₆ alkenyl and C₂₋₆ alkynyl groups are each independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each of R¹ and R^{W2} has the meaning described in the present invention.

In some embodiments of the compounds of the present invention, each of A₁, A₂, A₃, A₄, A₅, C₁, C₂, C₃, C₄ and C₅ is independently a bond, C₃₋₆ alkylene, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, C₂₋₄ heteroalkyl, C₂₋₄ alkenyl and C₂₋₄ alkynyl, -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of the C₃₋₆ alkylene, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, C₂₋₄ heteroalkyl, C₂₋₄ alkenyl and C₂₋₄ alkynyl groups is independently optionally substituted by 1, 2, 3 or 4 R^{W2}, and 1, 2, 3, 4 or 5 methylene groups in each of the C₃₋₆ alkylene, C₂₋₄ heteroalkyl, C₂₋₄ alkenyl and C₂₋₄ alkynyl groups are each independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each of R¹ and R^{W2} has the meaning described in the present invention.

In some embodiments of the compounds of the present invention, each of A₁, A₂, A₃, A₄, A₅, C₁, C₂, C₃, C₄ and C₅ is independently a bond, -(CH₂)₃-, -(CH₂)₄-, -(CH₂)₅-, -(CH₂)₆-, -(CH₂)₇-, -(CH₂)₈-, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, -O(CH₂)₄NHCH₂-, -O(CH₂)₃NHCH₂-, -O(CH₂)₅NHCH₂-, -O(CH₂)₄NH-, -O(CH₂)₃NH-, -O(CH₂)₅NH-, -CH₂O(CH₂)₄NH-, -CH₂O(CH₂)₃NH-, -CH₂O(CH₂)₂NH-, C₂₋₆ alkenyl, C₂₋₆ alkynyl, -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of the -(CH₂)₃-, -(CH₂)₄-, -(CH₂)₅-, -(CH₂)₆-, -(CH₂)₇-, -(CH₂)₈-, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, -O(CH₂)₄NHCH₂-, -O(CH₂)₃NHCH₂-, -O(CH₂)₅NHCH₂-, -O(CH₂)₄NH-, -O(CH₂)₃NH-, -O(CH₂)₅NH-, -CH₂O(CH₂)₄NH-, -CH₂O(CH₂)₃NH-, -CH₂O(CH₂)₂NH-, C₂₋₆ alkenyl and C₂₋₆ alkynyl groups is independently optionally substituted by 1, 2, 3 or 4 R^{W2} , and 1, 2, 3, 4 or 5 methylene groups in each of the -(CH₂)₃-, -(CH₂)₄-, -(CH₂)₅-, -(CH₂)₆-, -(CH₂)₇-, -(CH₂)₈-, -O(CH₂)₄NHCH₂-, -O(CH₂)₃NHCH₂-, -O(CH₂)₅NHCH₂-, -O(CH₂)₄NH-, -O(CH₂)₃NH-, -O(CH₂)₅NH-, -CH₂O(CH₂)₄NH-, -CH₂O(CH₂)₃NH-, -CH₂O(CH₂)₂NH-, C₂₋₆ alkenyl and C₂₋₆ alkynyl groups are each independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each of R¹ and R^{W2} has the meaning described in the present invention.

In some embodiments of the compounds of the present invention, each of B₁, B₂, B₃, B₄ and B₅ is independently cyclopropyl, cyclobutyl, cyclopentyl, cyclohexyl, a heterocyclic group consisting of 3 to 6 ring atoms, C₁₋₆ alkylene, C₁₋₆ heteroalkyl, C₂₋₆ alkenyl or C₂₋₆ alkynyl, wherein at least one of B₁ and B₂ is a cyclopropyl, cyclobutyl, cyclopentyl, cyclohexyl or a heterocyclic group consisting of 3-6 ring atoms, at least one of B₃, B₄ and B₅ is a cyclopropyl, cyclobutyl, cyclopentyl, cyclohexyl or a heterocyclic group consisting of 3-6 ring atoms, each of the cyclopropyl, cyclobutyl, cyclopentyl, cyclohexyl, a heterocyclic group consisting of 3-6 ring atoms, C₁₋₆ alkylene, C₁₋₆ heteroalkyl, C₂₋₆ alkenyl and C₂₋₆ alkynyl groups is independently optionally substituted with 1, 2, 3 or 4 R^{W3}, and 1, 2, 3, 4 or 5 methylene groups in each of the C₁₋₆ alkylene, C₁₋₆ heteroalkyl, C₂₋₆ alkenyl and C₂₋₆ alkynyl groups are each independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each of R¹ and R^{W3} has the meaning described in the present invention.

In some embodiments of the compounds of the present invention, each of B₁, B₂, B₃, B₄ and B₅ is independently cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, C₁₋₄ alkylene, C₁₋₄ heteroalkyl, C₂₋₄ alkenyl or C₂₋₄ alkynyl, wherein at least one of B₁ and B₂ is cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl, at least one of B₃, B₄ and B₅ is cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl, and each of the cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, C₁₋₄ alkylene, C₁₋₄ heteroalkyl, C₂₋₄ alkenyl and C₂₋₄ alkynyl is independently optionally substituted with 1, 2, 3 or 4 R^{W3}, and 1, 2, 3, 4 or 5 methylene groups in each of the C₁₋₄ alkylene, C₁₋₄ heteroalkyl, C₂₋₄ alkenyl and C₂₋₄ alkynyl groups are each independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each of R¹ and R^{W3} has the meanings described in the present invention.

In some embodiments of the compounds of the present invention, Z and Y are each independently H, deuterium, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, p-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methylsulfonyl, p-toluenesulfonyl, C₁₋₁₂ alkyl, C₁₋₁₂ alkylC(=O)-, C₁₋₁₂ alkylmethyl, C₁₋₁₂ alkylsilyl, C₆₋₁₀ arylC₁₋₆ alkyl, HOC(=O)(CH₂)ⱼC(=O)-, M-C(=O)(CH₂)ⱼC(=O)-, triphenylmethyl, MMTr, DMTr or 4',4',4'-trimethoxytrityl, wherein each of M and j has the meaning described in the present invention.

In some embodiments of the compounds of the present invention, Z and Y are each independently H, deuterium, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, p-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methylsulfonyl, p-toluenesulfonyl, C₁₋₁₀ alkyl, C₁₋₁₀ alkylC(=O)-, C₁₋₁₀ alkylmethyl, C₁₋₁₀ alkylsilyl, C₆₋₁₀ arylC₁₋₄ alkyl, HOC(=O)(CH₂)ⱼC(=O)-, M-C(=O)(CH₂)ⱼC(=O)-, triphenylmethyl, MMTr, DMTr or 4',4',4'-trimethoxytrityl, wherein each of M and j has the meaning described in the present invention.

In some embodiments of the compounds of the present invention, Z and Y are each independently H, deuterium, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, p-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methylsulfonyl, p-toluenesulfonyl, C₁₋₈ alkyl, C₁₋₈ alkylC(=O)-, C₁₋₈ alkylmethyl, C₁₋₈ alkylsilyl, phenylC₁₋₃ alkyl, HOC(=O)(CH₂)ⱼC(=O)-, M-C(=O)(CH₂)ⱼC(=O)-, triphenylmethyl, MMTr, DMTr or 4',4',4'-trimethoxytrityl, wherein each of M and j has the meaning described in the present invention.

In some embodiments of the compounds of the present invention, Z and Y are each independently H, deuterium, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, p-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methylsulfonyl, p-toluenesulfonyl, C₁₋₆ alkyl, C₁₋₆ alkylC(=O)-, C₁₋₆ alkylmethyl, C₁₋₆ alkylsilyl, benzyl, phenethyl, HOC(=O)(CH₂)ⱼC(=O)-, M-C(=O)(CH₂)ⱼC(=O)-, triphenylmethyl, MMTr, DMTr or 4',4',4'-trimethoxytrityl, wherein each of M and j has the meaning described in the present invention.

In some embodiments of the compounds of the present invention, Z and Y are each independently H, deuterium, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, p-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methylsulfonyl, p-toluenesulfonyl, methyl, ethyl, n-propyl, isopropyl, n-butyl, isobutyl, methyl C(=O)-, ethyl C(=O)-, n-propyl C(=O)-, isopropyl C(=O)-, n-butyl C(=O)-, isobutyl C(=O)-, tert-butyl C(=O)-, C₁₋₄ alkylmethyl, C₁₋₄ alkylsilyl, phenylmethyl, phenylethyl, HOC(=O)(CH₂)ⱼC(=O)-, M-C(=O)(CH₂)ⱼC(=O)-, triphenylmethyl, MMTr, DMTr or 4',4',4'-trimethoxytrityl, wherein each of M and j has the meaning described in the present invention.

In some embodiments of the compounds of the present invention, Ring A is a heterocyclic group consisting of 3-7 ring atoms or a cycloalkyl group consisting of 3-6 ring atoms.

In some embodiments of the compounds of the present invention, Ring A is a heterocyclic group consisting of 3-6 ring atoms or a cycloalkyl group consisting of 3-6 ring atoms.

In some embodiments of the compounds of the present invention, Ring A is cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl.

In some embodiments of the compounds of the present invention, each of X₁, X₂, X₃, X₄, X₅, X₆, X₇, X₈, X₉, X₁₀, X₁₁, X₁₂, X₁₃, X₁₄ and X₁₅ is independently H, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, methyl, tert-butyl, 2-trimethylsilylethyl, 1,1-dimethyl-2-propenyl, 3-methyl-3-butenyl, allyl, benzyl, p-methoxybenzyldiphenylmethyl, trityl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, p-toluenesulfonyl, trimethylsilyl, triethylsilyl, triisopropylsilyl, C₁₋₆ alkoxy, or R²C(=O)-, wherein R² has the meaning described in the present invention.

In some embodiments of the compounds of the present invention, each of Y₁, Y₂, Y₃, Y₄, and Y₅ is independently H, 9-fluorenylmethoxycarbonyl or R³C(=O)-, wherein R³ has the meaning described in the present invention.

In some embodiments of the compounds of the present invention, each of R² and R³ is independently C₁₋₆ alkyl, halo-substituted C₁₋₆ alkyl, C₁₋₆ alkoxy, phenyl, halogen-substituted phenyl, C₁₋₆ alkylphenyl or benzyl.

In some embodiments of the compounds of the present invention, each of R² and R³ is independently C₁₋₄ alkyl, halo-substituted C₁₋₆ alkyl, C₁₋₄ alkoxy, phenyl, halogen-substituted phenyl, C₁₋₄ alkylphenyl or benzyl.

In some embodiments of the compounds of the present invention, each of R² and R³ is independently methyl, ethyl, n-propyl, isopropyl, trifluoromethyl, difluoromethyl, monofluoromethyl, trichloromethyl, dichloromethyl, monochloromethyl, 2,2,2-trichloroethyl, methoxy, ethoxy, 1-propoxy, 2-propoxy, 1-butoxy, tert-butoxy, 2-methyl-1-propoxy, 2-butoxy, phenyl, halogen-substituted phenyl, C₁₋₃ alkylphenyl or benzyl.

In some embodiments of the compounds of the present invention, each R¹ is independently H, deuterium or C₁₋₆ alkyl.

In some embodiments of the compounds of the present invention, each R¹ is independently H, deuterium or C₁₋₄ alkyl.

In some embodiments of the compounds of the present invention, each R¹ is independently H, deuterium, methyl, ethyl, n-propyl, isopropyl, n-butyl or isobutyl.

In some embodiments of the compounds of the present invention, each of R^{W1}, R^{W2}, and R^{W3} is independently deuterium, =O, hydroxy, nitro, cyano, F, Cl, Br, I, C₁₋₆ alkyl, C₁₋₆ alkoxy, C₃₋₆ cycloalkyl, C₂₋₆ alkenyl or C₁₋₆ heteroalkyl.

In some embodiments of the compounds of the present invention, each of R^{W1}, R^{W2}, and R^{W3} is independently deuterium, =O, hydroxy, nitro, cyano, F, Cl, Br, I, C₁₋₄ alkyl, C₁₋₄ alkoxy, cyclopropyl, cyclobutyl, cyclopentyl, cyclohexyl, C₂₋₄ alkenyl or C₁₋₄ heteroalkyl.

In some embodiments of the compounds of the present invention, each of R^{W1}, R^{W2}, and R^{W3} is independently deuterium, =O, hydroxy, nitro, cyano, F, Cl, Br, I, methyl, ethyl, n-propyl, isopropyl, n-butyl, isobutyl, methoxy, ethoxy, 1-propoxy, 2-propoxy, 1-butoxy, 2-methyl-1-propoxy, 2-butoxy, cyclopropyl, cyclobutyl, cyclopentyl, cyclohexyl, C₂₋₄ alkenyl or C₁₋₄ heteroalkyl.

In some embodiments of the compounds of the present invention, R is a structure represented by the following Formula (a-1) or Formula (b-1): 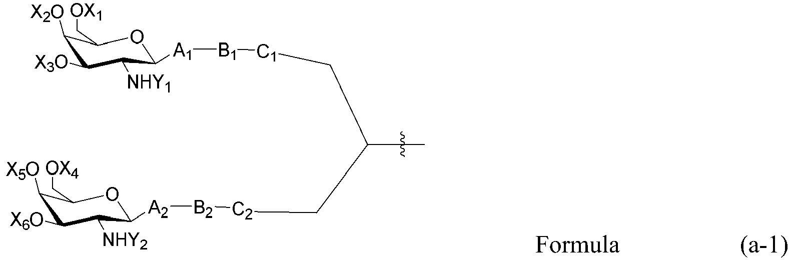 or 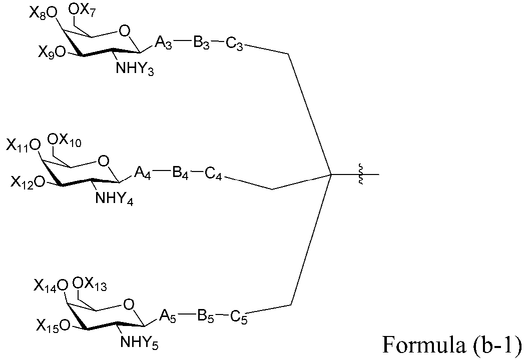 wherein each of X₁, X₂, X₃, X₄, X₅, X₆, X₇, X₈, X₉, X₁₀, X₁₁, X₁₂, X₁₃, X₁₄, X₁₅, Y₁, Y₂, Y₃, Y₄, Y₅, A₁, A₂, A₃, A₄, A₅, B₁, B₂, B₃, B₄, B₅, C₁, C₂, C₃, C₄ and C₅ has the meaning as defined in the present invention.

In some embodiments, the present invention provides a compound having the structure represented by Formula (II), or a stereoisomer, tautomer, or acceptable salt of the compound represented by Formula (II), 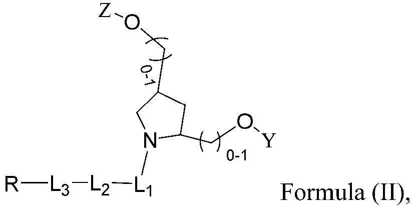 wherein each of Z, Y, L₁, L₂, L₃ and R has the meaning described in the present invention.

In some embodiments, the present invention provides a compound having the structure represented by Formula (II-1), or a stereoisomer, tautomer, or acceptable salt of the compound represented by Formula (II-1), 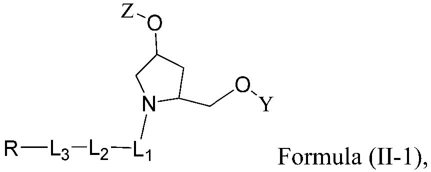 wherein each of Z, Y, L₁, L₂, L₃ and R has the meaning described in the present invention.

In some embodiments, the present invention provides a compound having the structure represented by Formula (II-2), or a stereoisomer, tautomer, or acceptable salt of the compound represented by Formula (II-2), 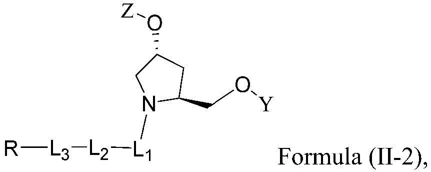 wherein each of Z, Y, L₁, L₂, L₃ and R has the meaning described in the present invention.

In some embodiments, the present invention provides a compound having the structure represented by Formula (III), or a stereoisomer, tautomer, or acceptable salt of the compound represented by Formula (III), 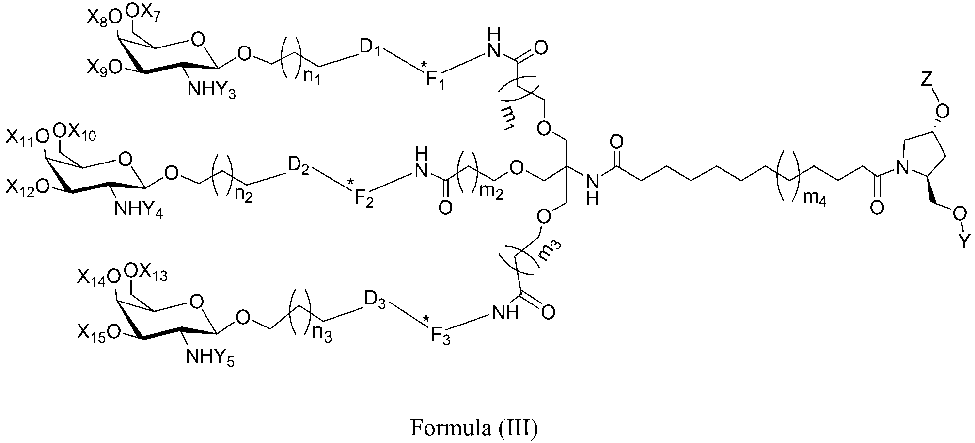 wherein each of D₁, D₂ and D₃ is independently -C(=O)-*, -C(=O)O-*, -OC(=O)-*, -C(=O)N(R¹)-*, -N(R¹)C(=O)-*, -C(=O)N(R¹)CH₂-* or -N(R¹)C(=O)CH₂-*;
each of F₁, F₂, and F₃ is independently cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl;
each of n₁, n₂, n₃, m₁, m₂, m₃ and m₄ is independently 0, 1, 2, 3 or 4;
wherein each of R¹, X₇, X₈, X₉, X₁₀, X₁₁, X₁₂, X₁₃, X₁₄, X₁₅, Y₃, Y₄, Y₅, Z and Y has the meaning as defined in the present invention.

In some embodiments of the present invention, the present invention comprises one of the compounds shown below, or a stereoisomer, tautomer, or acceptable salt thereof,

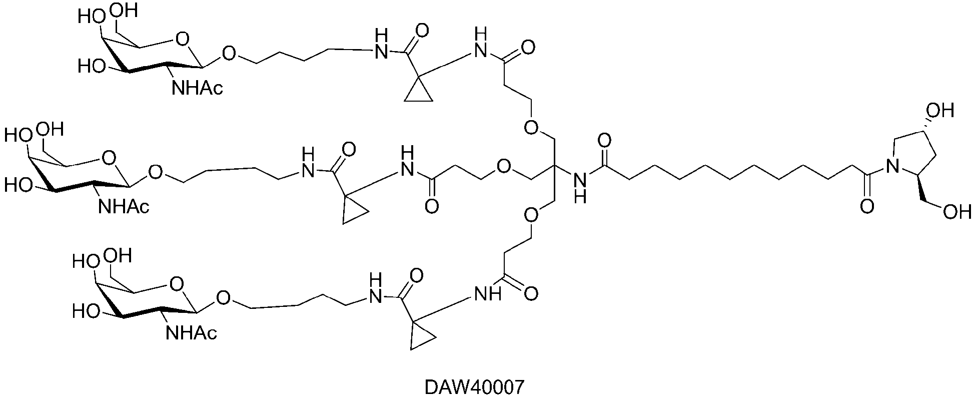

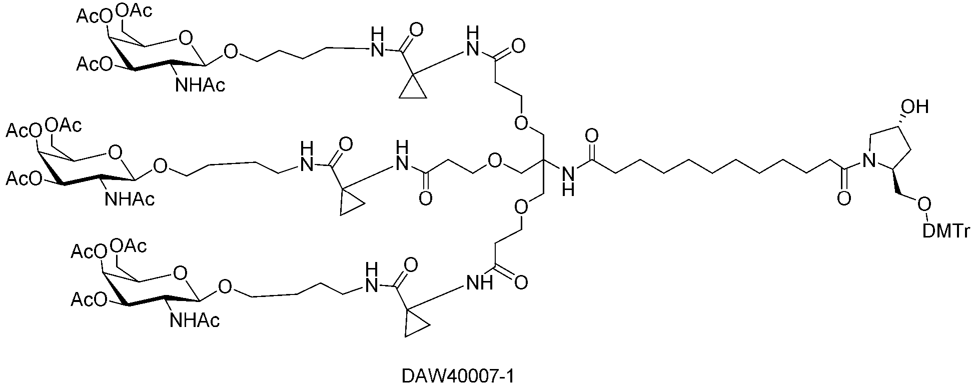

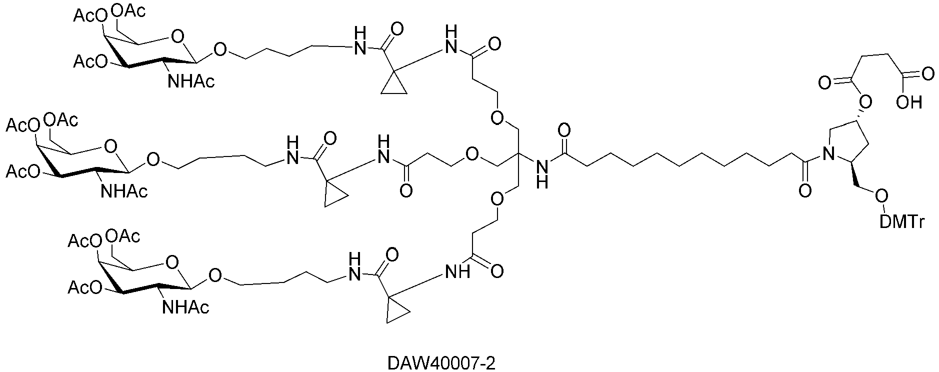

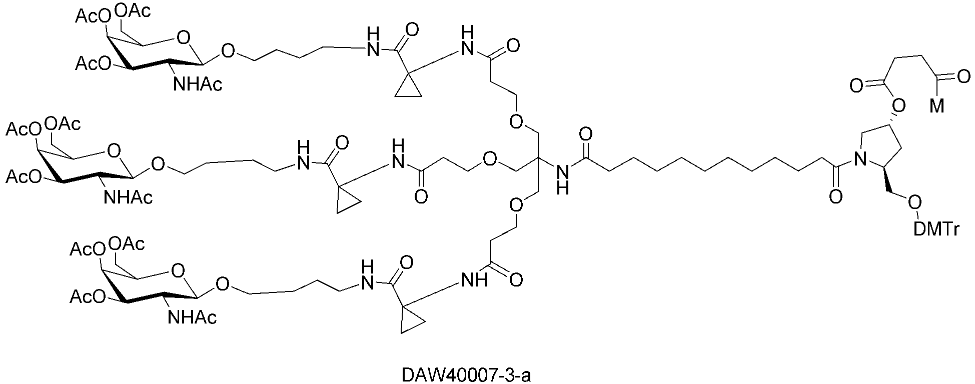

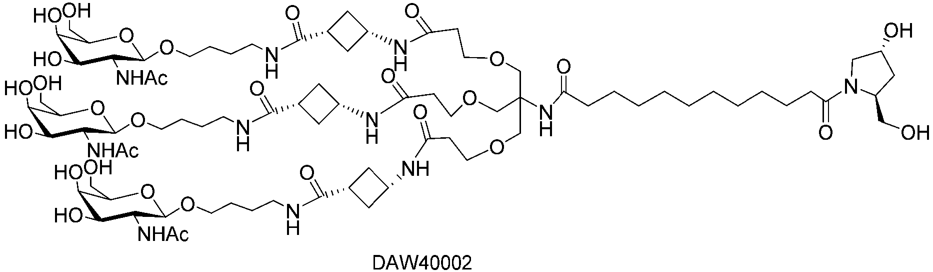

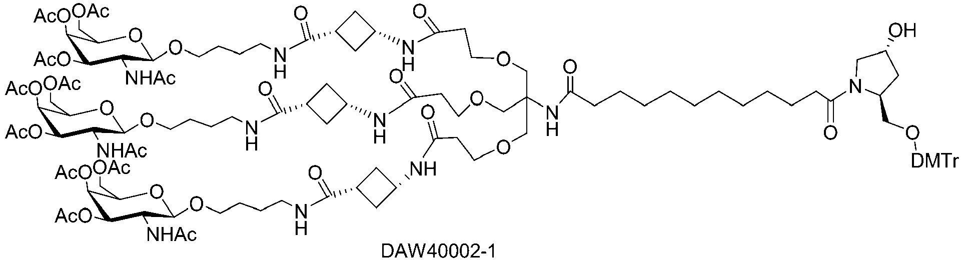

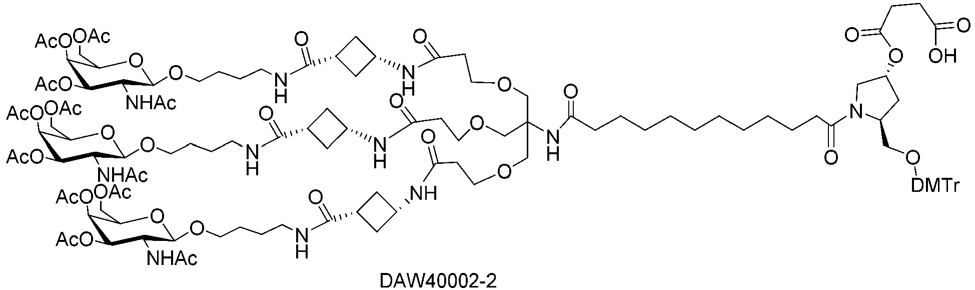

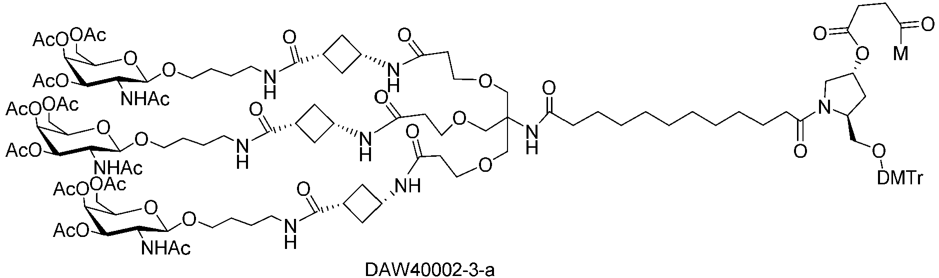

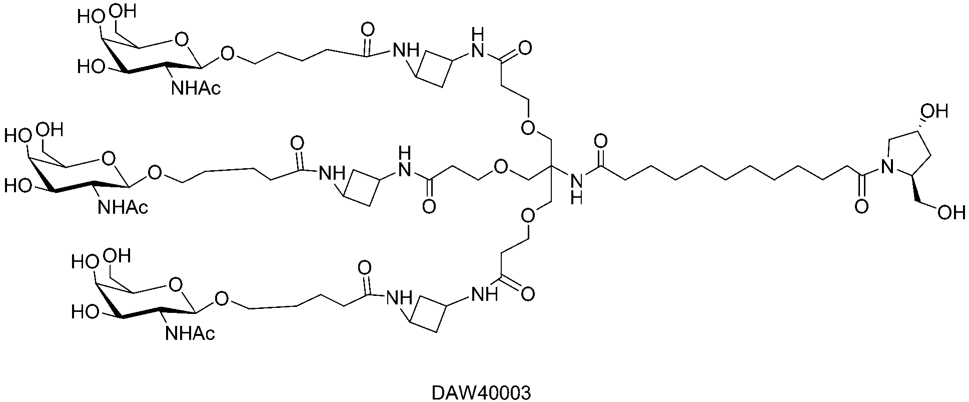

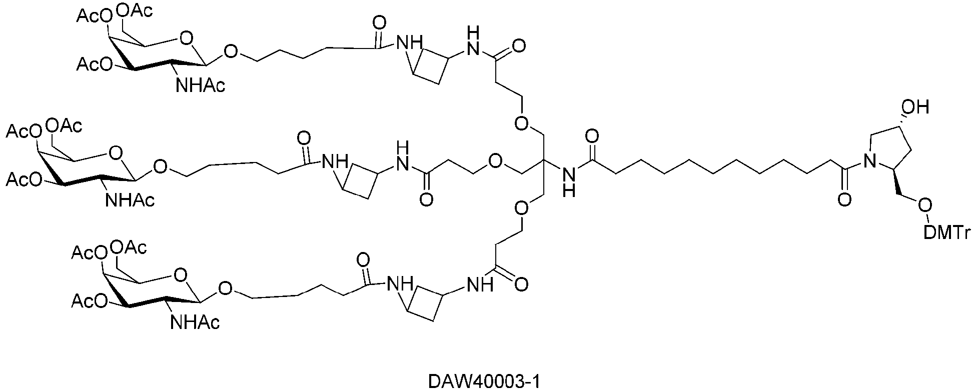

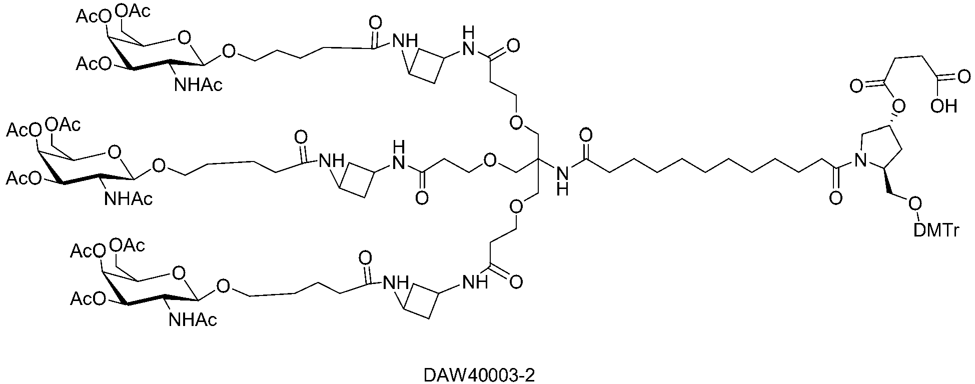

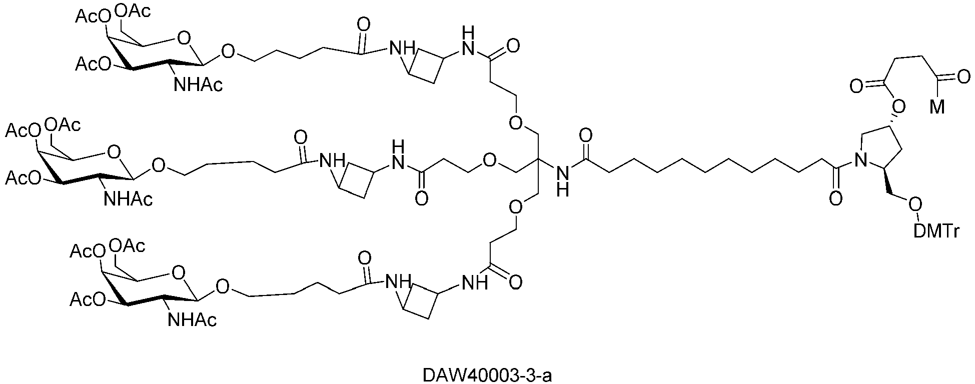

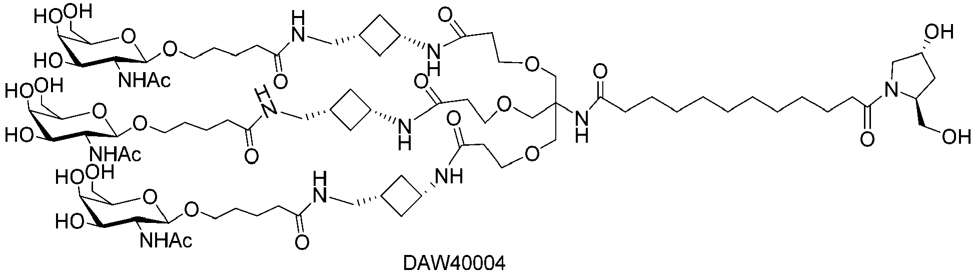

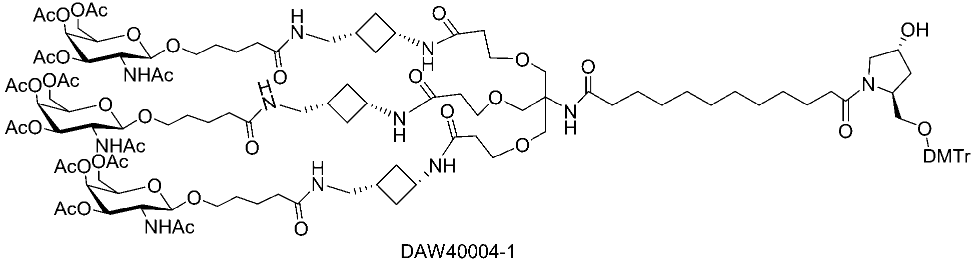

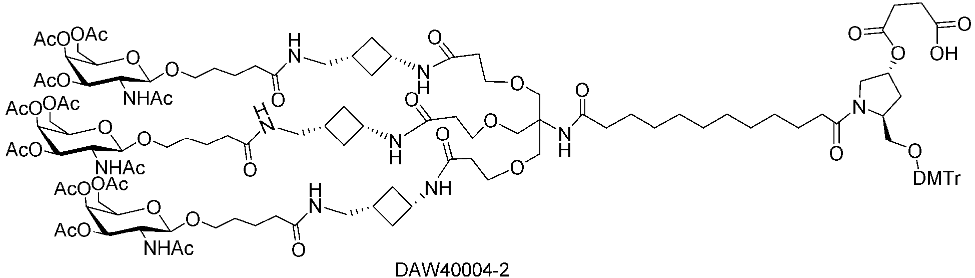

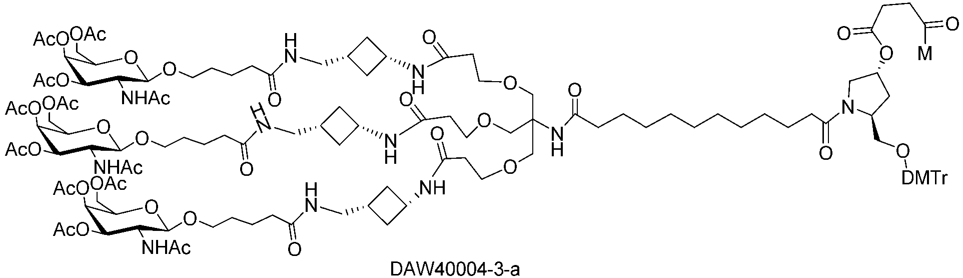

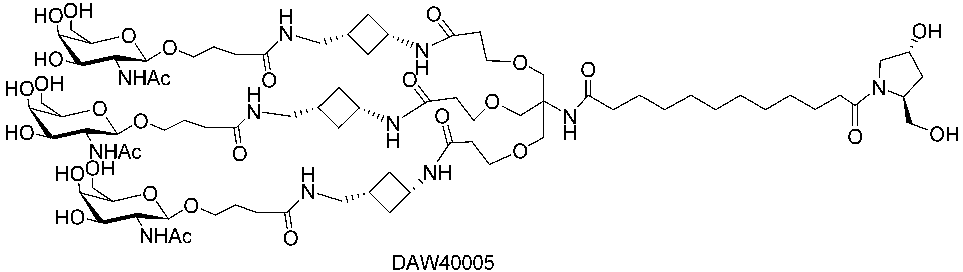

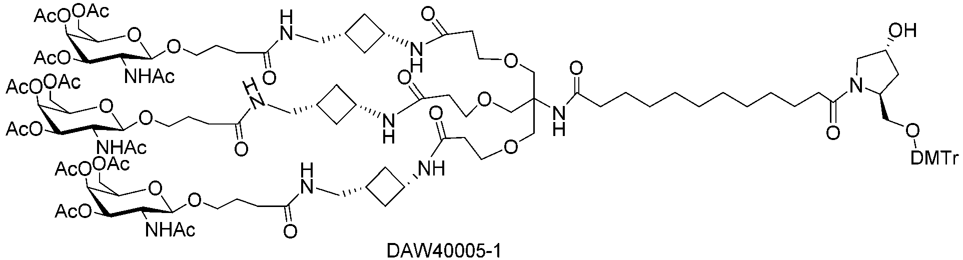

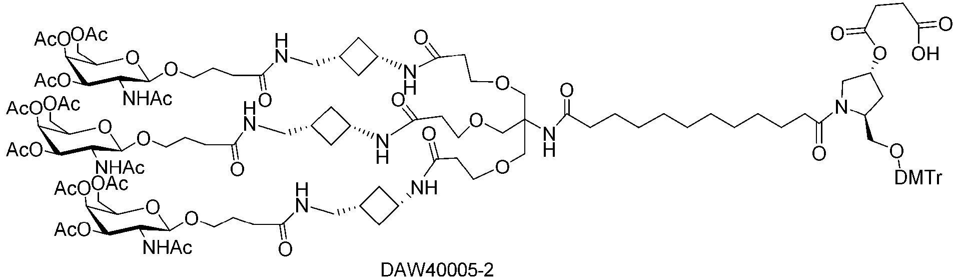

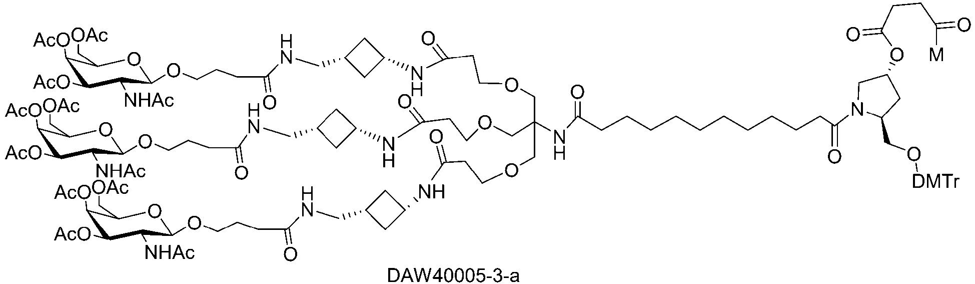

M is a solid support, preferably a hydroxyl- or amino-functionalized solid support, more preferably a resin or CPG, further preferably a macroporous resin or CPG, further preferably an aminomethyl resin, a hydroxy resin, an aminoCPG, or a hydroxyCPG; further preferably an aminomethyl resin, a hydroxy resin or -NHCPG.

In another aspect, the present invention relates to a conjugated group comprising a structure represented by Formula (I-a), or a stereoisomer, tautomer, or acceptable salt of the structure represented by Formula (I-a), 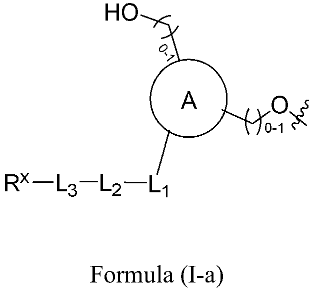 wherein each of ring A, L₁, L₂, L₃ and R^{x} has the meaning as defined in the present invention.

In some embodiments of the conjugated group of the present invention, R^{x} is a structure of Formula (c) or Formula (d): 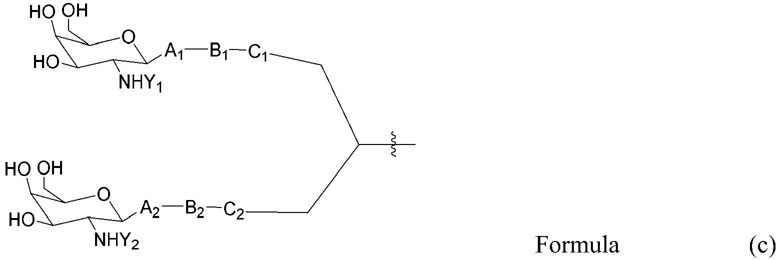 or 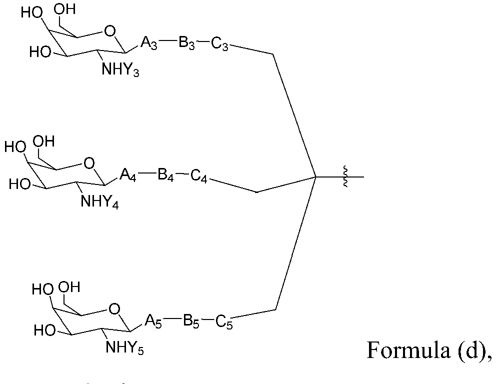 each of A₁, A₂, A₃, A₄, A₅, B₁, B₂, B₃, B₄, B₅, C₁, C₂, C₃, C₄, C₅, Y₁, Y₂, Y₃, Y₄ and Y₅ has the meaning described in the present invention.

In some embodiments of the conjugated groups of the present invention, Ring A is a heterocyclyl group consisting of 3-12 ring atoms or a cycloalkyl group consisting of 3-12 ring atoms.

In some embodiments of the conjugated groups of the present invention, Ring A is a heterocyclyl group consisting of 3-7 ring atoms or a cycloalkyl group consisting of 3-6 ring atoms.

In some embodiments of the conjugated groups of the present invention, Ring A is a heterocyclyl group consisting of 3-6 ring atoms or a cycloalkyl group consisting of 3-6 ring atoms.

In some embodiments of the conjugated groups of the present invention, the ring A is cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl.

In some embodiments of the conjugated groups of the present invention, each of L₁, L₂ and L₃ is independently C₁₋₁₂ alkylene, C₃₋₁₂ cycloalkyl, a heterocyclyl consisting of 3-12 ring atoms, C₁₋₁₂ heteroalkyl, C₂₋₁₂ alkenyl, C₂₋₁₂ alkynyl, -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of the C₁₋₁₂ alkylene, C₃₋₁₂ cycloalkyl, a heterocyclyl consisting of 3-12 ring atoms, C₁₋₁₂ heteroalkyl, C₂₋₁₂ alkenyl and C₂₋₁₂ alkynyl isindependently optionally substituted by 1, 2, 3 or 4 R^{W1}, and 1, 2, 3, 4 or 5 methylene groups in each of the C₁₋₁₂ alkylene, C₁₋₁₂ heteroalkyl, C₂₋₁₂ alkenyl and C₂₋₁₂ alkynyl are independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each R¹ has the meaning described in the present invention.

In some embodiments of the conjugated groups of the present invention, each of A₁, A₂, A₃, A₄, A₅, C₁, C₂, C₃, C₄ and C₅ is independently a bond, C₁₋₁₂ alkylene, C₃₋₁₂ cycloalkyl, a heterocyclyl consisting of 3-12 ring atoms, C₁₋₁₂ heteroalkyl, C₂₋₁₂ alkenyl or C₂₋₁₂ alkynyl, wherein each of C₁₋₁₂ alkylene, C₃₋₁₂ cycloalkyl, a heterocyclyl consisting of 3-12 ring atoms, C₁₋₁₂ heteroalkyl, C₂₋₁₂ alkenyl and C₂₋₁₂ alkynyl is independently optionally substituted by 1, 2, 3 or 4 R^{W2}, and 1, 2, 3, 4 or 5 methylene groups in each of the C₁₋₁₂ alkylene, C₁₋₁₂ heteroalkyl, C₂₋₁₂ alkenyl and C₂₋₁₂ alkynyl groups are independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each R¹ has the meaning described in the present invention.

In some embodiments of the conjugated groups of the present invention, each of B₁, B₂, B₃, B₄ and B₅ is independently a C₃₋₁₂ cycloalkyl, a heterocyclyl consisting of 3-12 ring atoms, a C₁₋₁₂ alkylene, a C₁₋₁₂ heteroalkyl, a C₂₋₁₂ alkenyl or a C₂₋₁₂ alkynyl, wherein at least one of B₁ and B₂ is a C₃₋₁₂ cycloalkyl or a heterocyclyl consisting of 3-12 ring atoms, and at least one of B₃, B₄ and B₅ is a C₃₋₁₂ cycloalkyl or a heterocyclyl consisting of 3-12 ring atoms, each of the C₃₋₁₂ cycloalkyl, heterocyclyl consisting of 3-12 atoms, C₁₋₁₂ alkylene, C₁₋₁₂ heteroalkyl, C₂₋₁₂ alkenyl and C₂₋₁₂ alkynyl groups is independently optionally substituted by 1, 2, 3 or 4 R^{W3}, and 1, 2, 3, 4 or 5 methylene groups in each of the C₁₋₁₂ alkylene, C₁₋₁₂ heteroalkyl, C₂₋₁₂ alkenyl and C₂₋₁₂ alkynyl groups are independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each R¹ has the meaning described in the present invention.

In some embodiments of the conjugated groups of the present invention, each of L₁, L₂ and L₃ is independently C₃₋₁₂ alkylene, C₃₋₆ cycloalkyl, a heterocyclyl consisting of 3-6 ring atoms, C₂₋₁₀ heteroalkyl, C₂₋₁₀ alkenyl, C₂₋₁₀ alkynyl, -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of the C₃₋₁₂ alkylene, C₃₋₆ cycloalkyl, a heterocyclyl consisting of 3-6 ring atoms, C₂₋₁₀ heteroalkyl, C₂₋₁₀ alkenyl and C₂₋₁₀ alkynyl is independently optionally substituted by 1, 2, 3 or 4 R^{W1}, and 1, 2, 3, 4 or 5 methylene groups in each of the C₃₋₁₂ alkylene, C₂₋₁₀ heteroalkyl, C₂₋₁₀ alkenyl and C₂₋₁₀ alkynyl are independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each of R¹ and R^{W1} has the meaning described in the present invention.

In some embodiments of the conjugated groups of the present invention, each of L₁, L₂ and L₃ is independently C₃₋₁₀ alkylene, cyclopropyl, cyclobutyl, cyclopentyl, cyclohexyl, a heterocyclyl consisting of 3-6 ring atoms, C₂₋₈ heteroalkyl, C₂₋₈ alkenyl, C₂₋₈ alkynyl, -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of the C₃₋₁₀ alkylene, cyclopropyl, cyclobutyl, cyclopentyl, cyclohexyl, a heterocyclyl consisting of 3-6 ring atoms, C₂₋₈ heteroalkyl, C₂₋₈ alkenyl and C₂₋₈ alkynyl is independently optionally substituted by 1, 2, 3 or 4 R^{W1}, and 1, 2, 3, 4 or 5 methylene groups in each of the C₃₋₁₀ alkylene, C₂₋₈ heteroalkyl, C₂₋₈ alkenyl and C₂₋₈ alkynyl are independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each of R¹ and R^{W1} has the meaning described in the present invention.

In some embodiments of the conjugated groups of the present invention, each of L₁, L₂ and L₃ is independently C₃₋₈ alkylene, cyclopropyl, cyclobutyl, cyclopentyl, cyclohexyl, a heterocyclyl consisting of 3-6 ring atoms, C₂₋₆ heteroalkyl, C₂₋₆ alkenyl, C₂₋₆ alkynyl, -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of the C₃₋₈ alkylene, cyclopropyl, cyclobutyl, cyclopentyl, cyclohexyl, a heterocyclyl consisting of 3-6 ring atoms, C₂₋₆ heteroalkyl, C₂₋₆ alkenyl and C₂₋₆ alkynyl is independently optionally substituted by 1, 2, 3 or 4 R^{W1}, and 1, 2, 3, 4 or 5 methylene groups in each of the C₃₋₈ alkylene, C₂₋₆ heteroalkyl, C₂₋₆ alkenyl and C₂₋₆ alkynyl are independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each of R¹ and R^{W1} has the meaning described in the present invention.

In some embodiments of the conjugated groups of the present invention, each of L₁, L₂ and L₃ is independently C₃₋₆ alkylene, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, C₂₋₄ heteroalkyl, C₂₋₄ alkenyl, C₂₋₄ alkynyl, -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of the C₃₋₆ alkylene, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, C₂₋₄ heteroalkyl, C₂₋₄ alkenyl and C₂₋₄ alkynyl is independently optionally substituted by 1, 2, 3 or 4 R^{W1}, and 1, 2, 3, 4 or 5 methylene groups in each of the C₃₋₆ alkylene, C₂₋₄ heteroalkyl, C₂₋₄ alkenyl and C₂₋₄ alkynyl are independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each of R¹ and R^{W1} has the meaning described in the present invention.

In some embodiments of the conjugated groups of the present invention, each of L₁, L₂ and L₃ is independently -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, C₂₋₄ heteroalkyl, C₂₋₄ alkenyl, C₂₋₄ alkynyl, -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of the -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, C₂₋₄ heteroalkyl, C₂₋₄ alkenyl and C₂₋₄ alkynyl is independently optionally substituted by 1, 2, 3 or 4 R^{W1}, and 1, 2, 3, 4 or 5 methylene groups in each of the -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, C₂₋₄ heteroalkyl, C₂₋₄ alkenyl and C₂₋₄ alkynyl are independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each of R¹ and R^{W1} has the meaning described in the present invention.

In some embodiments of the conjugated groups of the present invention, each of L₁, L₂ and L₃ is independently -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, C₂₋₁₀ heteroalkyl, C₂₋₁₀ alkenyl, C₂₋₁₀ alkynyl, -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of the -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, C₂₋₁₀ heteroalkyl, C₂₋₁₀ alkenyl and C₂₋₁₀ alkynyl is independently optionally substituted by 1, 2, 3 or 4 R^{W1}, and 1, 2, 3, 4 or 5 methylene groups in each of the -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, C₂₋₁₀ heteroalkyl, C₂₋₁₀ alkenyl and C₂₋₁₀ alkynyl are independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each of R¹ and R^{W1} has the meaning described in the present invention.

In some embodiments of the conjugated groups of the present invention, each of A₁, A₂, A₃, A₄, A₅, C₁, C₂, C₃, C₄ and C₅ is independently a bond, C₃₋₁₂ alkylene, C₃₋₆ cycloalkyl, a heterocyclyl consisting of 3-6 ring atoms, C₂₋₁₀ heteroalkyl, C₂₋₁₀ alkenyl or C₂₋₁₀ alkynyl, wherein each of C₃₋₁₂ alkylene, C₃₋₆ cycloalkyl, a heterocyclyl consisting of 3-6 ring atoms, C₂₋₁₀ heteroalkyl, C₂₋₁₀ alkenyl and C₂₋₁₀ alkynyl is independently optionally substituted by 1, 2, 3 or 4 R^{W2}, and 1, 2, 3, 4 or 5 methylene groups in each of the C₃₋₁₂ alkylene, C₂₋₁₀ heteroalkyl, C₂₋₁₀ alkenyl and C₂₋₁₀ alkynyl groups are independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each R¹ and R^{W2} has the meaning described in the present invention.

In some embodiments of the conjugated groups of the present invention, each of A₁, A₂, A₃, A₄, A₅, C₁, C₂, C₃, C₄ and C₅ is independently a bond, C₃₋₁₀ alkylene, cyclopropyl, cyclobutyl, cyclopentyl, cyclohexyl, a heterocyclyl consisting of 3-6 ring atoms, C₂₋₈ heteroalkyl, C₂₋₈ alkenyl or C₂₋₈ alkynyl, wherein each of C₃₋₁₀ alkylene, cyclopropyl, cyclobutyl, cyclopentyl, cyclohexyl, a heterocyclyl consisting of 3-6 ring atoms, C₂₋₈ heteroalkyl, C₂₋₈ alkenyl and C₂₋₈ alkynyl is independently optionally substituted by 1, 2, 3 or 4 R^{W2}, and 1, 2, 3, 4 or 5 methylene groups in each of the C₃₋₁₀ alkylene, C₂₋₈ heteroalkyl, C₂₋₈ alkenyl and C₂₋₈ alkynyl groups are independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each R¹ and R^{W2} has the meaning described in the present invention.

In some embodiments of the conjugated groups of the present invention, each of A₁, A₂, A₃, A₄, A₅, C₁, C₂, C₃, C₄ and C₅ is independently a bond, C₃₋₈ alkylene, cyclopropyl, cyclobutyl, cyclopentyl, cyclohexyl, a heterocyclyl consisting of 3-6 ring atoms, C₂₋₆ heteroalkyl, C₂₋₆ alkenyl, C₂₋₆ alkynyl, -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of C₃₋₈ alkylene, cyclopropyl, cyclobutyl, cyclopentyl, cyclohexyl, a heterocyclyl consisting of 3-6 ring atoms, C₂₋₆ heteroalkyl, C₂₋₆ alkenyl and C₂₋₆ alkynyl is independently optionally substituted by 1, 2, 3 or 4 R^{W2}, and 1, 2, 3, 4 or 5 methylene groups in each of the C₃₋₈ alkylene, C₂₋₆ heteroalkyl, C₂₋₆ alkenyl and C₂₋₆ alkynyl groups are independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each R¹ and R^{W2} has the meaning described in the present invention.

In some embodiments of the conjugated groups of the present invention, each of A₁, A₂, A₃, A₄, A₅, C₁, C₂, C₃, C₄ and C₅ is independently a bond, C₃₋₆ alkylene, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, C₂₋₄ heteroalkyl, C₂₋₄ alkenyl, C₂₋₄ alkynyl, -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of C₃₋₆ alkylene, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, C₂₋₄ heteroalkyl, C₂₋₄ alkenyl and C₂₋₄ alkynyl is independently optionally substituted by 1, 2, 3 or 4 R^{W2}, and 1, 2, 3, 4 or 5 methylene groups in each of the C₃₋₆ alkylene, C₂₋₄ heteroalkyl, C₂₋₄ alkenyl and C₂₋₄ alkynyl groups are independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each R¹ and R^{W2} has the meaning described in the present invention.

In some embodiments of the conjugated groups of the present invention, each of A₁, A₂, A₃, A₄, A₅, C₁, C₂, C₃, C₄ and C₅ is independently a bond, -(CH₂)₃-, -(CH₂)₄-, -(CH₂)₅-, -(CH₂)₆-, -(CH₂)₇-, -(CH₂)₈-, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, -O(CH₂)₄NHCH₂-, -O(CH₂)₃NHCH₂-, -O(CH₂)₅NHCH₂-, -O(CH₂)₄NH-, -O(CH₂)₃NH-, -O(CH₂)₅NH-, -CH₂O(CH₂)₄NH-, -CH₂O(CH₂)₃NH-, -CH₂O(CH₂)₂NH-, C₂₋₆ alkenyl, C₂₋₆ alkynyl, -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of -(CH₂)₃-, -(CH₂)₄-, -(CH₂)₅-, -(CH₂)₆-, -(CH₂)₇-, -(CH₂)₈-, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, -O(CH₂)₄NHCH₂-, -O(CH₂)₃NHCH₂-, -O(CH₂)₅NHCH₂-, -O(CH₂)₄NH-, -O(CH₂)₃NH-, -O(CH₂)₅NH-, -CH₂O(CH₂)₄NH-, -CH₂O(CH₂)₃NH-, -CH₂O(CH₂)₂NH-, C₂₋₆ alkenyl and C₂₋₆ alkynyl is independently optionally substituted by 1, 2, 3 or 4 R^{W2}, and 1, 2, 3, 4 or 5 methylene groups in each of the -(CH₂)₃-, -(CH₂)₄-, -(CH₂)₅-, -(CH₂)₆-, -(CH₂)₇-, -(CH₂)₈-, -O(CH₂)₄NHCH₂-, -O(CH₂)₃NHCH₂-, -O(CH₂)₅NHCH₂-, -O(CH₂)₄NH-, -O(CH₂)₃NH-, -O(CH₂)₅NH-, -CH₂O(CH₂)₄NH-, -CH₂O(CH₂)₃NH-, -CH₂O(CH₂)₂NH-, C₂₋₆ alkenyl and C₂₋₆ alkynyl groups are independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each R¹ and R^{W2} has the meaning described in the present invention.

In some embodiments of the conjugated groups of the present invention, each of B₁, B₂, B₃, B₄ and B₅ is independently a cyclopropyl, cyclobutyl, cyclopentyl, cyclohexyl, a heterocyclyl consisting of 3-6 ring atoms, a C₁₋₆ alkylene, a C₁₋₆ heteroalkyl, a C₂₋₆ alkenyl or a C₂₋₆ alkynyl, wherein at least one of B₁ and B₂ is a cyclopropyl, cyclobutyl, cyclopentyl, cyclohexyl or a heterocyclyl consisting of 3-6 ring atoms, and at least one of B₃, B₄ and B₅ is a cyclopropyl, cyclobutyl, cyclopentyl, cyclohexyl or a heterocyclyl consisting of 3-6 ring atoms, wherein each of the cyclopropyl, cyclobutyl, cyclopentyl, cyclohexyl, heterocyclyl consisting of 3-6 atoms, C₁₋₆ alkylene, C₁₋₆ heteroalkyl, C₂₋₆ alkenyl and C₂₋₆ alkynyl groups is independently optionally substituted by 1, 2, 3 or 4 R^{W3}, and 1, 2, 3, 4 or 5 methylene groups in each of the C₁₋₆ alkylene, C₁₋₆ heteroalkyl, C₂₋₆ alkenyl and C₂₋₆ alkynyl groups are independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each R¹ and R^{W3}has the meaning described in the present invention.

In some embodiments of the conjugated groups of the present invention, each of B₁, B₂, B₃, B₄ and B₅ is independently a cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, a C₁₋₄ alkylene, a C₁₋₄ heteroalkyl, a C₂₋₄ alkenyl or a C₂₋₄ alkynyl, wherein at least one of B₁ and B₂ is a cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl, and at least one of B₃, B₄ and B₅ is a cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl, wherein each of the C₁₋₄ alkylene, C₁₋₄ heteroalkyl, C₂₋₄ alkenyl and C₂₋₄ alkynyl groups is independently optionally substituted by 1, 2, 3 or 4 R^{W3}, and 1, 2, 3, 4 or 5 methylene groups in each of the C₁₋₄ alkylene, C₁₋₄ heteroalkyl, C₂₋₄ alkenyl and C₂₋₄ alkynyl groups are independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-, wherein each R¹ and R^{W3} has the meaning described in the present invention.

In some embodiments of the conjugated groups of the present invention, each R¹ is independently H, deuterium or C₁₋₆ alkyl.

In some embodiments of the conjugated groups of the present invention, each R¹ is independently H, deuterium or C₁₋₄ alkyl.

In some embodiments of the conjugated groups of the present invention, each R¹ is independently H, deuterium, methyl, ethyl, n-propyl, isopropyl, n-butyl or isobutyl.

In some embodiments of the conjugated groups of the present invention, each of R^{W1}, R^{W2} and R^{W3} is independently deuterium, =O, hydroxyl, nitro, cyano, F, Cl, Br, I, C₁₋₆ alkyl, C₁₋₆ alkoxy, C₃₋₆ cycloalkyl, C₂₋₆ alkenyl or C₁₋₆ heteroalkyl.

In some embodiments of the conjugated groups of the present invention, each of R^{W1}, R^{W2} and R^{W3} is independently deuterium, =O, hydroxyl, nitro, cyano, F, Cl, Br, I, C₁₋₄ alkyl, C₁₋₄ alkoxy, cyclopropyl, cyclobutyl, cyclopentyl, cyclohexyl, C₂₋₄ alkenyl or C₁₋₄ heteroalkyl.

In some embodiments of the conjugated groups of the present invention, each of R^{W1}, R^{W2} and R^{W3} is independently deuterium, =O, hydroxyl, nitro, cyano, F, Cl, Br, I, methyl, ethyl, n-propyl, isopropyl, n-butyl, isobutyl, methoxy, ethoxy, 1-propoxy, 2-propoxy, 1-butoxy, 2-methyl-1-propoxy, 2-butoxy, cyclopropyl, cyclobutyl, cyclopentyl, cyclohexyl, C₂₋₄ alkenyl or C₁₋₄ heteroalkyl.

In some embodiments of the conjugated groups of the present invention, R^{x} is a structure represented by Formula (c-1) or Formula (d-1): 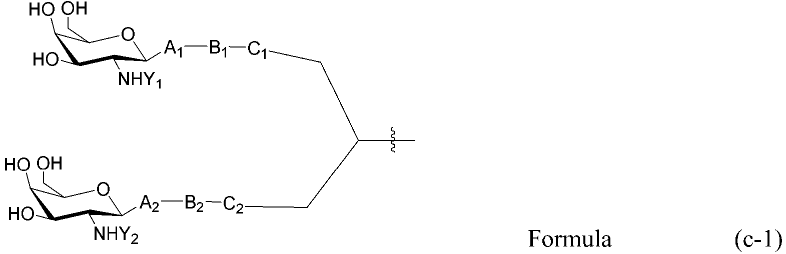 or 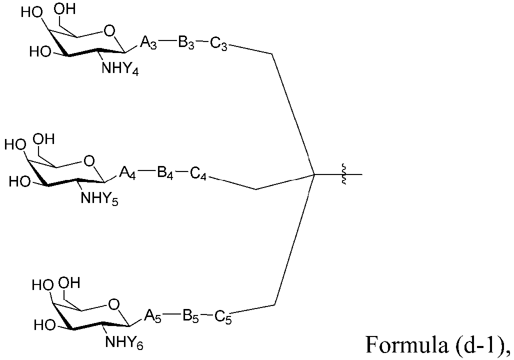 wherein each of A₁, A₂, A₃, A₄, A₅, B₁, B₂, B₃, B₄, B₅, C₁, C₂, C₃, C₄, C₅, Y₁, Y₂, Y₃, Y₄ and Y₅ has the meaning described in the present invention.

In some embodiments of the conjugated groups of the present invention, wherein the conjugated group has a structure represented by Formula (II-a), or a stereoisomer, tautomer, or acceptable salt of the structure represented by Formula (II-a), 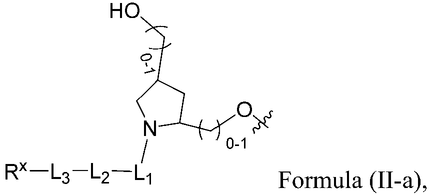 wherein each of R^{x}, L₁, L₂ and L₃ has the meaning described in the present invention.

In some embodiments of the conjugated groups of the present invention, wherein the conjugated group has a structure represented by Formula (II-a-1), or a stereoisomer, tautomer, or acceptable salt of the structure represented by Formula (II-a-1), 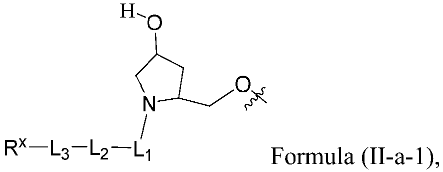 wherein each of L₁, L₂, L₃ and R^{x} has the meaning described in the present invention.

In some embodiments of the conjugated groups of the present invention, wherein the conjugated group has a structure represented by Formula (II-a-2), or a stereoisomer, tautomer, or acceptable salt of the structure represented by Formula (II-a-2), 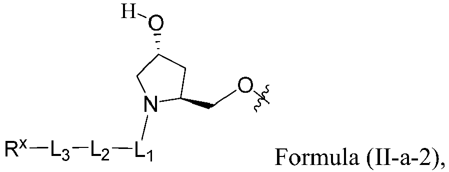 wherein each of L₁, L₂, L₃ and R^{x} has the meaning described in the present invention.

In some embodiments of the conjugated groups of the present invention, wherein the conjugated group has a structure represented by Formula (III-a), or a stereoisomer, tautomer, or acceptable salt of the structure represented by Formula (III-a), 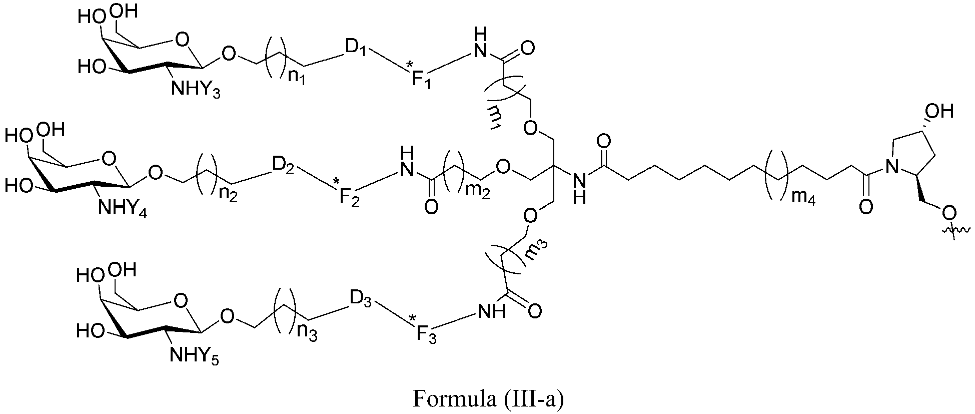 wherein each of D₁, D₂ and D₃ is independently -C(=O)-*, -C(=O)O-*, -OC(=O)-*, -C(=O)N(R¹)-*, -N(R¹)C(=O)-*, -C(=O)N(R¹)CH₂-* or -N(R¹)C(=O)CH₂-*;
each of F₁, F₂ and F₃ is independently cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl;
each of n₁, n₂, n₃, m₁, m₂, m₃ and m₄ is independently 0, 1, 2, 3 or 4;
wherein each of R₁, Y₁, Y₂, Y₃, Y₄ and Y₅ has the meaning described in the present invention.

In some embodiments of the conjugated groups of the present invention, wherein the conjugated group has one of the following structures, or a stereoisomer, tautomer or acceptable salt thereof,

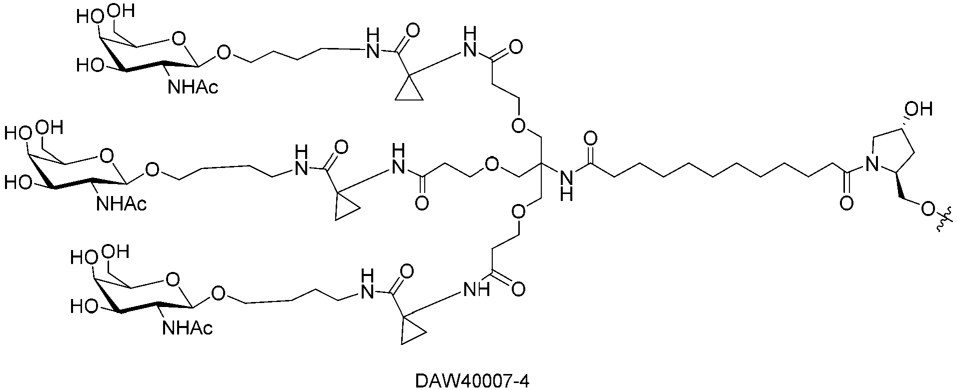

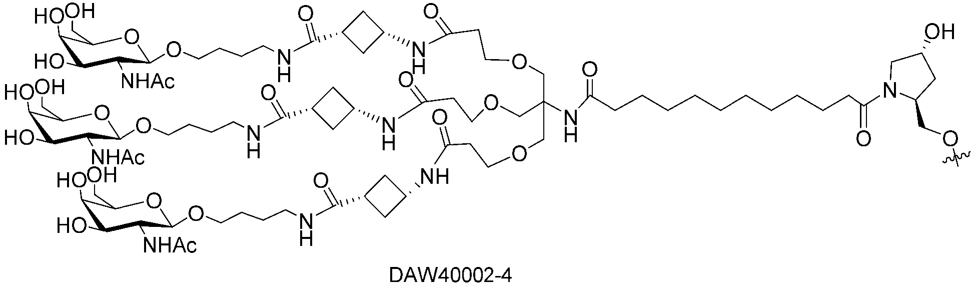

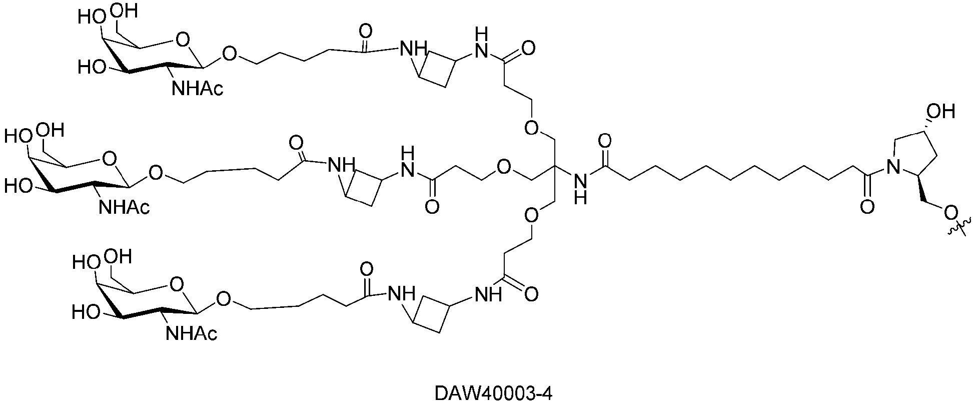

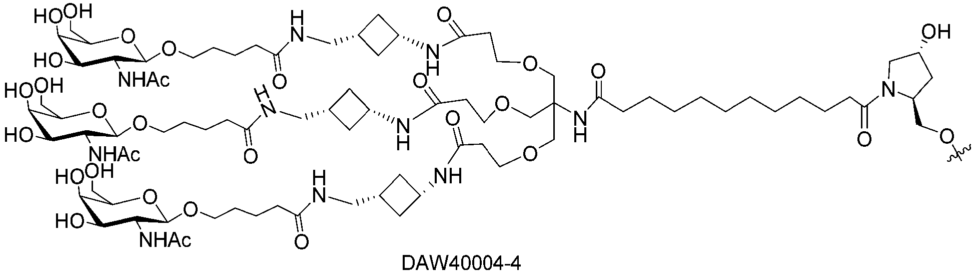

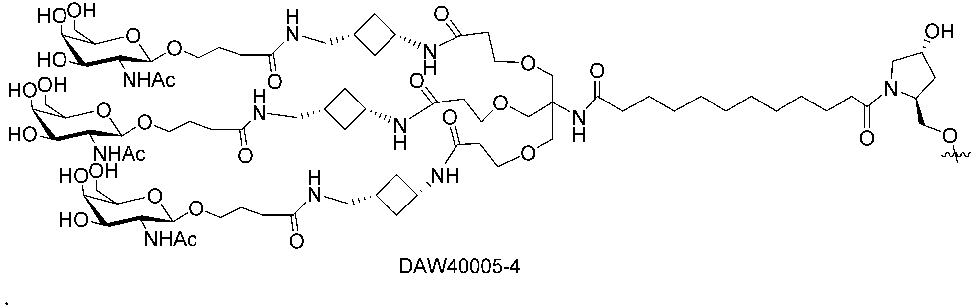

In another aspect, the present invention relates to a nucleic acid conjugate comprising the conjugated group described herein and a nucleic acid, wherein the nucleic acid may be modified or unmodified.

In some embodiments of the nucleic acid conjugate described herein, the nucleic acid is linked to the conjugated group via a phosphate group, a phosphorothioate group, or a phosphate ester group.

In some embodiments of the nucleic acid conjugate described herein, wherein the nucleic acid is a single-stranded nucleic acid or a double-stranded nucleic acid, preferably siRNA; optionally, the conjugated group is preferably conjugated to the 3' end or 5' end of the sense strand of the siRNA, more preferably conjugated to the 3' end of the sense strand of the siRNA.

In some embodiments of the nucleic acid conjugate described herein, wherein the conjugated group is conjugated to the 3' end or 5' end of the nucleic acid.

In another aspect, the present invention relates to a siRNA conjugate, which is one of DAW30223, DAW30062, DAW30219, DAW30221, DAW30222 and DAW30255,
DAW30223:
   Sense Strand (5'-3'): gs-us-g-u-Gf-c-Af-Cf-Uf-u-c-g-c-u-u-c-a-c-a-DAW40002-4 (SEQ ID NO: 2)
   Antisense Strand (5'-3'): us-Gfs-u-g-a-Agn-g-Cf-Gf-a-a-g-u-Gf-c-Af-c-a-cs-us-u (SEQ ID NO: 7)
DAW30062:
   Sense Strand (5'-3'): gs-us-g-u-Gf-c-Af-Cf-Uf-u-c-g-c-u-u-c-a-c-a-DAW40003-4 (SEQ ID NO: 3)
   Antisense Strand (5'-3'): us-Gfs-u-g-a-Agn-g-Cf-Gf-a-a-g-u-Gf-c-Af-c-a-cs-us-u (SEQ ID NO: 7)
DAW30219:
   Sense Strand (5'-3'): gs-us-g-u-Gf-c-Af-Cf-Uf-u-c-g-c-u- u-c-a-c-a-DAW40004-4 (SEQ ID NO: 4)
   Antisense Strand (5'-3'): us-Gfs-u-g-a-Agn-g-Cf-Gf-a-a-g-u-Gf-c-Af-c-a-cs-us-u (SEQ ID NO: 7)
DAW30221:
   Sense Strand (5'-3'): gs-us-g-u-Gf-c-Af-Cf-Uf-u-c-g-c-u-u-c-a-c-a-DAW40005-4 (SEQ ID NO: 5)
   Antisense Strand (5'-3'): us-Gfs-u-g-a-Agn-g-Cf-Gf-a-a-g-u-Gf-c-Af-c-a-cs-us-u (SEQ ID NO: 7)
DAW30222:
   Sense Strand (5'-3'): gs-us-g-u-Gf-c-Af-Cf-Uf-u-c-g-c-u-u-c-a-c-a-DAW40007-4 (SEQ ID NO: 6)
   Antisense Strand (5'-3'): us-Gfs-u-g-a-Agn-g-Cf-Gf-a-a-g-u-Gf-c-Af-c-a-cs-us-u (SEQ ID NO: 7)
DAW30255:
   Sense Strand (5'-3'): gs-us-c-a-u-c-Cf-a-Cf-Af-Af-u-g-a-g-a-g-u-a-c-a-DAW40007-4 (SEQ ID NO: 8)
   Antisense Strand (5'-3'): us-Gfs-u-a-c-(Tgn)-c-u-c-a-u-u-g-Uf-g-Gf-a-u-g-a-cs-gs-a (SEQ ID NO: 9).

In some embodiments, wherein the conjugated groups DAW40002-4, DAW40003-4, DAW40004-4, DAW40005-4 and DAW40007-4 are each conjugated to the 3' end of the siRNA sense strand through a phosphate ester group, and their structures are shown below:

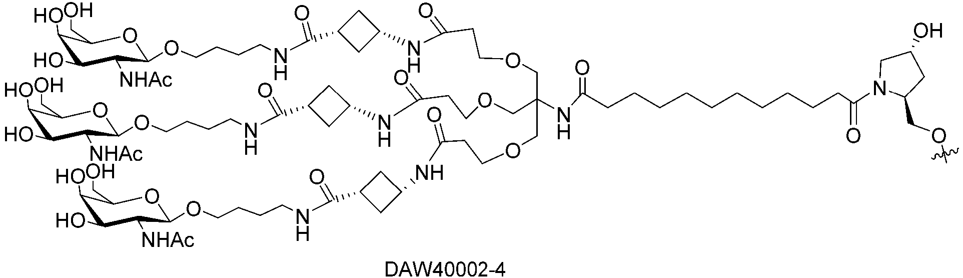

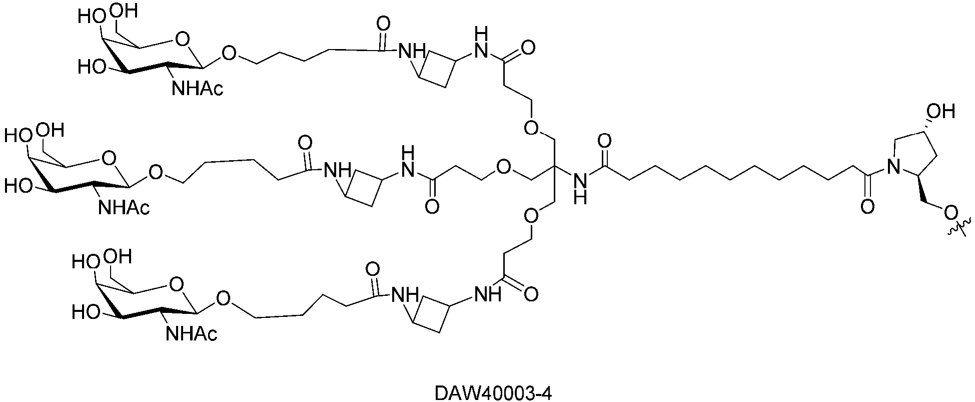

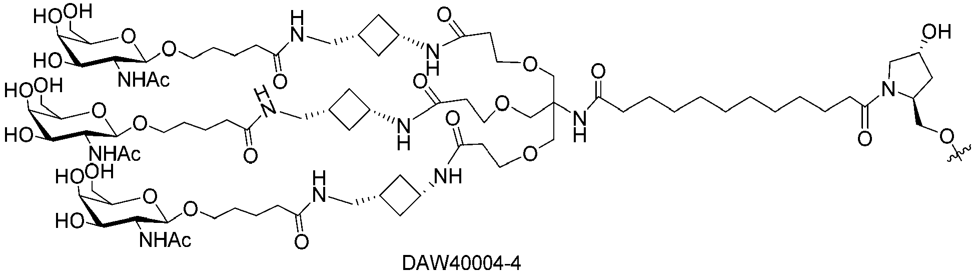

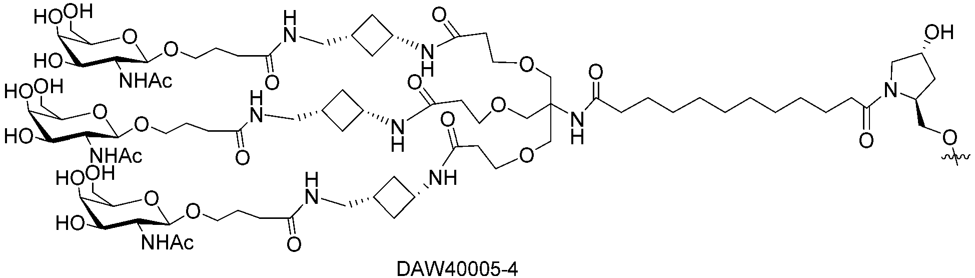

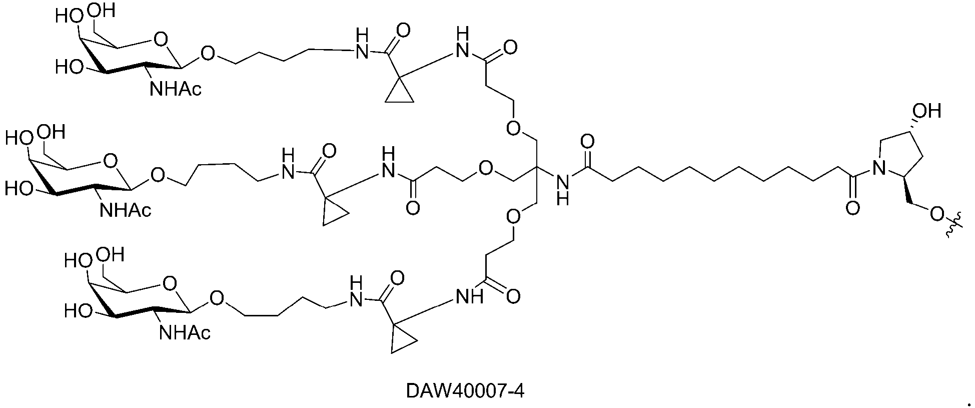

In another aspect, the present invention relates to a pharmaceutical composition comprising the siRNA conjugate herein and a pharmaceutically acceptable carrier.

In another aspect, the present invention relates to a use of the compound, the conjugated group, or the nucleic acid conjugate herein in the preparation of a medicament for treating and/or preventing a pathological condition or disease caused by the expression of a specific gene in hepatocytes.

In some embodiments, the present invention also relates to the use of the compound or the conjugated group of the present invention in drug delivery for treating and/or preventing pathological conditions or diseases caused by the expression of specific genes in hepatocytes.

In some embodiments of the uses described herein, the compound or conjugated group is used for in vivo liver-targeted delivery of a small nucleic acid drug.

In some embodiments of the uses described herein, the compound or conjugated group is used for in vivo liver-targeted delivery of an siRNA drug or an ASO drug.

In some embodiments, the present invention further relates to the use of the nucleic acid conjugates described herein in drug delivery for treating and/or preventing pathological conditions or diseases caused by the expression of specific genes in hepatocytes.

In some embodiments, the present invention also relates to the use of the compound or the conjugated group of the present invention in drug delivery for treating and/or preventing pathological conditions or diseases caused by the expression of specific genes in hepatocytes.

In some embodiments of the uses described herein, the compound or conjugated group is used for *in vivo* liver-targeted delivery of a small nucleic acid drug.

In some embodiments of the uses described herein, the compound or conjugated group is used for *in vivo* liver-targeted delivery of an siRNA drug or an ASO drug.

In some embodiments, the present invention further relates to the use of the nucleic acid conjugates described herein in drug delivery for treating and/or preventing pathological conditions or diseases caused by the expression of specific genes in hepatocytes. In another aspect, the present invention relates to the use of the conjugate groups of the compounds described herein in drug delivery for treating and/or preventing pathological conditions or diseases caused by the expression of specific genes in hepatocytes.

In some embodiments of the present invention, wherein the specific gene is selected from ApoB, ApoC3, AGT, ANGPTL3, PCSK9, LPA, SCD1, FVII, p53, HBV or HCV.

In another aspect, the present invention relates to the use of the nucleic acid conjugate of the present invention for treating and/or preventing pathological conditions or diseases caused by the expression of specific genes in hepatocytes.

In some embodiments of the present invention, wherein the specific gene is selected from ApoB, ApoC3, AGT, ANGPTL3, PCSK9, LPA, SCD1, FVII, p53, HBV or HCV.

In another aspect, the present invention relates to the siRNA drugs DAW30223, DAW30062, DAW30219, DAW30221, and/or DAW30222, or pharmaceutical compositions thereof, for use in treating and/or preventing hepatitis B.

In another aspect, the present invention relates to DAW30255 or a pharmaceutical composition thereof for use in treating and/or preventing hypertension.

In another aspect, the present invention relates to a method for treating and/or preventing pathological conditions or diseases caused by the expression of specific genes in hepatocytes, which comprises using the compound or the conjugated group of the present invention as a drug delivery system.

In some embodiments of the present invention, wherein the specific gene is selected from ApoB, ApoC3, AGT, ANGPTL3, PCSK9, LPA, SCD1, FVII, p53, HBV or HCV.

In yet another aspect, the present invention relates to a method for treating and/or preventing a pathological condition or disease caused by the expression of a specific gene in hepatocytes, the method comprising administering the nucleic acid conjugate of the present invention.

In some embodiments of the present invention, wherein the specific gene is selected from ApoB, ApoC3, AGT, ANGPTL3, PCSK9, LPA, SCD1, FVII, p53, HBV or HCV.

In another aspect, the present invention relates to a method for preventing, managing, treating or alleviating hepatitis B or hypertension, comprising administering to a patient a therapeutically effective amount of DAW30223, DAW30062, DAW30219, DAW30221, and/or DAW30222, or a pharmaceutical composition thereof.

In another aspect, the present invention relates to a method for preventing, managing, treating or alleviating hepatitis B or hypertension, comprising administering to a patient a therapeutically effective amount of DAW30255, or a pharmaceutical composition thereof.

In some embodiments of the use described herein, the specific gene includes, but is not limited to, ApoB, ApoC3, AGT, ANGPTL3, PCSK9, LPA, SCD1, FVII, p53, HBV, or HCV.

In some embodiments of the use described herein, the specific gene is selected from ApoB, ApoC3, AGT, ANGPTL3, PCSK9, LPA, SCD1, FVII, p53, HBV, or HCV.

In another aspect, the present invention also relates to the use of DAW30223, DAW30062, DAW30219, DAW30221, and/or DAW30222 described herein, and compositions comprising DAW30223, DAW30062, DAW30219, DAW30221, and/or DAW30222 described herein and a pharmaceutically acceptable carrier in the preparation of a medicament for treating and/or preventing hepatitis B.

In another aspect, the present invention also relates to the use of DAW30255 described herein, or a composition thereof, in the preparation of a medicament for treating and/or preventing hypertension.

In another aspect, the present invention also provides a method for inhibiting the expression of a specific gene in liver cells in a patient, comprising administering the nucleic acid conjugate or composition thereof of the present invention to the patient, wherein the nucleic acid conjugate or composition thereof may be in a therapeutically effective amount.

In some embodiments of the present invention, the nucleic acid (or small nucleic acid) described herein is an siRNA or an ASO.

In another aspect, the present invention also provides a method for inhibiting the expression of a specific gene in liver cells in a patient, comprising administering the siRNA conjugate or composition thereof of the present invention to the patient, wherein the nucleic acid ligand conjugate or composition thereof may be in a therapeutically effective amount.

### Nucleic Acid Conjugates, Nucleic Acid Conjugate Formulations, Administration, and Disease Treatment Methods of the Present Invention

The effective amount of the nucleic acid conjugates (e.g., siRNA conjugates or pharmaceutical compositions) described herein may vary depending on the mode of administration and the severity of the disease being treated. The preferred effective amount can be determined by one of ordinary skill in the art based on various factors (e.g., through clinical trials). Such factors include, but are not limited to, the pharmacokinetic parameters of the active ingredient, such as bioavailability, metabolism, and half-life; the severity of the disease being treated, the patient's weight, the patient's immune status, and the route of administration. For example, as the exigencies of the therapeutic condition dictate, several divided doses may be administered daily, such as four times a day, three times a day, twice a day, once a day, or every other day, or the dosage may be proportionally reduced over several daily doses.

Administration to a subject can be via any suitable route known in the art, including, but not limited to, oral or parenteral routes, including intravenous, intramuscular, subcutaneous, transdermal, airway (aerosol), pulmonary, nasal, rectal, and topical (including buccal and sublingual) administration, with intravenous administration being preferred.

The pharmaceutical compositions disclosed herein include formulations suitable for parenteral administration. The formulations may conveniently be presented in unit dosage form and may be prepared by any methods well known in the art of pharmacy. The amount of active ingredient that can be combined with excipients to produce a single-dose form is generally that amount of siRNA that produces a therapeutic effect. Generally, on a per-cent basis, this amount ranges from about 1% to about 99% of the active ingredient, preferably from about 5% to about 70%, and most preferably from about 10% to about 30%.

In another aspect, the present invention provides a method for inhibiting the expression or activity of a hepatitis B virus gene. According to an embodiment of the present invention, the method comprises: contacting a cell with the aforementioned siRNA, siRNA conjugate, or pharmaceutical composition. As previously described, both the aforementioned siRNA and siRNA conjugate can degrade hepatitis B virus mRNA, inhibiting HBV expression and replication. Thus, the method of the present invention can be used to inhibit the expression or activity of a hepatitis B virus gene. For example, the expression or activity of hepatitis B virus genes can be inhibited in animals; or the expression or activity of hepatitis B virus genes can be inhibited for non-disease treatment purposes, such as in vitro inhibition of hepatitis B virus gene expression or activity for subsequent research.

In another aspect, the present invention provides a method for preventing and/or treating hepatitis B virus-induced diseases. According to an embodiment of the present invention, the method comprises: administering a pharmaceutically acceptable amount of the aforementioned siRNA, siRNA conjugate, or pharmaceutical composition to a subject. According to an embodiment of the present invention, the method can effectively prevent and/or treat diseases associated with hepatitis B virus.

### General Synthesis Methods of the Compounds and Nucleic Acid Conjugates of the Present Invention

Generally, the compounds and nucleic acid conjugates of the present invention can be prepared by the methods described herein. Unless otherwise specified, the substituents are defined as shown in Formulas (I), (II), (II-1), (II-2), (III), (I-a), (II-a), (II-a-1), (II-a-2), and (III-a). The following non-limiting schemes and examples are presented to further exemplify the invention.

In the examples described below, unless otherwise indicated all temperatures are set forth in degrees Celsius (°C). Column chromatography was conducted using a silica gel column. Silica gel (200 - 300 mesh) was purchased from Qingdao Ocean Chemical Factory, NH₂CPG was purchased from Hebei Dinaxingke. NMR spectra were obtained using CDC1₃, DMSO-*d₆*, CD₃OD, or acetone-*d₆* as solvents (in ppm), with TMS (0 ppm) or chloroform (7.25 ppm) as the reference standard. When multiple peaks are present, the following abbreviations are used: s (singlet), d (doublet), t (triplet), m (multiplet), br (broadened), dd (doublet of doublets), dt (doublet of triplets), br.s (broadened singlet), and q (quartet). The coupling constant, *J*, is expressed in Hertz (Hz).

Low-resolution mass spectral (MS) data were also determined on an Agilent 6320 series LC-MS spectrometer equipped with G1312A binary pumps, a G1316A TCC (Temperature Control of Column, maintained at 30 °C), a G1329A autosampler and a G1315B DAD detector were used in the analysis. An ESI source was used on the LC-MS spectrometer.

High-resolution mass spectral (MS) data were also determined on an Agilent 6130 series LC-MS spectrometer equipped with G1311A quaternary pumps, a G1316A TCC (Temperature Control of Column, maintained at 30 °C), a G1329A autosampler and a G1315D DAD detector were used in the analysis. An ESI source was used on the HR-MS spectrometer.
The following abbreviations are used throughout the specification:

| | | | |
|---|---|---|---|
| EDCI | 1-ethyl-3-(3-dimethylpropylamine)carbodiimide | EDCI | 1-(3-dimethylaminopropyl)-3-ethylcarbodiimide |
| HOBT | 1-hydroxybenzotriazole | EA | ethyl acetate |
| TFA | trifluoroacetic acid | HOBT | 1-hydroxybenzotriazole |
| Py | pyridine | DMAP | 4-dimethylaminopyridine |
| PE | petroleum ether | MsCl | methanesulfonyl chloride |
| ACN | acetonitrile | TMSOTf | trimethylsilyl |
| Ac₂O | acetic anhydride | THF | tetrahydrofuran |
| Boc | *tert*-butoxycarbonyl | mL | milliliter, milliliters |
| MeOH | methanol | min | minute, minutes |
| DMSO | dimethyl sulfoxide | M, N, mol/L | moles per liter |
| DMF | N,N-dimethylformamide | h | hour, hours |
| DCM | dichloromethane | RT, rt | room temperature |
| DIPEA | *N,N-* diisopropylethylamine | | |
| HBTU | benzotriazole-N,N,N',N'-tetramethyluronium hexafluorophosphate | | |

### EXAMPLES

The present invention will be explained below with reference to the following examples. Those skilled in the art will understand that the following examples are merely illustrative of the present invention and should not be construed as limiting its scope. In particular, the synthesis of small nucleic acids and nucleic acid conjugates can be achieved using the examples of the present invention or by adjusting the synthesis methods commonly employed in the art. If specific techniques or conditions are not specified in the examples, they should be performed according to techniques or conditions described in literature in the art or according to product specifications. Reagents and instruments used without manufacturer identification are commercially available.

### Synthesis of the Compounds Described in the Present Invention

The following synthesis schemes outline the general experimental procedures for preparing the compounds disclosed in this invention. Those skilled in the art can prepare the compounds described in this invention by making appropriate modifications to the methods or adjusting the starting materials, as appropriate for their specific circumstances. wherein each of M, X₇, X₈, X₉, D₁, F₁, n₁, Y₃, L₁, L₂, L₃, m₁, m₂, m₃ and R has the meaning as defined in the present invention. Unless otherwise specified, the compounds described herein can be prepared by the methods described in the following synthetic schemes.

### Synthetic Scheme 1:

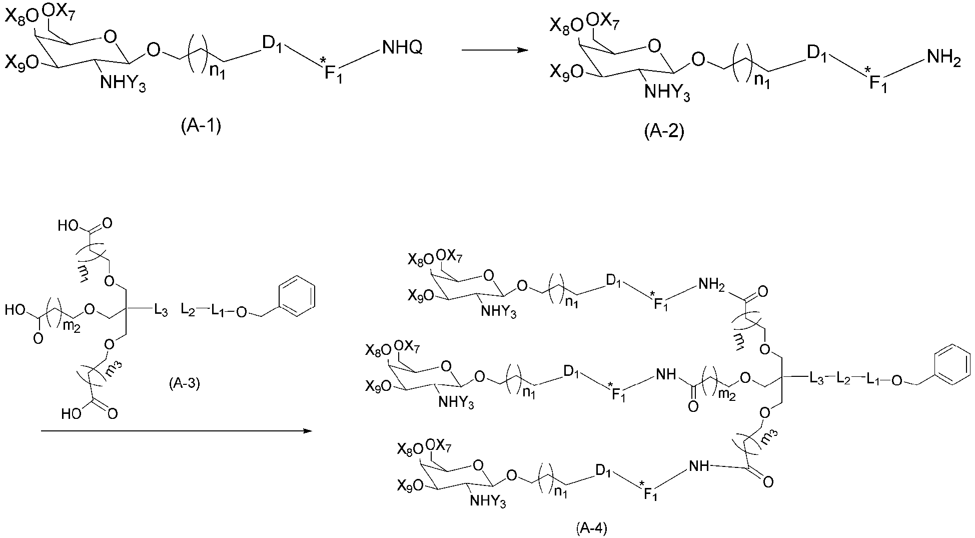

The compound represented by Formula (A-4) can be prepared by the method described in Synthetic Scheme 1, wherein Q is an amino protecting group, such as Cbz, Boc, etc. Compound (A-1) is subjected to removal of the amino protecting group Q to obtain compound (A-2); compound (A-2) undergoes a condensation reaction with compound (A-3) to obtain compound (A-4).

### Synthetic Scheme 2:

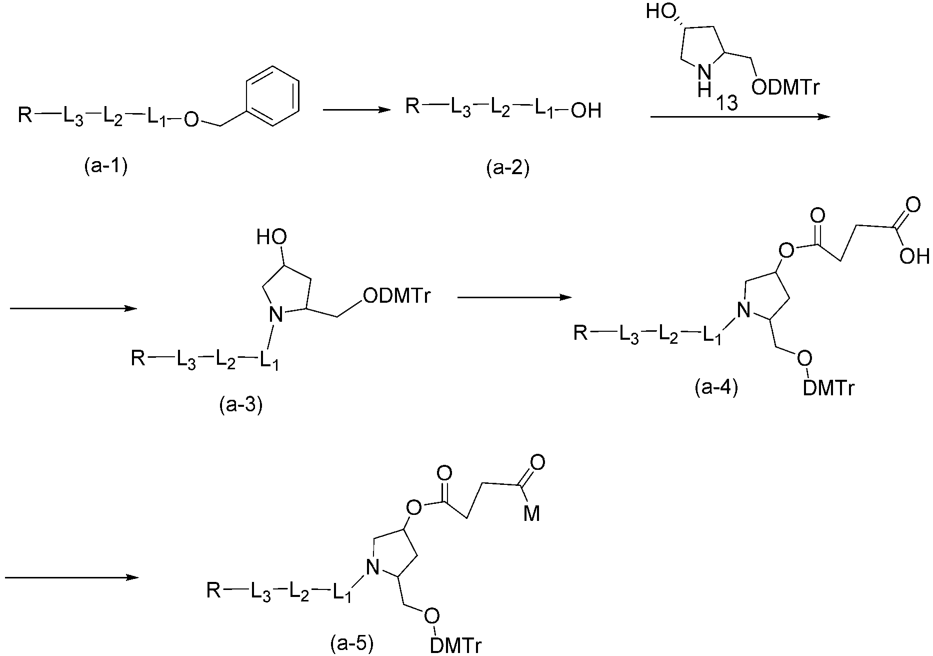

The compound represented by Formula (a-5) can be prepared by the method described in Synthesis Scheme 2, wherein L1 contains a carbonyl group at the end linked to the benzyloxy group. Compound (a-1) is subjected to removal of the benzyl group to yield compound (a-2); compound (a-2) undergoes a condensation reaction with compound 13 to yield compound (a-3); compound (a-3) reacts with succinic anhydride to yield compound (a-4); and compound (a-4) reacts with a solid support to yield compound (a-5).

### Preparation Examples

In the following preparation examples, the inventors take some of the compounds of the present invention as examples to describe in detail the preparation process of the compounds of the present invention, wherein 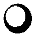 is CPG .

### Example 1: Compound DAW40003-3

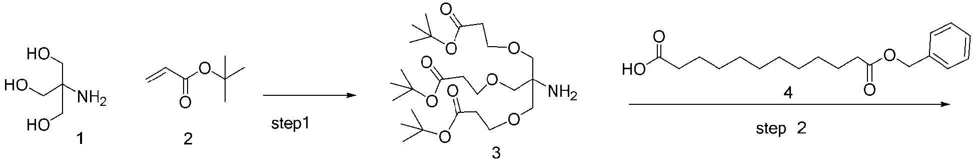

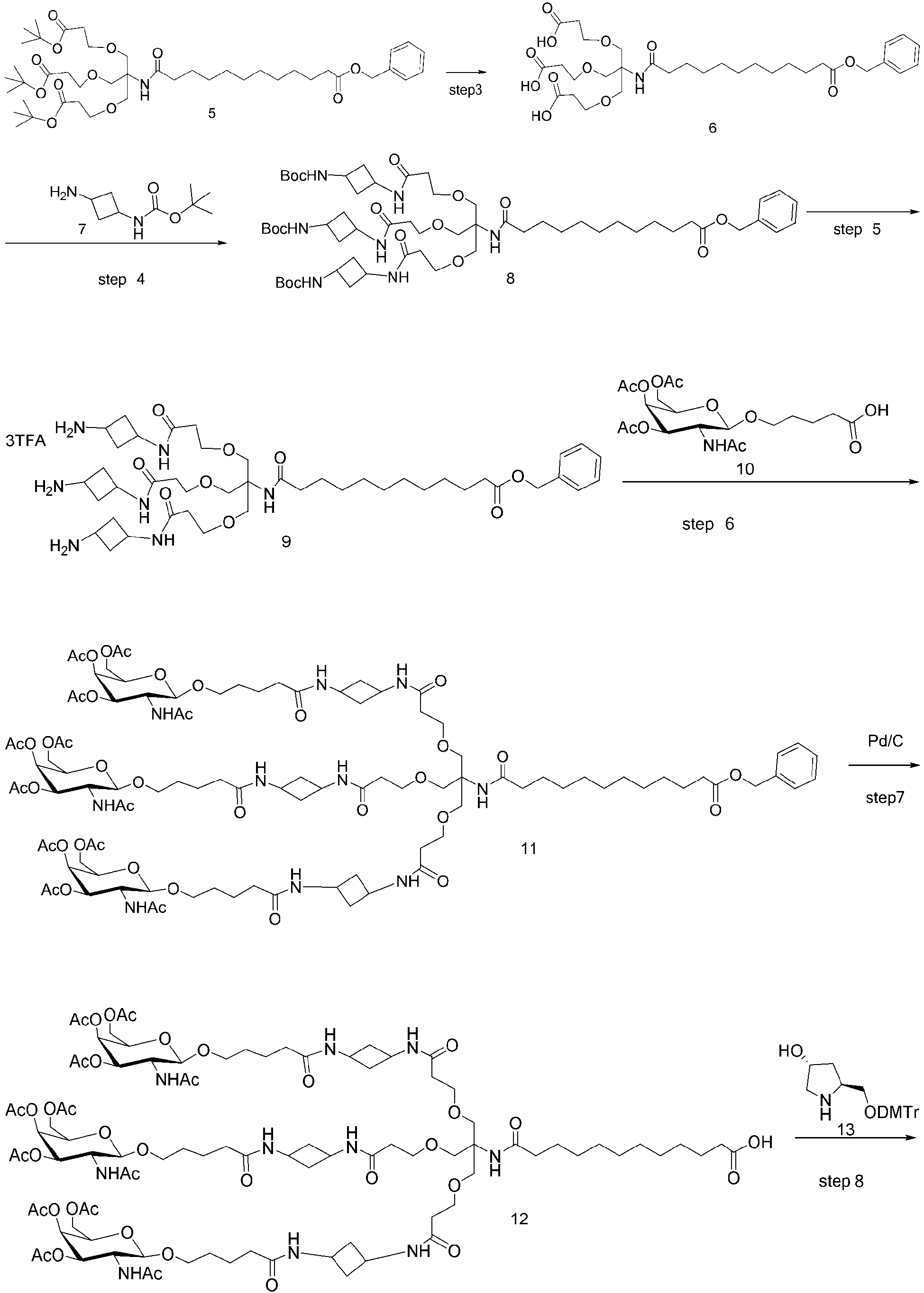

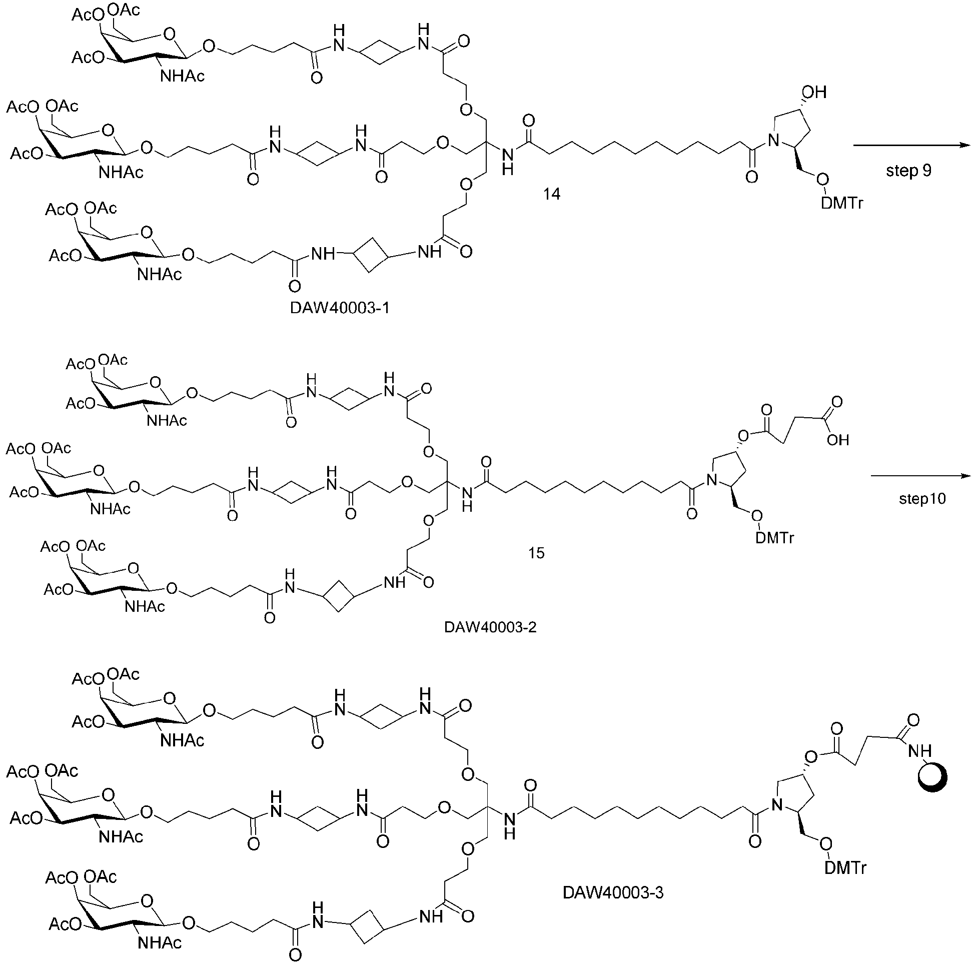

### Step 1: Synthesis of Compound 3

Compound 1 (2.42 g, 19.98 mmol) and sodium hydroxide (0.40 mL, 2.00 mmol) were dissolved in DMSO (4 mL), then compound 2 (8.96 g, 69.93 mmol) was slowly added, and the resulted mixture was stirred at room temperature for 36 h. The solvent was then evaporated under reduced pressure, and the resulting residue was purified by silica gel column chromatography (PE/EA (v/v) = 1/2 + 0.05% concentrated aqueous ammonia) to afford compound 3 (4.61 g, 45.64%) as a pale yellow oil. MS (ESI, pos. ion) m/z: 506.4 [M+H]⁺.

### Step 2: Synthesis of Compound 5

Compound 4 (prepared by referring to the synthesis of compound 76 in WO2020093053A1) (0.15 g, 0.47 mmol), HBTU (0.23 g, 0.60 mmol), and DIPEA (0.16 g, 1.20 mmol) were dissolved in DMF (1 mL), and then stirred at room temperature for 5 min, Compound 3 (0.20 g, 0.40 mmol) was then added and stirred for 16 h. After completion of the reaction, the mixture was diluted with water (10 mL), extracted with DCM (2 × 50 mL), dried over anhydrous sodium sulfate, and the solvent evaporated under reduced pressure to afford a pale yellow viscous compound 5 (0.32 g, 100.12%), which was directly used in the next step. MS (ESI, pos. ion) m/z: 404.5 [M/2+H]⁺.

### Step 3: Synthesis of Compound 6

Compound 5 (0.30 g, 0.37 mmol) was dissolved in TFA (3 mL), the solution was stirred at room temperature for 3 h. The solvent was evaporated under reduced pressure, and the concentrated residue was purified by C18 reverse-phase preparative chromatography (acetonitrile/water (v/v) = 1/1 to 5/1 + 0.1% TFA), then lyophilized under reduced pressure to afford compound 6 (0.20 g, 84.21%) as a white solid. MS (ESI, pos. ion) m/z: 640.3 [M+H]⁺.

### Step 4: Synthesis of Compound 8

Compound 6 (0.15 g, 0.23 mmol), compound 7 (0.15 g, 0.81 mmol), EDCI (0.19 g, 0.97 mmol), and HOBT (0.13 g, 0.97 mmol) were dissolved in DCM (3 mL), the solution was stirred at room temperature for 15 h. The reaction was quenched with water (5 mL), then diluted with DCM (40 mL), washed sequentially with hydrochloric acid solution (20 mL, 1 M), saturated sodium bicarbonate solution (10 mL), and saturated sodium chloride solution (10 mL), the organic phase dried over anhydrous sodium sulfate, and the solvent evaporated under reduced pressure. The resulting residue was purified by silica gel column chromatography (DCM/MeOH (v/v) = 10/1 to 5/1) to afford a pale yellow viscous compound 8 (0.26 g, 96.81%). MS (ESI, pos. ion) m/z: 1144.7 [M+H]⁺.

### Step 5: Synthesis of Compound 9

Compound 8 (0.26 g, 0.23 mmol) was dissolved in TFA (5 mL) and stirred at room temperature for 5 h. The solvent evaporated under reduced pressure to afford a pale yellow viscous compound 9 (0.27 g), which was directly used in the next step. MS (ESI, pos. ion) m/z: 844.8 [M+H]⁺.

### Step 6: Synthesis of Compound 11

Compound 10 (0.10 g, 0.23 mmol) (purchased from Lingkai Pharmaceuticals), HBTU (0.33 g, 0.87 mmol), HOBT (0.12 g, 0.87 mmol), and DIPEA (0.30 g, 2.30 mmol) were dissolved in DMF (2 mL) and stirred at room temperature for 5 min. A solution of compound 9 (0.27 g) obtained in Step 5 in DMF (1 mL) was then slowly added, and the mixture was stirred at room temperature for 19 h. After the reaction was complete, the reaction mixture was diluted with DCM (100 mL), then washed sequentially with saturated sodium bicarbonate solution (100 mL) and saturated brine (100 mL). The organic phase was dried over anhydrous sodium sulfate, and the solvent was evaporated under reduced pressure. The resulting residue was purified by silica gel column chromatography (DCM/MeOH (V/V) = 20/1 to 5/1) to afford compound 11 (0.39 g, 80.42%) as a slightly yellow solid. MS (ESI, pos. ion) m/z: 2131.0 [M- H]⁺.

### Step 7: Synthesis of Compound 12

Compound 11 (0.11 g, 0.052 mmol) and palladium/carbon (0.0055 g, 0.0052 mmol, 10%) were added to methanol (5 mL). The reaction system was purged with hydrogen three times and then stirred at room temperature under a hydrogen atmosphere for 8 h. After completion of the reaction, the mixture was filtered through celite and the filtrate was evaporated to dryness under reduced pressure to afford compound 12 (0.10 g, 94.92%) as a yellow-brown oil, which was used directly in the next reaction. MS (ESI, pos. ion) m/z: 2041.0 [M-H]⁻.

### Step 8: Synthesis of Compound DAW40003-1

Compound 12 (0.28 g, 0.14 mmol), HBTU (0.058 g, 0.15 mmol), and DIPEA (0.062 g, 0.48 mmol) were dissolved in DCM (2 mL) and stirred at room temperature for 5 min, a solution of compound 13 (0.059 g, 0.14 mmol) in DCM (1 mL) was then slowly added dropwise. The mixture was stirred at room temperature for 12 h. The solvent was evaporated under reduced pressure, and the resulted residue was dissolved in DMF (5 mL) and separated by reverse preparative column (acetonitrile/water solution (V/V) = 43% to 49% + 0.1% ammonium acetate). Salt was added to the preparative solution containing the product to saturate it. The organic phase was separated, and the aqueous phase was extracted with acetonitrile (2 × 100 mL). The organic phases were combined, dried over anhydrous sodium sulfate, and the solvent was evaporated under reduced pressure to obtain compound DAW40003-1 (0.18 g, 53.72%) as a pale yellow solid. MS (ESI, pos. ion) m/z: 2442.0 [M-H]⁻.

### Step 9: Synthesis of Compound DAW40003-2

Under nitrogen, compound DAW40003-1 (0.45 g, 0.19 mmol), succinic anhydride (0.022 g, 0.22 mmol), DMAP (0.032 g, 0.26 mmol), and DIPEA (0.048 g, 0.37 mmol) were dissolved in DCM (2 mL) and stirred at room temperature for 41.5 h. After completion of the reaction, the mixture was diluted with DCM (100 mL) and washed sequentially with saturated sodium bicarbonate solution (20 mL) and saturated brine (20 mL). The organic phase was dried over anhydrous sodium sulfate, and the solvent was evaporated under reduced pressure to yield a pale yellow viscous compound DAW40003-2 (0.19 g, 40.56%), which was used directly in the next reaction. MS (ESI, pos. ion) m/z: 2542.2 [M- H]⁺.

### Step 10: Synthesis of Compound DAW40003-3

Compound DAW40003-2 (0.18 g, 0.071 mmol), HBTU (0.040 g, 0.11 mmol), and DIPEA (0.024 mL, 0.14 mmol) were dissolved in ACN (5 mL) and stirred at room temperature for 5 min. Then, the mixture was transferred to a solid-phase synthesizer containing 0.7 g of H₂N-CPG (purchased from Hebei Dinaxingke) and shaken for 22.5 h. The mixture was then filtered, the filter cake was filtered and rinsed with DCM/MeOH (V/V = 9/1, 10 mL) and DCM (10 mL), respectively. The filter cake was dried, then diluted in 25% Ac₂O/Py solution (5 mL) and sitrred for 3 h, then filtered and rinsed with DCM/MeOH (V/V = 9/1, 10 mL) and DCM (10 mL) respectively. The filter cake was dried under reduced pressure to obtain compound DAW40003-3 (0.67 g) as a white solid with a loading of 29.39 µmol/g.

### Example 2: Compound DAW40004-3

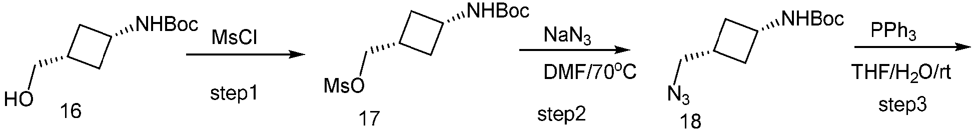

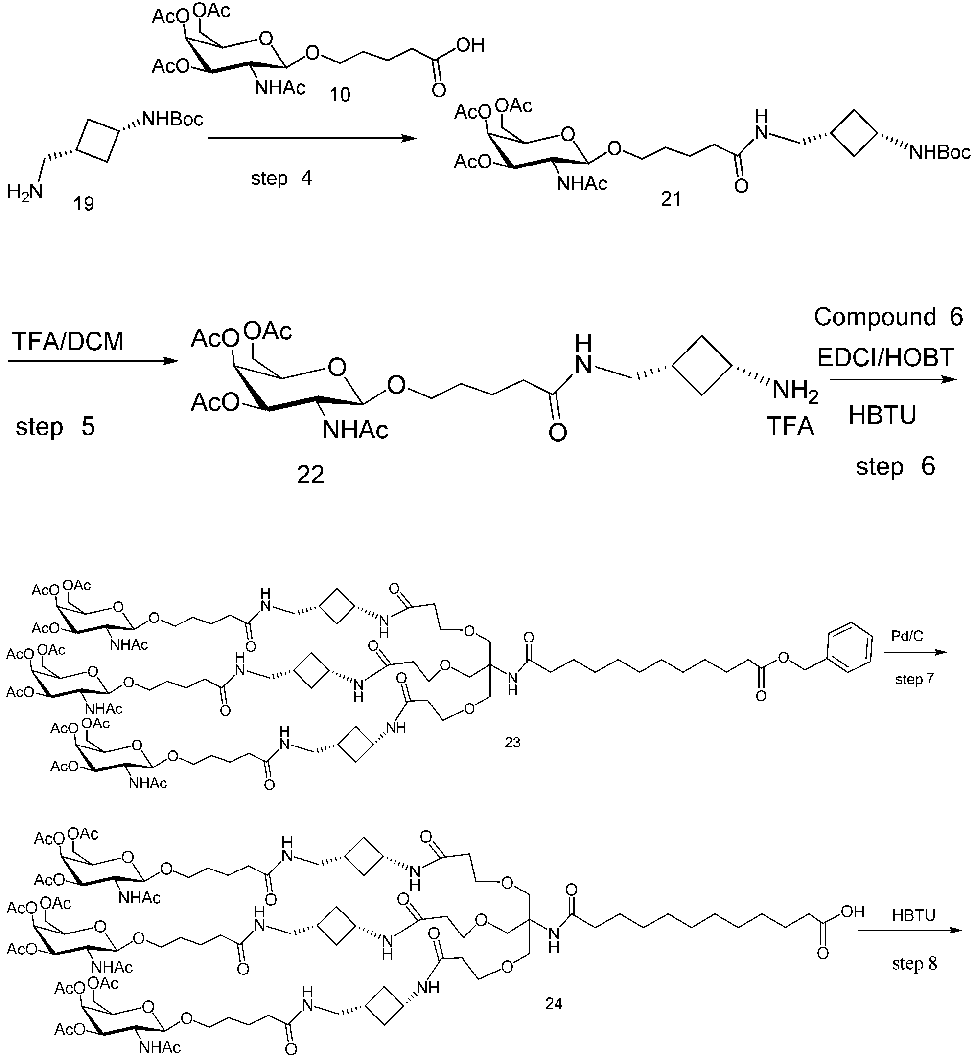

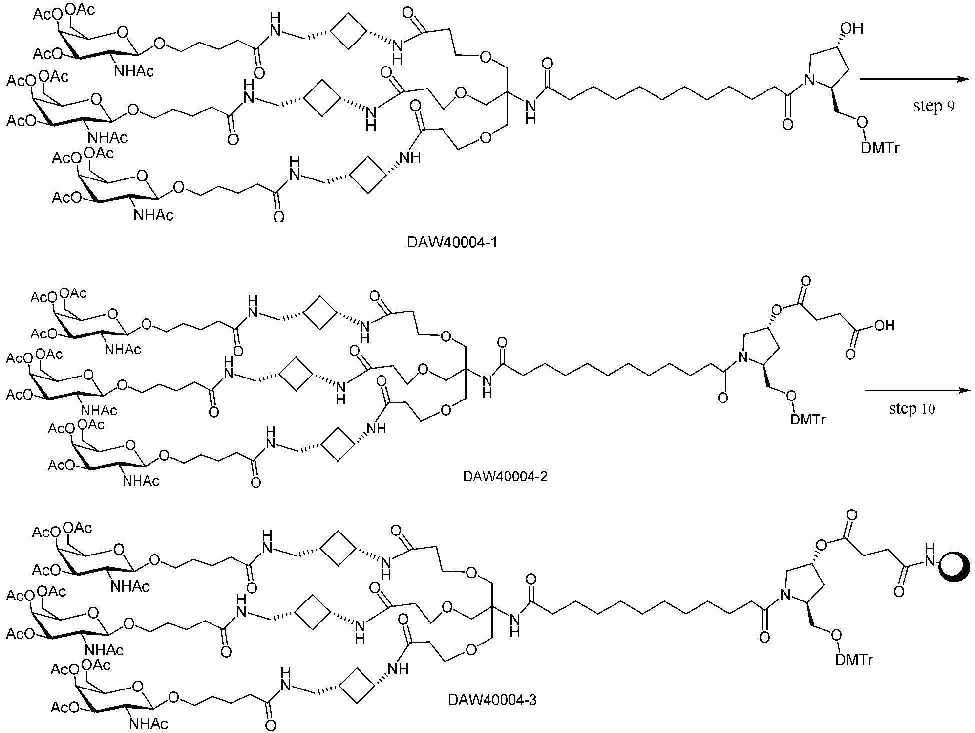

### Step 1: Synthesis of Compound 17

Compound 16 (2.00 g, 9.94 mmol) and triethylamine (3.02 g, 29.82 mmol) were dissolved in DCM (20 mL). Methanesulfonyl chloride (1.37 g, 11.93 mmol) was added dropwise to the solution in an ice bath. The reaction mixture was stirred at room temperature for 31 h. After completion of the reaction, DCM (100 mL) and dilute hydrochloric acid (0.2 M, 20 mL) were added sequentially. The organic phase was separated, washed with saturated brine (2 × 50 mL), dried over anhydrous sodium sulfate, and the solvent was evaporated under reduced pressure to yield compound 17 (2.64 g, 95.1%) as a pale yellow solid. MS (ESI, pos. ion) m/z: 302.4 [M+Na]⁺.

### Step 2: Synthesis of Compound 18

Compound 17 (2.50 g, 8.95 mmol) and sodium azide (1.16 g, 17.9 mmol) were added to DMF (40 mL) and stirred at 70 °C for 21 h. The mixture was cooled to room temperature, and EA (300 mL) and water (200 mL) were added. The organic phase was separated, washed with saturated brine (2 × 200 mL), and dried over anhydrous sodium sulfate. The solvent was evaporated under reduced pressure to give an oil, which was cooled to give compound 18 (2.0 g, 98.76%) as a white solid. MS (ESI, pos. ion) m/z: 227.1 [M+H]⁺.

### Step 3: Synthesis of Compound 19

Compound 18 (0.53 g, 2.34 mmol) and triphenylphosphine (0.92 g, 3.51 mmol) were dissolved in tetrahydrofuran (11.5 mL) and water (0.23 mL) and stirred at room temperature for 10 h. After completion of the reaction, the reaction mixture was dried over anhydrous sodium sulfate, and the solvent was evaporated under reduced pressure to yield compound 19 (0.44 g, 93.8%) as a slightly yellow viscous substance. MS (ESI, pos. ion) m/z: 201.3 [M+H]⁺.

### Step 4: Synthesis of Compound 21

Compound 10 (1.11 g, 2.48 mmol), HOBT (0.46 g, 3.38 mmol), and HBTU (1.28 g, 3.38 mmol) were dissolved in DMF (5 mL) and stirred at 25°C for 5 min. A solution of compound 19 (0.45 g, 2.25 mmol) in DMF (1 mL) was then slowly added dropwise, followed by the addition of DIPEA (2.61 mL, 15.75 mmol). The reaction mixture was allowed to react at room temperature for 18 h. After the reaction was completed, DCM (200 mL) was added for dilution and the mixture was washed sequentially with saturated sodium bicarbonate solution (20 mL), brine (5%, 100 mL × 2), and saturated brine (50 mL). The organic phase was dried over anhydrous sodium sulfate, and the solvent was evaporated under reduced pressure. The resulting residue was purified by silica gel column chromatography (MeOH/DCM (V/V) = 1/5) to obtain compound 21 (0.85 g, 60.08%) as a white solid. MS (ESI, pos. ion) m/z: 630.7 [M+H]⁺.

### Step 5: Synthesis of Compound 22

Compound 21 (3.4 g, 5.4 mmol) was dissolved in DCM (54 mL), and TFA (4.01 mL, 54 mmol) was added. The reaction mixture was stirred at room temperature for 5 h. The solvent was evaporated under reduced pressure to afford compound 22 (3.47 g, 102.3%) as a slightly yellow oil. MS (ESI, pos. ion) m/z: 530.1 [M+H]⁺.

### Step 6: Synthesis of Compound 23

Compound 23 was prepared with reference to the synthetic method of Step 6 of Example 1, and it is a white solid (1.6 g, 47%). MS (ESI, pos. ion) m/z: 2173.0588 [M-H]⁻.

### Step 7: Synthesis of Compound 24

Compound 24 was prepared with reference to the synthetic method of Step 7 of Example 1, and it is a white solid (1.53 g, 84.21%). MS (ESI, pos. ion) m/z: 1042.3[M/2+H]⁺.

### Step 8: Synthesis of Compound DAW40004-1

Compound DAW40004-1 was prepared with reference to the synthetic method of Step 8 of Example 1, and it is a light yellow solid (0.40 g, 33.5%). MS (ESI, pos. ion) m/z: 2484.2 [M+H]⁺.

### Step 9: Synthesis of Compound DAW40004-2

Compound DAW40004-2 was prepared with reference to the synthetic method of Step 9 of Example 1, and it is a white solid (0.4 g, 96.13%). MS (ESI, pos. ion) m/z: 1291.6 [M/2+H]⁺.

### Step 10: Synthesis of Compound DAW40004-3

Compound DAW40004-3 was prepared with reference to the synthetic method of Step 10 of Example 1, and it is a white solid (1.59 g), with a measured loading of 25.94 µmol/g.

### Example 3: Compound DAW40005-3

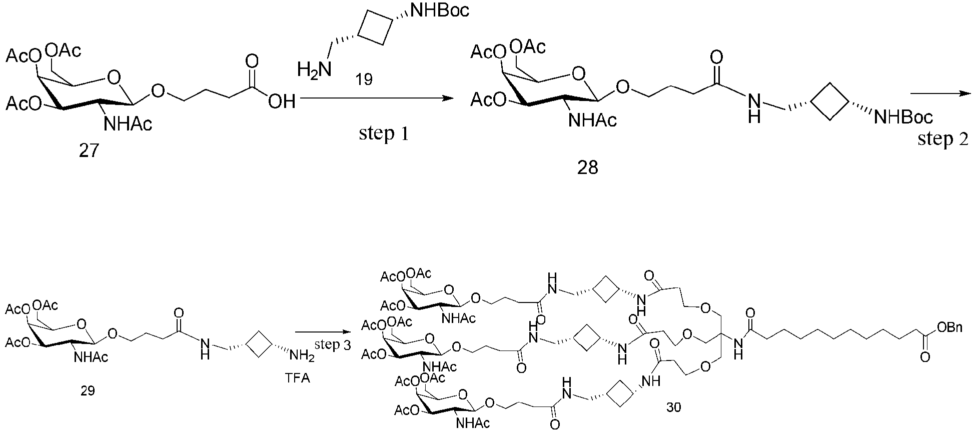

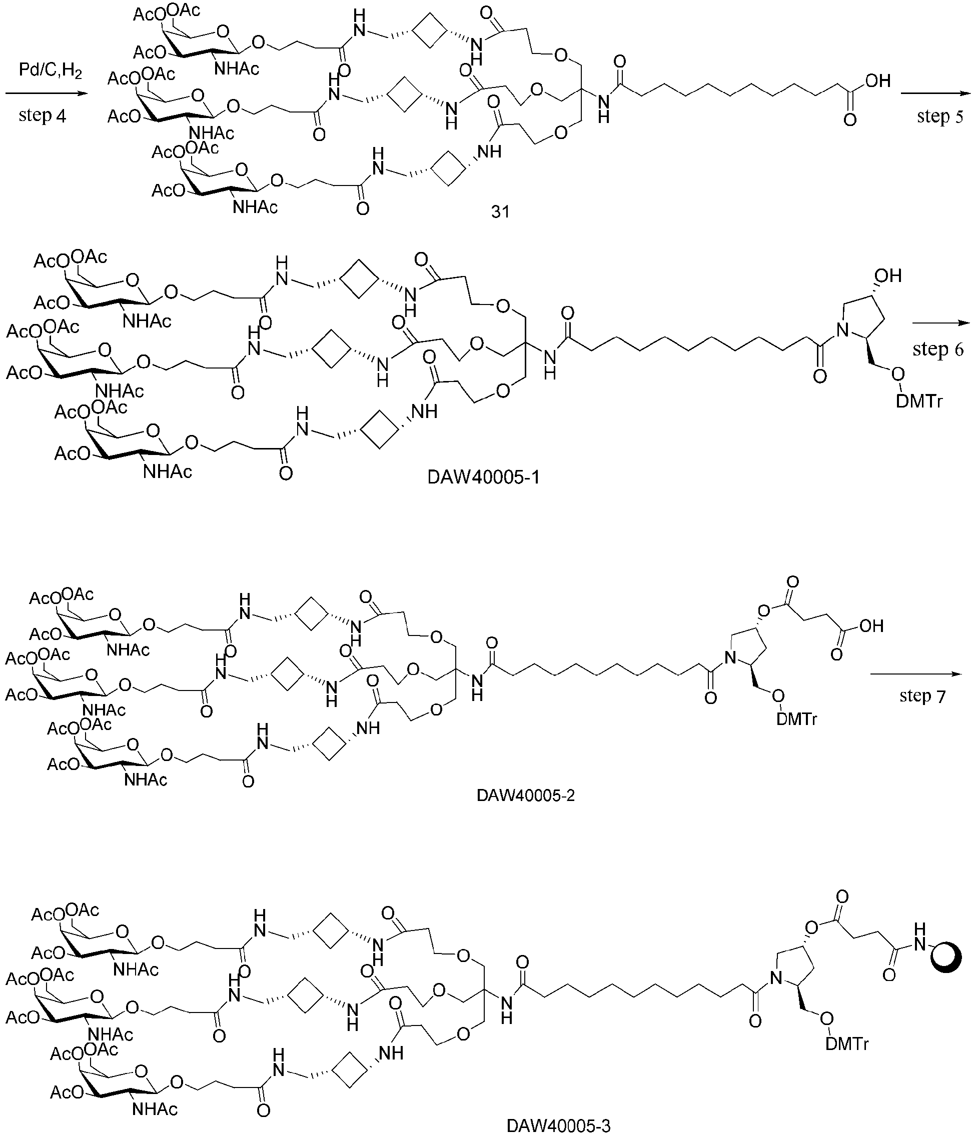

### Step 1: Synthesis of Compound 28

Compound 27 (1.52 g, 3.5 mmol) (prepared according to the synthesis method in Journal of Organic Chemistry (2020), 85(10), 6593-6604), HOBT (0.71 g, 5.25 mmol) and HBTU (1.99 g, 5.25 mmol) were dissolved in DMF (10 mL) and stirred at 25 °C for 5 min. Then, a solution of compound 19 (0.7 g, 3.5 mmol) in DMF (2 mL) was slowly added dropwise, followed by the addition of DIPEA (2.9 mL, 17.5 mmol). The reaction mixture was reacted at room temperature for 16 h. The reaction mixture diluted withwater (30 mL) , then extracted with EA (50 mL × 4). The organic phases were combined, washed with saturated brine (50 mL), and dried over anhydrous sodium sulfate. The solvent was evaporated under reduced pressure, and the resulting residue was purified by silica gel column chromatography (DCM to MeOH/DCM (V/V) = 1/10) to give Compound 28 (1.24 g, 57.63%) as a white solid. MS (ESI, pos. ion) m/z: 616.6 [M+H]⁺.

### Step 2: Synthesis of Compound 29

Compound 28 (0.60 g, 0.97 mmol) was dissolved in DCM (8 mL), and TFA (0.72 mL, 9.7 mmol) was added. The reaction mixture was stirred at room temperature for 5 h, and the solvent was evaporated under reduced pressure to give compound 29 (0.60 g, 97.95%) as a brown oil. MS (ESI, pos. ion) m/z: 516.6 [M+H]⁺.

### Step 3: Synthesis of Compound 30

Compound 6 (0.19 g, 0.30 mmol), compound 29 (0.66 g, 1.05 mmol), HOBT (0.17 g, 1.26 mmol), HBTU (0.48 g, 1.26 mmol), and DIPEA (0.50 mL, 3 mmol) were dissolved in DCM (20 mL) and stirred at room temperature for 16 h. The reaction mixtrue was quenched by adding water (20 mL), extracted with DCM (100 mL × 2) and the combined organic phases were washed sequentially with saturated sodium bicarbonate (40 mL) and saturated sodium chloride solution (40 mL), and dried over anhydrous sodium sulfate, and the solvent was evaporated under reduced pressure. The residue was purified by silica gel column chromatography (DCM/MeOH (V/V) = 10/1) to give compound 30 (0.275 g, 43.42%) as a white solid. MS (ESI, pos. ion) m/z: 1066.7 [M/2+H]⁺.

### Step 4: Synthesis of Compound 31

Compound 31 was prepared with reference to the synthetic method of Step 7 of Example 1, and it is a white solid (0.26 g, 98.72%). MS (ESI, pos. ion) m/z: 1022.5 [M/2+H]⁺.

### Step 5: Synthesis of Compound DAW40005-1

Compound DAW40005-1 was prepared with reference to the synthetic method of Step 8 of Example 1, and it is a light yellow solid (0.08 g, 25.8%). MS (ESI, neg. ion) m/z: 2442.1 [M-H]⁻.

### Step 6: Synthesis of Compound DAW40005-2

Compound DAW40005-2 was prepared with reference to the synthetic method of Step 9 of Example 1, and it is a white solid (0.08 g, 96.07%). MS (ESI, pos. ion) m/z: 2542.2 [M-H]⁻.

### Step 7: Synthesis of Compound DAW40005-3

Compound DAW40005-3 was prepared with reference to the synthetic method of Step 10 of Example 1, and it is a white solid (0.22 g), with a measured loading of 22.32 µmol/g.

### Example 4: Compound DAW40002-3

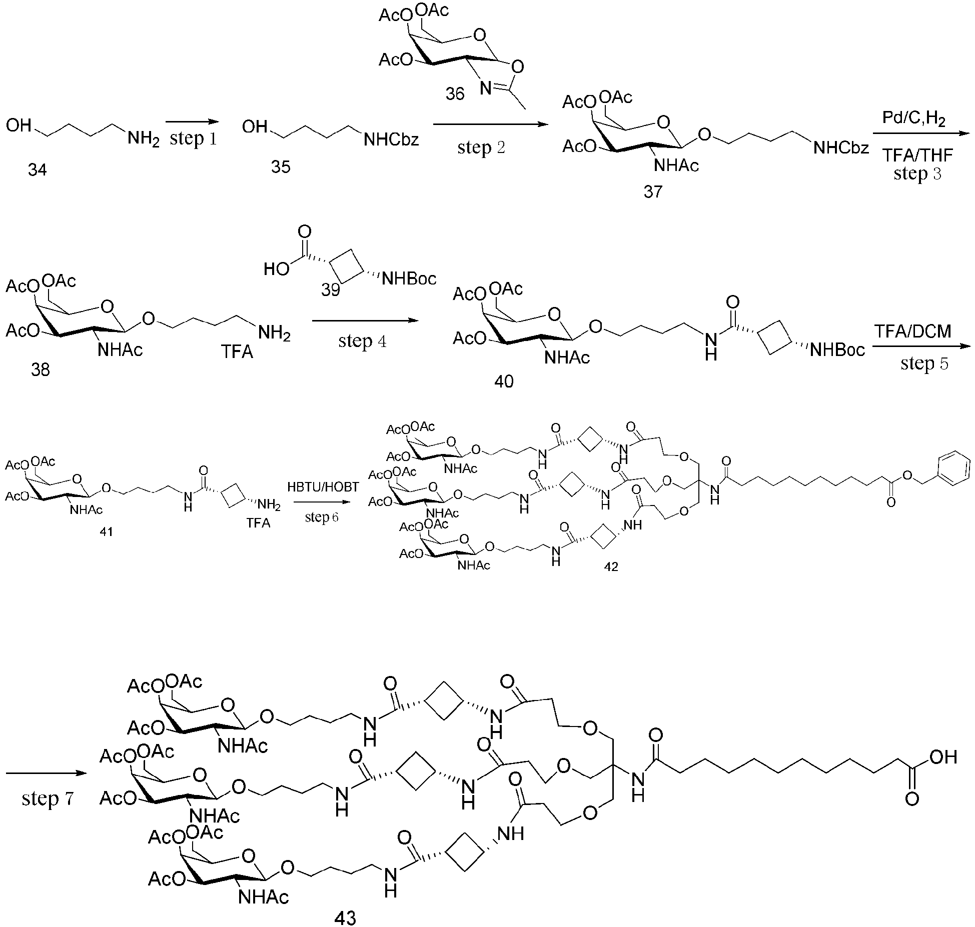

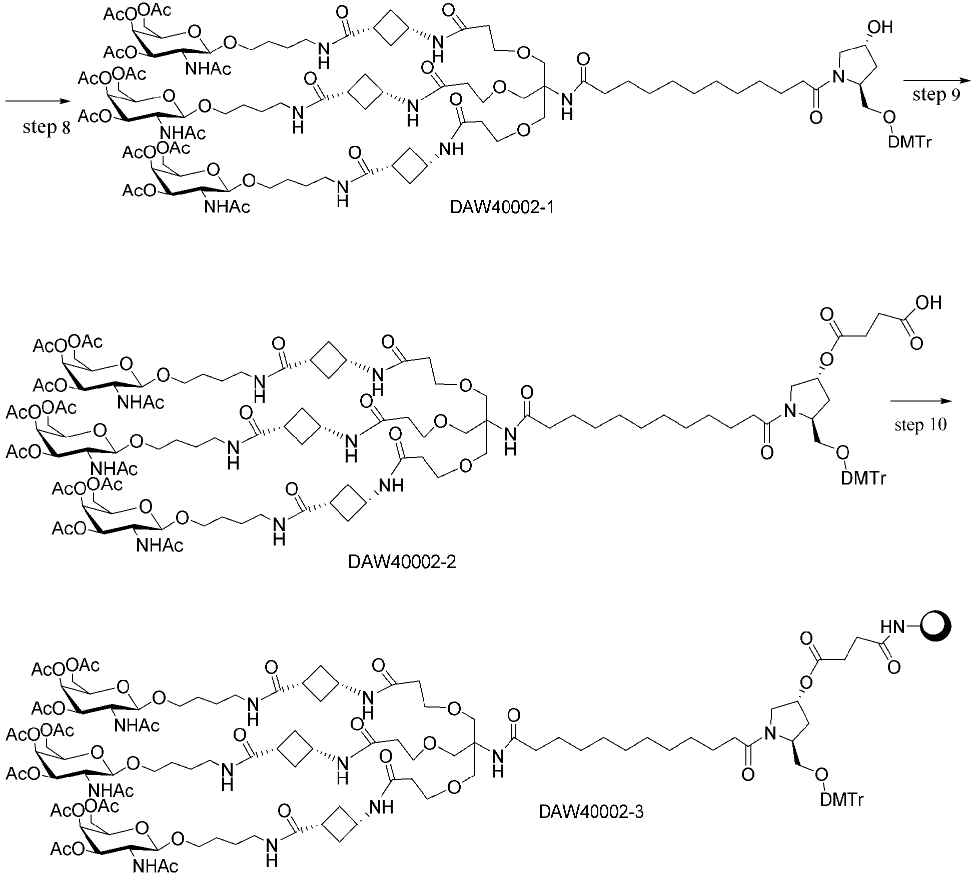

### Step 1: Synthesis of Compound 35

Compound 34 (2.50 g, 28.05 mmol) and triethylamine (7.8 mL, 56.1 mmol) were dissolved in DCM (120 mL). Benzyl chloroformate (9.57 g, 56.1 mmol) was added dropwise at 0 °C. After the addition was completed, the reaction mixture was warmed to room temperature and stirred for 20 h. Saturated ammonium chloride solution (50 mL) was then added for dilution. The layers were separated, the aqueous phase was discarded, and the organic phase was concentrated. The resulting residue was purified by silica gel column chromatography (MeOH/DCM (V/V) = 1/30) to afford compound 35 (2.96 g, 47.2%) as a white solid.
MS (ESI, pos. ion) m/z:224.2 [M+H]⁺;
¹H NMR (400 MHz, CDCl₃) δ 7.37 (d, *J =* 4.2 Hz, 5H), 5.11 (s, 2H), 5.00 (s, 1H), 4.19 - 4.14 (m, 1H), 3.70 - 3.64 (m, 2H), 3.24 (t, *J* = 6.4 Hz, 2H), 3.15 (q, *J =* 4.6 Hz, 1H), 1.90 (dq, *J =* 14.2, 5.0, 4.3 Hz, 2H), 1.77 - 1.71 (m, 1H).

### Step 2: Synthesis of Compound 37

Compound 36 (1.5 g, 4.56 mmol) and compound 35 (1.22 g, 5.47 mmol) were dissolved in 1,2-dichloroethane (30 mL). 3A molecular sieves (2.0 g) were added and stirred at room temperature for 10 min. TMSOTf (0.51 g, 2.28 mmol) was then added, and the reaction mixture was stirred at room temperature for 18 h. The reaction mixture was poured into saturated sodium bicarbonate solution (100 mL), extracted with DCM (100 mL), the organic phase washed with saturated brine (100 mL), dried over anhydrous sodium sulfate, and the solvent evaporated under reduced pressure. The resulting residue was purified by silica gel column chromatography (PE/EA (V/V) = 2/1 to EA) to afford compound 37 (1.8 g, 71.51%) as a light brown oil. MS (ESI, pos. ion) m/z: 553.3 [M+H]⁺.

### Step 3: Synthesis of Compound 38

Compound 37 (0.57 g, 1.03 mmol) and palladium on carbon (0.11 g, 0.1 mmol, 10%) were added to THF (10 mL), followed by the addition of TFA (0.12 g, 1.03 mmol). The mixture was then replaced with hydrogen three times and stirred at room temperature under a hydrogen atmosphere for 19 h. After the reaction was completed, the mixture was filtered through celite and the solvent was evaporated under reduced pressure to give compound 38 (0.55 g, 100.32%) as a light brown oil. MS (ESI, pos. ion) m/z: 419.3 [M +H]⁺.

### Step 4: Synthesis of Compound 40

Compound 39 (0.55 g, 2.55 mmol) and compound 38 (1.45 g, 2.8 mmol) were dissolved in DCM (30 mL). HBTU (1.50 g, 3.95 mmol) and DIPEA (1.4 mL, 8.47 mmol) were added sequentially. The reaction mixture was reacted at room temperature for 16 h. After completion of the reaction, water (20 mL) and DCM (100 mL) were added sequentially for dilution. The organic phase was washed sequencely with saturated sodium bicarbonate solution (20 mL) and saturated brine (20 mL). The solvent was evaporated under reduced pressure, and the resulting residue was purified by silica gel column chromatography (MeOH/EA (V/V) = 1/20) to obtain compound 40 (0.60 g, 38%) as a white solid. MS (ESI, pos. ion) m/z: 616.6 [M+H]⁺.

### Step 5: Synthesis of Compound 41

Compound 40 (0.72 g, 1.17 mmol) was dissolved in DCM (8 mL), and TFA (0.87 mL, 11.7 mmol) was added. The mixture was stirred at 25 °C for 16 h, and the solvent was concentrated to afford compound 41 (0.74 g, 100%) as a brown oil. MS (ESI, pos. ion) m/z:516.3 [M+H]⁺.

### Step 6: Synthesis of Compound 42

Compound 6 (0.30 g, 0.42 mmol, i.e., compound 6 in Example 1, prepared by the synthesis method of compound 6 in Example 1) and compound 41 (0.71 g, 1.39 mmol) were dissolved in DCM (30 mL). HOBT (0.24 mL, 1.76 mmol), HBTU (0.67 g, 1.76 mmol), and DIPEA (0.7 mL, 4.2 mmol) were added, and the reaction mixture was stirred at 30 °C for 3 h. Water (20 mL) was added to quench the reaction, and the mixture was extracted with DCM (100 mL × 2). The organic phases were combined and washed sequentially with saturated sodium bicarbonate (40 mL) and saturated sodium chloride solution (40 mL), dried over anhydrous sodium sulfate, and the solvent was evaporated under reduced pressure. The resulting residue was purified by silica gel column chromatography (DCM to DCM/MeOH (V/V = 10/1)) to give Compound 42 (0.15 g, 17%) as a white solid. MS (ESI, pos. ion) m/z: 1066.2 [M/2+H]⁺.

### Step 7 to Step 9: Synthesis of Compound 43, Compound DAW40002-1 and Compound DAW40002-2

Compound 43, Compound DAW40002-1 and Compound DAW40002-2 were prepared respectively with reference to the synthetic methods of Step 7 to Step 9 of Example 1.

Compound DAW40002-1 was a white solid ((0.083g, 36.5%), HRMS (ESI, neg. ion) m/z: 2442.1905 [M-H]⁻;

Compound DAW40002-2 was a white solid (0.08 g, 96.07%), HRMS (ESI, neg. ion) m/z: 2542.1929 [M-H]⁻.

### Step 10: Synthesis of Compound DAW40002-3

Compound DAW40002-3 was prepared with reference to the synthetic method of Step 10 of Example 1, it was a white solid (0.35 g), with a measured loading of 21.806 uM/g.

### Example 5: Compound DAW40007-3

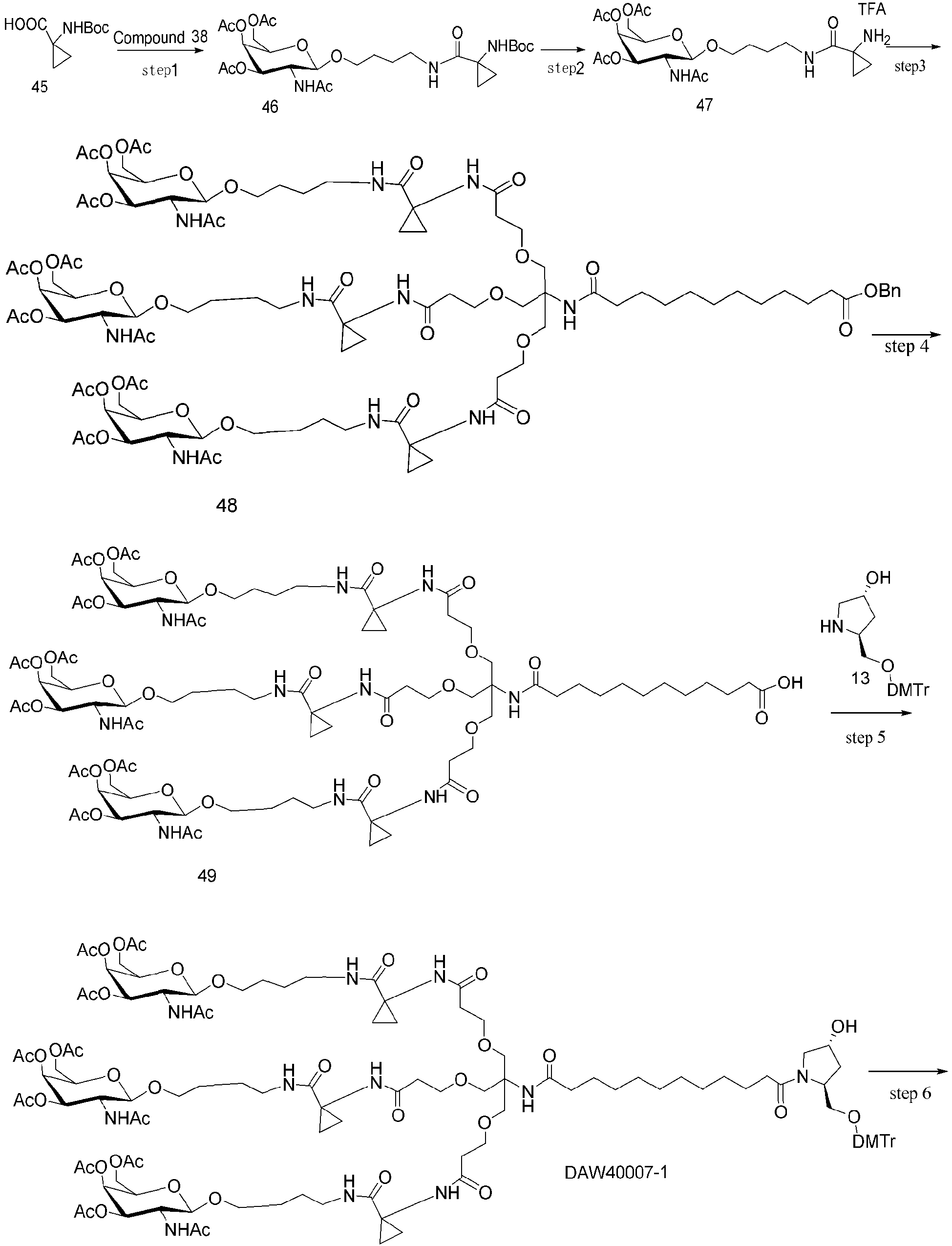

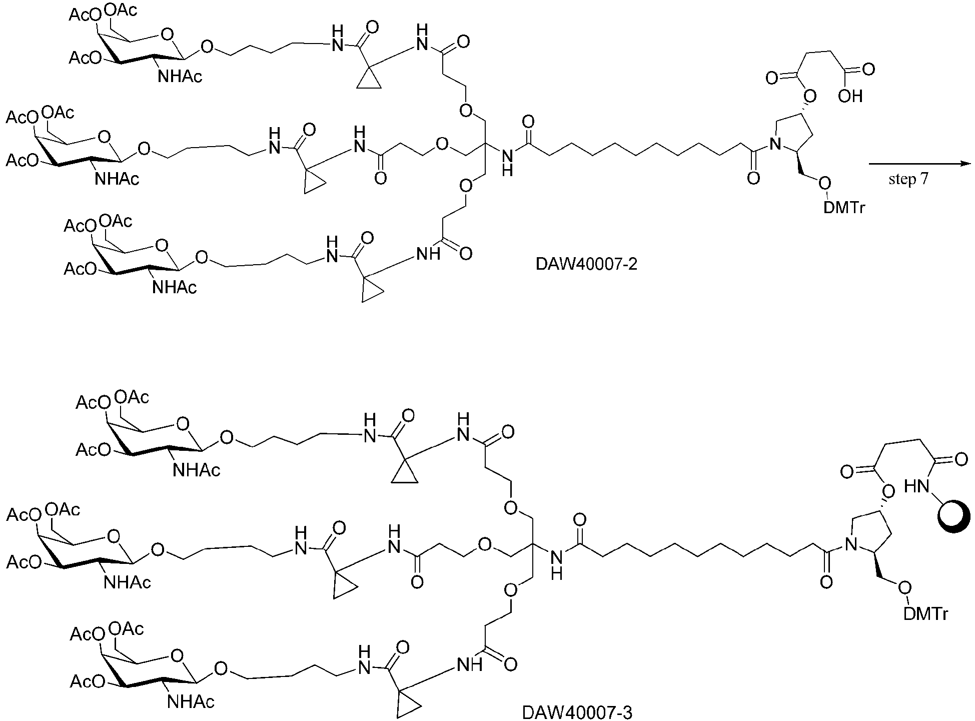

### Step 1: Synthesis of Compound 46

Compound 45 (0.46 g, 2.29 mmol, purchased from Shanghai Bid Pharmaceutical Technology Co., Ltd.) and compound 38 (1.16 g, 2.18 mmol, i.e., compound 38 in Example 4, prepared by the synthesis method of compound 38 in Example 1) were dissolved in DCM (30 mL). HOBT (0.46 g, 3.44 mmol), HBTU (1.30 g, 3.44 mmol), and DIPEA (2.66 mL, 16.03 mmol) were added sequentially, and the reaction mixture was reacted at room temperature for 16 h. After the reaction, water (20 mL) and DCM (50 mL × 2) were added sequentially. The organic phase was washed with saturated sodium bicarbonate solution (30 mL) and saturated brine (20 mL) in sequence. The solvent was evaporated under reduced pressure, and the resulting residue was purified by silica gel column chromatography (MeOH/EA (V/V) = 1/20) to obtain compound 46 (1.0 g, 72.7%) as a white solid.

MS (ESI, pos. ion) m/z: 602.3 [M+H]⁺;
¹H NMR (400 MHz, CD₃OD) δ 5.39 - 5.33 (m, 1H), 5.07 (dd, *J* = 11.2, 3.4 Hz, 1H), 4.58 (d, *J* = 8*.*4 Hz, 1H), 4.21 - 4.09 (m, 3H), 4.04 (t, *J =* 6.7 Hz, 1H), 3.89 (dt, *J =* 10.4, 5.1 Hz, 1H), 3.60 - 3.50 (m, 1H), 3.30 - 3.13 (m, 2H), 2.16 (s, 3H), 2.04 (s, 3H), 1.97 (s, 3H), 1.94 (s, 3H), 1.67 - 1.56 (m, 4H), 1.48 (s, 9H), 1.41 - 1.37 (m, 2H), 1.02 - 0.98 (m, 2H).

### Step 2: Synthesis of Compound 47

Compound 46 (0.72 g, 1.17 mmol) was dissolved in DCM (8 mL), and then TFA (0.87 mL, 11.7 mmol) was added. The reaction mixture was stirred at 25 °C for 16 h, and the solvent was concentrated to give compound 47 (0.74 g, 103.1%) as a brown oil.

MS (ESI, pos. ion) m/z:502.2 [M+H]⁺;
¹H NMR (400 MHz, CD₃OD) δ 5.36 (d, *J =* 3.3 Hz, 1H), 5.08 (dd, *J =* 11.3, 3.3 Hz, 1H), 4.58 (d, *J =* 8.4 Hz, 1H), 4.16 - 4.10 (m, 3H), 4.08 - 4.02 (m, 1H), 3.92 - 3.85 (m, 1H), 3.58 - 3.50 (m, 1H), 3.26 - 3.20 (m, 2H), 2.16 (s, 3H), 2.04 (d, *J =* 5.2 Hz, 6H), 1.98 (s, 3H), 1.97 (s, 3H), 1.61 - 1.56 (m, 4H), 1.54 - 1.52 (m, 2H), 1.42 - 1.38 (m, 2H).

### Step 3: Synthesis of Compound 48

Compound 6 (0.19 g, 0.3 mmol, i.e., compound 6 in Example 1, which was prepared according to the synthesis method of compound 6 in Example 1) and compound 47 (0.5 g, 0.99 mmol) were dissolved in DCM (30 mL). HOBT (0.17 g, 1.26 mmol), HBTU (0.48 g, 1.26 mmol), and DIPEA (0.5 mL, 3.0 mmol) were then added in sequence. The reaction mixture was stirred at 30 °C for 3 h. After the reaction was completed, water (20 mL) was added to quench the reaction, and the mixture was extracted with DCM (100 mL × 2). The organic phases were combined, washed with saturated sodium bicarbonate solution (40 mL) and saturated sodium chloride solution (40 mL) in sequence, and dried over anhydrous sodium sulfate. The solvent was evaporated under reduced pressure, and the resulting residue was purified by silica gel column chromatography (DCM to DCM/MeOH (V/V) = 10/1) to obtain compound 48 (0.23 g, 37%) as a white solid.

MS (ESI, pos. ion) m/z: 1046.3 [M/2+H]⁺;
¹H NMR (400 MHz, CD₃OD) δ 7.78 (t, *J =* 5.8 Hz, 2H), 7.38 - 7.36 (m, 3H), 5.36 (d, *J =* 3.4 Hz, 3H), 5.13 (s, 2H), 5.08 (dd, *J =* 11.2, 3.4 Hz, 3H), 4.58 (d, *J =* 8.4 Hz, 3H), 4.21 - 4.08 (m, 9H), 4.04 (t, *J =* 6.7 Hz, 3H), 3.93 - 3.82 (m, 3H), 3.73 - 3.66 (m, 12H), 3.60 - 3.52 (m, 3H), 3.29 - 3.18 (m, 6H), 2.52 (t, *J =* 6.0 Hz, 6H), 2.38 (t, *J =* 7.4 Hz, 2H), 2.20 (t, *J =* 7.7 Hz, 2H), 2.16 (s, 9H), 2.04 (s, 9H), 1.97 (s, 9H), 1.95 (s, 9H), 1.62 - 1.56 (m, 12H), 1.50 - 1.42 (m, 6H), 1.35 - 1.27 (m, 16H), 1.04 - 0.97 (m, 6H).

### Step 4: Synthesis of Compound 49

Compound 48 (0.20 g, 0.094 mmol) was dissolved in methanol (10 mL), and then Pd/C (10 mg, 10%) was added. The atmosphere was replaced with hydrogen three times, and the reaction mixture was stirred at room temperature for 11 h under a hydrogen atmosphere. After the reaction, the reaction mixture was filtered through celite, and the filtrate was evaporated to dryness under reduced pressure to yield compound 49 (0.19 g, 100%) as a white solid.

MS (ESI, pos. ion) m/z: 1001.1, [M/2+H]⁺.

### Step 5: Synthesis of Compound DAW40007-1

Compound 49 (0.28 g, 0.14 mmol) was dissolved in DCM (20 mL), and HOBT (0.038 g, 0.28 mmol), HBTU (0.080 g, 0.21 mmol), DIPEA (0.054 g, 0.42 mmol) and compound 13 (0.068 g, 0.16 mmol) were added sequentially. The reaction mixture was stirred at room temperature for 13 h. After completion of the reaction, water (10 mL) was added to quench the reaction, and the mixture was extracted with DCM (20 mL). The organic phase was washed with saturated sodium bicarbonate solution (10 mL), and the solvent was evaporated under reduced pressure. The residue was dissolved in acetonitrile (10 mL) and separated by reverse preparative column (acetonitrile/water solution (V/V) = 43% to 60%, 50 min). Salt was added to the solution containing the product after preparative separation to saturate the solution. The organic phase was separated, and the aqueous phase was washed with acetonitrile (100 mL×2) , The organic phases were combined and dried over anhydrous sodium sulfate. The solvent was evaporated under reduced pressure. The residue was added with acetonitrile (30 mL), dried over sodium sulfate, filtered, and the filtrate was concentrated under reduced pressure to obtain compound DAW40007-1 (0.08 g, 24%) as a light yellow solid.

MS (ESI, neg. ion) m/z: 2400.18 [M-H]⁻;
¹H NMR (400 MHz, DMSO-*d₆*) δ 8.31 (s, 3H), 7.81 (d, *J =* 9.2 Hz, 3H), 7.57 (t, *J =* 6.0 Hz, 3H), 7.35 - 7.26 (m, 4H), 7.20 (td, *J =* 8.9, 3.0 Hz, 5H), 6.95 (s, 1H), 6.88 (ddd, *J* = 8.8, 5.8, 2.2 Hz, 4H), 5.22 (d, *J =* 3.4 Hz, 3H), 4.97 (dd, *J =* 11.2, 3.5 Hz, 4H), 4.49 (d, *J =* 8.5 Hz, 3H), 4.40 (d, *J* = 4.8 Hz, 1H), 4.15 (s, 1H), 4.07 - 4.00 (m, 9H), 3.88 (dt, *J =* 11.2, 8.8 Hz, 3H), 3.74 (s, 9H), 3.59 - 3.48 (m, 12H), 3.17 (dd, *J =* 8.8, 5.0 Hz, 1H), 3.10 - 2.95 (m, 8H), 2.35 (t, *J =* 6.3 Hz, 6H), 2.10 (s, 9H), 2.08 (s, 3H), 2.04 (d, *J =* 4.7 Hz, 2H), 2.00 (s, 9H), 1.89 (s, 9H), 1.78 (s, 9H), 1.40 (d, *J =* 12.2 Hz, 17H), 1.31 - 1.16 (m, 18H), 0.79 (q, *J =* 3.2 Hz, 6H).

### Step 6: Synthesis of Compound DAW40007-2

Compound DAW40007-1 (0.080 g, 0.033 mmol) was dissolved in DCM (10 mL), and DIPEA (0.029 mL, 0.17 mmol), succinic anhydride (0.008 g, 0.083 mmol) and DMAP (0.014 g, 0.12 mmol) were added. The mixture was stirred at 40 °C for 5 h, and succinic anhydride (10 mg) was added. After the reaction was continued for 16 h, DCM (10 mL), succinic anhydride (10 mg) and DIPEA (0.05 mL) were added. The mixture was stirred for another 9 h, and then succinic anhydride (10 mg) was added. After the reaction was continued for another 10 h, DCM (20 mL) was added to dilute the mixture, and the mixture was washed with saturated sodium bicarbonate solution (10 mL). The aqueous phase was discarded, and the organic phase was dried over anhydrous sodium sulfate. The solvent was concentrated under reduced pressure to obtain compound DAW40007-2 (0.08 g, 96.07%) as a white solid. MS (ESI, neg. ion) m/z: 2500.12 [M-H]⁻.

¹H NMR (400 MHz, DMSO-*d₆*) δ 8.45 (d, *J =* 6.6 Hz, 2H), 7.85 (d, *J =* 9.2 Hz, 3H), 7.62 (d, *J =* 5.2 Hz, 3H), 7.37 - 7.26 (m, 4H), 7.25 - 7.17 (m, 5H), 7.01 (s, 1H), 6.92 - 6.83 (m, 4H), 5.22 (d, *J=* 3.4 Hz, 3H), 4.98 (dd, *J =* 11.2, 3.4 Hz, 3H), 4.50 (d, *J =* 8.5 Hz, 3H), 4.24 - 4.12 (m, 2H), 4.03 (s, 9H), 3.93 - 3.85 (m, 4H), 3.74 (s, 6H), 3.72 - 3.64 (m, 4H), 3.63 - 3.48 (m, 13H), 3.47 - 3.38 (m, 4H), 3.28 - 3.19 (m, 1H), 3.10 - 3.01 (m, 6H), 2.35 (t, *J =* 6.6 Hz, 8H), 2.25 - 2.17 (m, 2H), 2.11 (s, 9H), 2.07 - 2.03 (m, 2H), 2.00 (s, 9H), 1.89 (s, 9H), 1.78 (s, 9H), 1.48 - 1.36 (m, 16H), 1.27 - 1.15 (m, 20H), 0.82 - 0.75 (m, 6H).

### Step 7: Synthesis of Compound DAW40007-3

Compound DAW40007-2 (0.08 g, 0.032 mmol), HBTU (0.015 g, 0.04 mmol), and DIPEA (0.011 mL, 0.064 mmol) were dissolved in ACN (5 mL) and stirred at room temperature for 5 min. Then, the mixture was transferred to a solid-phase synthesizer containing 0.35 g of H₂N-CPG (purchased from Hebei Dinaxingke) and shaken for 22.5 h. The mixture was filtered, and the filter cake was rinsed with DCM/MeOH (V/V = 9/1, 10 mL) and DCM (10 mL), and then dried under reduced pressure. The resulting filter cake was diluted in 25% Ac₂O/Py solution (5 mL) and stirred for 3 h. The mixture was filtered, and the filter cake was rinsed with DCM/MeOH (V/V = 9/1, 10 mL) and DCM (10 mL) in sequence, then dried under reduced pressure to obtain DAW40007-3 (0.357 g) as a white solid with a measured loading of 14.95 µmol/g.

### Synthesis of Compound L96

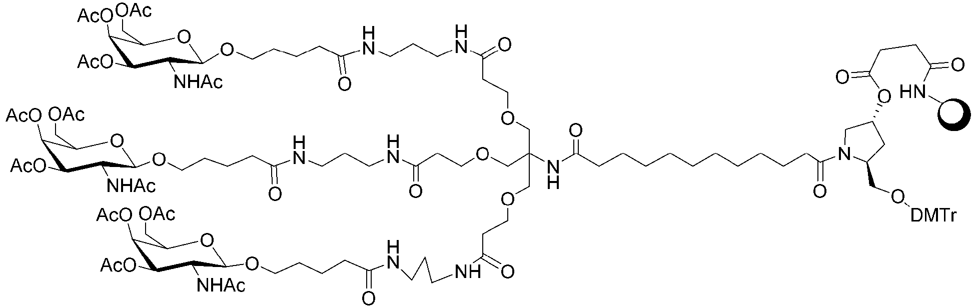

Compound L96 was prepared according to the method described in patent application WO2014025805A1.
The conjugated group structure of L96 is:

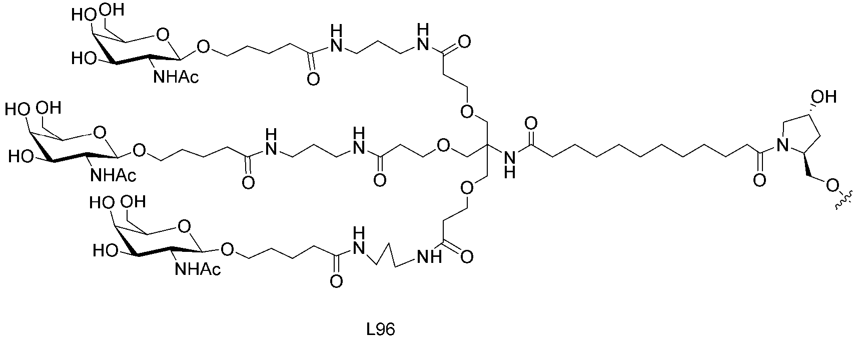

Alternatively, the compound of the present invention is linked to an siRNA molecule according to methods known in the art, followed by deprotection to produce an siRNA conjugate containing the conjugated group of the present invention. For example, DAW40007-3 is linked to an siRNA molecule and the protecting group is removed to produce an siRNA conjugate containing the conjugated group of DAW40007-4 (i.e., an siRNA drug).

### Example 5: Synthesis of siRNA conjugate

The synthesis method of the Antisense Strand us-Gfs-u-g-a-Agn-g-Cf-Gf-a-a-g-u-Gf-c-Af-c-a-cs-us-u (SEQ ID NO: 7) is as follows:
The synthesis was completed with a theoretical yield of 1 µmol. Weigh 1 µmol of the solid phase support CPG (purchased from Hebei Dinaxingke), 2'-methoxynucleoside monomers and 2'-fluoronucleoside monomers (purchased from Wuhu Huaren Technology), and AgN monomer (purchased from Beijing Coupling Technology Co., Ltd.) in a 0.1 M anhydrous acetonitrile solution. For oligonucleotides with phosphate backbone thiolation modifications, a 0.1 M DDTT solution was used as the thiolation reagent. 5-Ethylthio-1H-tetrazole acetonitrile solution (0.25 M) was used as the activating agent (purchased from Suzhou Kelema), a 0.02 M iodine-in-pyridine/water solution was used as the oxidizing agent, and a 3% trichloroacetic acid solution in dichloromethane was used as the deprotection reagent. These were placed in the designated reagent compartment of the KA-H8 automated DNA/RNA synthesizer. Set up the synthesizer and input the specified oligonucleotide base sequence. After verification, initiated the oligonucleotide synthesis cycle. The coupling time for each step was 6 minutes, and the sulfurization time was 6 minutes. After automatic cycling, the oligonucleotide containing the solid support CPG was obtained.

Dried the obtained nucleotide containing the solid support CPG using dry argon gas, then transfered it to a 2 mL EP tube and added 28% ammonia solution (1.8 mL), heated at 55°C for 5 to 18 hours. Filtered the mixture, washed the filter cake with water (0.5 mL), and combined the filtrates. After concentrating under reduced pressure, a white or yellow gelatinous solid was obtained. After reverse phase preparative purification, the preparation solution was concentrated and passed through a gel column to remove excess salt to obtain oligonucleotides. The concentration of the resulting oligonucleotide was determined using a micro-volume UV spectrophotometer (SPECTRO stat Nano). Analysis was performed with mass spectrometry on an Agilent 6530 LC-MS Q-Tof system. The molecular weight of the nucleic acid was calculated after primary scanning and deconvolution. The measured values were consistent with the theoretical values, indicating that the target antisense strand was synthesized.

### Synthesis of the Sense Strand of siRNA Conjugates:

The generic solid-phase support CPG was replaced with the GalNAc solid-phase support CPG prepared in this invention. The sense strand of the siRNA conjugates of this invention was prepared using the same method for synthesizing the antisense strand. The measured molecular weight was consistent with the theoretical value, indicating that the target siRNA sense strand was synthesized. The HPLC purity and measured molecular weight of the sense strand of some siRNA conjugates are shown in Table 1 below:

**Table 1: the HPLC purity and measured molecular weight of the sense strand of some siRNA conjugates**

| The numbers of the sense strand of siRNA conjugates | the sense strand of siRNA conjugates | SEQ ID NO | HPLC purity | molecular weight (the theoretical values) | molecular weight (the measured values) |
|---|---|---|---|---|---|
| DAW30025S S | gs-us-g-u-Gf-c-Af-Cf-Uf-u-c-g-c- u-u-c-a-c-a-L96 | 1 | 94.21% | 8014.18 | 8014.2876 |
| DAW30062S S | gs-us-g-u-Gf-c-Af-Cf-Uf-u-c-g-c- u-u-c-a-c-a-DAW40003-4 | 3 | 99.15% | 8050.05 | 8050.30 |
| DAW30219S S | gs-us-g-u-Gf-c-Af-Cf-Uf-u-c-g-c- u-u-c-a-c-a-DAW40004-4 | 4 | 96.0% | 8092.00 | 8092.34 |
| DAW30221S S | gs-us-g-u-Gf-c-Af-Cf-Uf-u-c-g-c- u-u-c-a-c-a-DAW40005-4 | 5 | 93.14% | 8050.05 | 8050.16 |
| DAW30223S S | gs-us-g-u-Gf-c-Af-Cf-Uf-u-c-g-c- u-u-c-a-c-a-DAW40002-4 | 2 | 99.72% | 8050.05 | 8050.19 |
| DAW30222S S | gs-us-g-u-Gf-c-Af-Cf-Uf-u-c-g-c- u-u-c-a-c-a-DAW40007-4 | 6 | 99.83% | 8008.50 | 8008.35 |

Annealing Step: The sense strand of the siRNA conjugate (obtained from the synthesis above) was mixed with an equimolar amount of the antisense strand (also obtained above). The mixture was heated to 95 °C and maintained at this temperature for 10 minutes, then slowly cooled to room temperature. The resulting annealed product was then lyophilized to obtain the target siRNA conjugate. The numbers and sequences are listed in Table 2 below.

**Table 2: siRNA conjugate numbers and their corresponding sense strands (including conjugate groups) and antisense strands**

| The numbers of siRNA conjugates | SS(5'-3') | SEQ ID NO | AS(5'-3') | SEQ ID NO |
|---|---|---|---|---|
| DAW30025 | gs-us-g-u-Gf-c-Af-Cf-Uf-u-c-g- c-u-u-c-a-c-a-L96 | 1 | us-Gfs-u-g-a-Agn-g-Cf-Gf-a-a- g-u-Gf-c-Af-c-a-cs-us-u | 7 |
| DAW30223 | gs-us-g-u-Gf-c-Af-Cf-Uf-u-c-g- c-u-u-c-a-c-a-DAW40002-4 | 2 | us-Gfs-u-g-a-Agn-g-Cf-Gf-a-a- g-u-Gf-c-Af-c-a-cs-us-u | 7 |
| DAW30062 | gs-us-g-u-Gf-c-Af-Cf-Uf-u-c-g- c-u-u-c-a-c-a-DAW40003-4 | 3 | us-Gfs-u-g-a-Agn-g-Cf-Gf-a-a- g-u-Gf-c-Af-c-a-cs-us-u | 7 |
| DAW30219 | gs-us-g-u-Gf-c-Af-Cf-Uf-u-c-g- c-u-u-c-a-c-a-DAW40004-4 | 4 | us-Gfs-u-g-a-Agn-g-Cf-Gf-a-a- g-u-Gf-c-Af-c-a-cs-us-u | 7 |
| DAW30221 | gs-us-g-u-Gf-c-Af-Cf-Uf-u-c-g- c-u-u-c-a-c-a-DAW40005-4 | 5 | us-Gfs-u-g-a-Agn-g-Cf-Gf-a-a- g-u-Gf-c-Af-c-a-cs-us-u | 7 |
| DAW30222 | gs-us-g-u-Gf-c-Af-Cf-Uf-u-c-g- c-u-u-c-a-c-a-DAW40007-4 | 6 | us-Gfs-u-g-a-Agn-g-Cf-Gf-a-a- g-u-Gf-c-Af-c-a-cs-us-u | 7 |
| DAW30255 | gs-us-c-a-u-c-Cf-a-Cf-Af-Af-u-g -a-g-a-g-u-a-c-a-DAW40007-4 | 8 | us-Gfs-u-a-c-(Tgn)-c-u-c-a-u-u -g-Uf-g-Gf-a-u-g-a-cs-gs-a | 9 |

The base letters are lowercase 2'-OMe modified, f is 2'-F modified, s is phosphate backbone thio modification, Agn is adenosine-glycol nucleic acid (GNA) modified, Tgn represents a thymine-glycol nucleotide residue, DAW40002-4, DAW40003-4, DAW40004-4, DAW40005-4 and DAW40007-4 are the conjugated groups described in the present invention, each of which is independently conjugated to the 3' end of the sense strands through a phosphate group.

### Example 7: Hepatitis B Virus Transgenic Mice Experiment

### Experimental Methods

Experimental Animals: HBV-Tg transgenic mice, C57B/6N-Tg(1.28HBV)/Vst, SPF-grade male, 6-8 weeks old, 16-20 g, purchased from Beijing Weitongda Biotechnology Co., Ltd., Animal Production Certificate No.: SCXK(Beijing)2019-0002. Animals were maintained by Beijing Weitongda Biotechnology Co., Ltd. and housed individually in single cages.

Animal Grouping: Based on the quantitative results of serum HBsAg (primary) and HBeAg (secondary), animals were randomly divided into stratified groups, with 5 mice per group.

Test Article Preparation and Administration: Calculate the required dosage of drug powder based on the purity of each test article. Add the appropriate saline solution at a dosing concentration of 0.6 mg/mL and vortex to mix until a colorless, transparent liquid is obtained. The day of the first dose was designated Day 0. All groups received a single subcutaneous dose of 5 mL/kg on Day 0. If blood sampling was required on the day of dosing, administration was performed after blood collection.

Main Outcome Measures: animals were weighed and blood was collected once on days 3, 7, 14, 21, 28, and 35. Serum was separated for HBsAg and HBeAg level determination.

Serum HBsAg and HBeAg Level Determination: After blood collection, serum was separated, diluted with PBS, and submitted for testing. 10 µL of serum was collected from each sample and diluted to 500 µL with PBS (50-fold dilution). Serum HBsAg and HBeAg levels were measured using a Hepatitis B e Antigen Assay Kit (Mike Biotech) and a Hepatitis B Surface Antigen Assay Kit (Mike Biotech), respectively.

### Results of experiments in hepatitis B virus transgenic mice

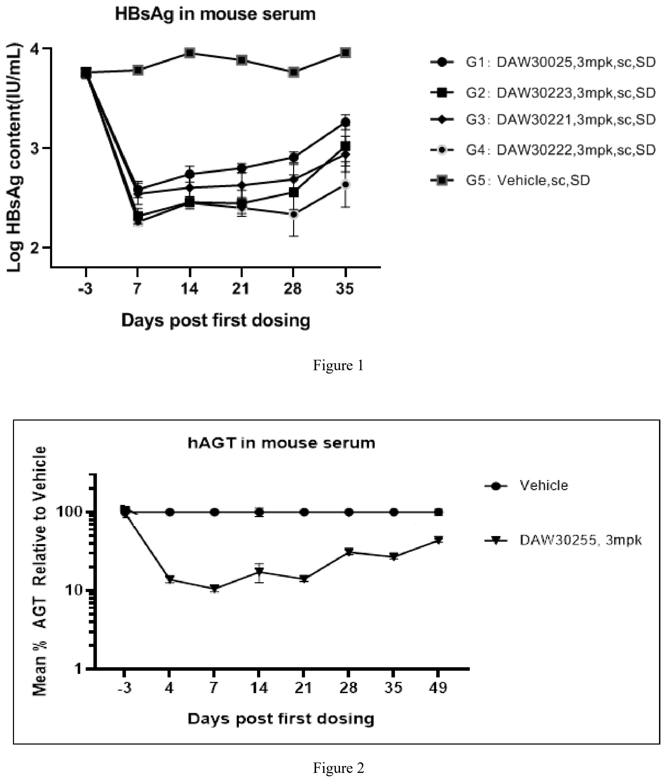

The experimental results demonstrate that the siRNA conjugates of the present invention could significantly reduce HBsAg in the Tg/HBV mouse model. Furthermore, compared to the L96 vector used in four currently marketed Galnac-siRNAs, while maintaining the same sequence, the siRNA conjugates of the present invention were superior to the DAW30025 (the siRNA conjugate using L96) in reducing HBsAg, as were the siRNA conjugates DAW30221, DAW30222, and DAW30223 of the present invention (the vectors, also known as conjugation groups, are DAW40002-4, DAW40005-4, and DAW40007-4, respectively). The siRNA conjugates of the present invention significantly outperformed the DAW30025 (the siRNA conjugate using L96) in reducing HBsAg, and their HBsAg-lowering effect was more sustained (see Figure 1 for the HBsAg-inhibiting effects of the conjugates DAW30221, DAW30222, DAW30223, and DAW30025). In addition, the siRNA conjugates of the present invention also demonstrated a significant inhibitory effect on HBeAg in a Tg/HBV mouse model.

### Example 8: In Vivo Knockdown Activity Testing of AGT siRNA Conjugates in Mice

### Experimental Methods:

To evaluate the *in vivo* activity of the siRNA conjugates coupled with the GalNAc vector of the present invention against AGT, an AGT humanized mouse model (6-9 mice per group) was used. Baseline serum samples were collected from each group of mice 3 days prior to administration. On D0, a single dose of 1 mg/kg or 3 mg/kg of the present GalNAc-siRNA or vehicle was administered subcutaneously at the nape of the neck. Blood samples were collected on D4/7/14/21/28/35/49, and human AGT protein concentrations were measured using an ELISA (ab287170). The knockdown percentage of human AGT was calculated by comparing the human AGT protein levels in the siRNA group with those in the vehicle group. The knockdown effect of DAW30255 on AGT is shown in Figure 2. Experimental results demonstrate that the siRNA conjugate of the present invention significantly reduces AGT expression. Therefore, DAW30255 of the present invention has promising application prospects in the treatment of hypertension.

The aforementioned mouse experiments demonstrate that the GalNAc vector of the present invention effectively delivers siRNA drugs targeting AGT and HBV. According to common knowledge in the art, the present invention specifically utilizes a vector for liver-targeted delivery of an anti-hepatitis B drug for the treatment of hepatitis B, or an AGT drug for the treatment of hypertension, demonstrating excellent delivery efficacy. Therefore, the vector of the present invention is also applicable to the delivery of liver-targeted drugs for other indications.

While the present invention has been described in detail above using general explanations, specific embodiments, and experiments, it will be readily apparent to those skilled in the art that modifications and improvements may be made to the present invention. Accordingly, such modifications and improvements, as long as they do not depart from the spirit of the present invention, are within the scope of protection claimed herein.

## Claims

1. A compound having a structure as shown in formula (I), or a stereoisomer, tautomer, or acceptable salt of the compound as shown in formula (I), 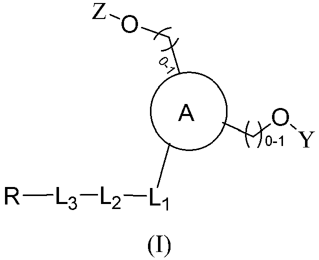 wherein Ring A is a heterocyclic group consisting of 3-12 ring atoms or a cycloalkyl group consisting of 3-12 ring atoms;
Z and Y are each independently H, deuterium, a hydroxyl protecting group, HOC(=O)(CH₂)ⱼC(=O)- or M-C(=O)(CH₂)ⱼC(=O)-;
M is a solid support, preferably a hydroxyl- or amino-functionalized solid support, more preferably a resin or CPG, further preferably a macroporous resin or CPG, and even more preferably an aminomethyl resin, hydroxyl resin or -NHCPG;
each of L₁, L₂ and L₃ is independently C₁₋₁₂ alkylene, C₃₋₁₂ cycloalkyl, heterocyclic group consisting of 3-12 ring atoms, C₁₋₁₂ heteroalkyl, C₂₋₁₂ alkenyl, C₂₋₁₂ alkynyl, -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of the C₁₋₁₂ alkylene, C₃₋₁₂ cycloalkyl, heterocyclyl consisting of 3-12 ring atoms, C₁₋₁₂ heteroalkyl, C₂₋₁₂ alkenyl and C₂₋₁₂ alkynyl groups is independently optionally substituted by 1, 2, 3 or 4 R^{W1}, and 1, 2, 3, 4 or 5 methylene groups in each of the C₁₋₁₂ alkylene, C₁₋₁₂ heteroalkyl, C₂₋₁₂ alkenyl and C₂₋₁₂ alkynyl groups is independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-;
R is a structure represented by the following Formula (a) or Formula (b): 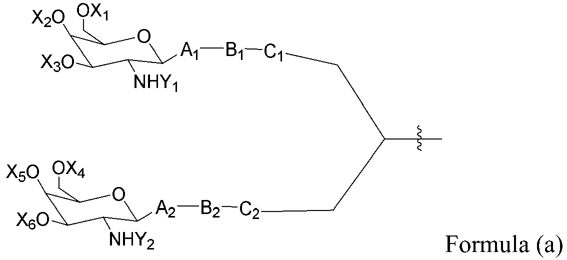 or 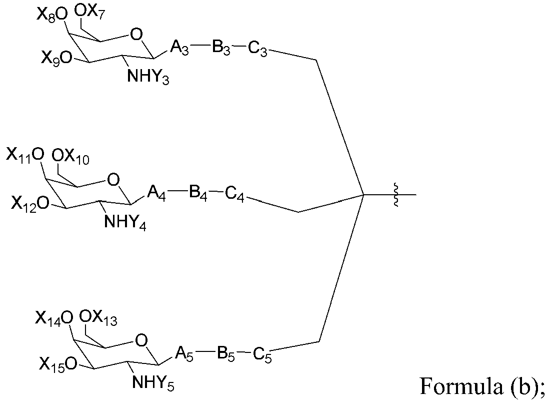
wherein each of X₁, X₂, X₃, X₄, X₅, X₆, X₇, X₈, X₉, X₁₀, X₁₁, X₁₂, X₁₃, X₁₄ and X₁₅ is independently H or a hydroxyl protecting group; each of Y₁, Y₂, Y₃, Y₄ and Y₅ is independently H or an amino protecting group;
each of A₁, A₂, A₃, A₄, A₅, C₁, C₂, C₃, C₄ and C₅ is independently a bond, C₁₋₁₂ alkylene, C₃₋₁₂ cycloalkyl, a heterocyclyl consisting of 3-12 ring atoms, C₁₋₁₂ heteroalkyl, C₂₋₁₂ alkenyl or C₂₋₁₂ alkynyl, wherein each of the C₁₋₁₂ alkylene, C₃₋₁₂ cycloalkyl, heterocyclyl consisting of 3-12 ring atoms, C₁₋₁₂ heteroalkyl, C₂₋₁₂ alkenyl and C₂₋₁₂ alkynyl groups is independently optionally substituted by 1, 2, 3 or 4 R^{W2}, and 1, 2, 3, 4 or 5 methylene groups in each of the C₁₋₁₂ alkylene, C₁₋₁₂ heteroalkyl, C₂₋₁₂ alkenyl and C₂₋₁₂ alkynyl groups are each independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-;
each of B₁, B₂, B₃, B₄ and B₅ is independently a C₃₋₁₂ cycloalkyl, a heterocyclyl consisting of 3-12 ring atoms, a C₁₋₁₂ alkylene, a C₁₋₁₂ heteroalkyl, a C₂₋₁₂ alkenyl or a C₂₋₁₂ alkynyl, wherein at least one of B₁ and B₂ is a C₃₋₁₂ cycloalkyl or a heterocyclyl consisting of 3-12 ring atoms, and at least one of B₃, B₄ and B₅ is a C₃₋₁₂ cycloalkyl or a heterocyclyl consisting of 3-12 ring atoms, each of the C₃₋₁₂ cycloalkyl, heterocyclyl consisting of 3-12 atoms, C₁₋₁₂ alkylene, C₁₋₁₂ heteroalkyl, C₂₋₁₂ alkenyl and C₂₋₁₂ alkynyl groups is independently optionally substituted by 1, 2, 3 or 4 R^{W3}, and 1, 2, 3, 4 or 5 methylene groups in each of the C₁₋₁₂ alkylene, C₁₋₁₂ heteroalkyl, C₂₋₁₂ alkenyl and C₂₋₁₂ alkynyl groups are independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-;
each R¹ is independently H, deuterium or C₁₋₆ alkyl;
each j is independently 1, 2, 3, 4 or 5;
each of R^{W1}, R^{W2} and R^{W3} is independently deuterium, =O, hydroxy, nitro, cyano, F, Cl, Br, I, C₁₋₆ alkyl, C₁₋₆ alkoxy, C₃₋₆ cycloalkyl, C₂₋₆ alkenyl or C₁₋₆ heteroalkyl.

2. The compound according to claim 1, wherein each of L₁, L₂ and L₃ is independently C₃₋₁₂ alkylene, C₃₋₆ cycloalkyl, a heterocyclic group consisting of 3-6 ring atoms, C₂₋₁₀ heteroalkyl, C₂₋₁₀ alkenyl, C₂₋₁₀ alkynyl, -O-, -S-, -S(=O)-, -S(=O)₂-, -S(=O)(=NR¹)-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- or -N(R¹)-, wherein each of the C₃₋₁₂ alkylene, C₃₋₆ cycloalkyl, heterocyclyl consisting of 3-6 ring atoms, C₂₋₁₀ heteroalkyl, C₂₋₁₀ alkenyl and C₂₋₁₀ alkynyl groups is independently optionally substituted by 1, 2, 3 or 4 R^{W1}, and 1, 2, 3, 4 or 5 methylene groups in each of the C₃₋₁₂ alkylene, C₂₋₁₀ heteroalkyl, C₂₋₁₀ alkenyl and C₂₋₁₀ alkynyl groups areindependently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-.

3. The compound according to claim 1 or 2, wherein each of A₁, A₂, A₃, A₄, A₅, C₁, C₂, C₃, C₄ and C₅ is independently a bond, C₃₋₁₂ alkylene, C₃₋₆ cycloalkyl, a heterocyclic group consisting of 3-6 ring atoms, C₂₋₁₀ heteroalkyl, C₂₋₁₀ alkenyl and C₂₋₁₀ alkynyl, wherein each of the C₃₋₁₂ alkylene, C₃₋₆ cycloalkyl, heterocyclyl consisting of 3-6 ring atoms, C₂₋₁₀ heteroalkyl, C₂₋₁₀ alkenyl and C₂₋₁₀ alkynyl groups is independently optionally substituted by 1, 2, 3 or 4 R^{W2} and 1, 2, 3, 4 or 5 methylene groups in each of the C₃₋₁₂ alkylene, C₂₋₁₀ heteroalkyl, C₂₋₁₀ alkenyl and C₂₋₁₀ alkynyl groups are each independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-.

4. The compound according to any one of claims 1 to 3, wherein each of B₁, B₂, B₃, B₄ and B₅ is independently cyclopropyl, cyclobutyl, cyclopentyl, cyclohexyl, a heterocyclic group consisting of 3 to 6 ring atoms, C₁₋₆ alkylene, C₁₋₆ heteroalkyl, C₂₋₆ alkenyl or C₂₋₆ alkynyl, wherein at least one of B₁ and B₂ is a cyclopropyl, cyclobutyl, cyclopentyl, cyclohexyl or a heterocyclic group consisting of 3-6 ring atoms, at least one of B₃, B₄ and B₅ is a cyclopropyl, cyclobutyl, cyclopentyl, cyclohexyl or a heterocyclic group consisting of 3-6 ring atoms, each of the cyclopropyl, cyclobutyl, cyclopentyl, cyclohexyl, a heterocyclic group consisting of 3-6 ring atoms, C₁₋₆ alkylene, C₁₋₆ heteroalkyl, C₂₋₆ alkenyl and C₂₋₆ alkynyl groups is independently optionally substituted with 1, 2, 3 or 4 R^{W3}, and 1, 2, 3, 4 or 5 methylene groups in each of the C₁₋₆ alkylene, C₁₋₆ heteroalkyl, C₂₋₆ alkenyl and C₂₋₆ alkynyl groups are each independently optionally replaced by 1, 2, 3, 4 or 5 groups selected from -O-, -S-, -S(=O)-, -S(=O)₂-, -C(=O)-, -C(=O)O-, -OC(=O)-, -C(=O)C(=O)-, -C(=O)N(R¹)-, -N(R¹)C(=O)-, -OC(=O)N(R¹)-, -N(R¹)C(=O)O-, -N(R¹)C(=O)N(R¹)-, -C(=O)N(R¹)C(=O)-, -S(=O)₂N(R¹)-, -N(R¹)S(=O)₂- and -N(R¹)-.

5. The compound according to any one of claims 1 to 4, wherein Z and Y are each independently H, deuterium, C₁₋₁₂ alkyl, C₁₋₁₂ alkylC(=O)-, C₁₋₁₂ alkylmethyl, C₁₋₁₂ alkylsilyl, C₆₋₁₀ arylC₁₋₆ alkyl, HOC(=O)(CH₂)ⱼC(=O)-, M-C(=O)(CH₂)ⱼC(=O)-, triphenylmethyl, MMTr, DMTr or 4',4',4'-trimethoxytrityl; or
Z and Y are each independently H, deuterium, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, p-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methylsulfonyl, p-toluenesulfonyl, C₁₋₁₀ alkyl, C₁₋₁₀ alkylC(=O)-, C₁₋₁₀ alkylmethyl, C₁₋₁₀ alkylsilyl, C₆₋₁₀ arylC₁₋₄ alkyl, HOC(=O)(CH₂)ⱼC(=O)-, M-C(=O)(CH₂)ⱼC(=O)-, triphenylmethyl, MMTr, DMTr or 4',4',4'-trimethoxytrityl; or
Z and Y are each independently H, deuterium, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, p-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methylsulfonyl, p-toluenesulfonyl, C₁₋₈ alkyl, C₁₋₈ alkylC(=O)-, C₁₋₈ alkylmethyl, C₁₋₈ alkylsilyl, phenylC₁₋₃ alkyl, HOC(=O)(CH₂)ⱼC(=O)-, M-C(=O)(CH₂)ⱼC(=O)-, triphenylmethyl, MMTr, DMTr or 4',4',4'-trimethoxytrityl; or
Z and Y are each independently H, deuterium, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, p-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methylsulfonyl, p-toluenesulfonyl, C₁₋₆ alkyl, C₁₋₆ alkylC(=O)-, C₁₋₆ alkylmethyl, C₁₋₆ alkylsilyl, benzyl, phenethyl, HOC(=O)(CH₂)ⱼC(=O)-, M-C(=O)(CH₂)ⱼC(=O)-, triphenylmethyl, MMTr, DMTr or 4',4',4'-trimethoxytrityl;
wherein the ring A is a heterocyclic group consisting of 3-7 ring atoms or a cycloalkyl group consisting of 3-6 atoms, more preferably a cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl.

6. The compound according to any one of claims 1 to 5, wherein each of X₁, X₂, X₃, X₄, X₅, X₆, X₇, X₈, X₉, X₁₀, X₁₁, X₁₂, X₁₃, X₁₄ and X₁₅ is independently H, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, methyl, tert-butyl, 2-trimethylsilylethyl, 1,1-dimethyl-2-propenyl, 3-methyl-3-butenyl, allyl, benzyl, p-methoxybenzyldiphenylmethyl, trityl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, p-toluenesulfonyl, trimethylsilyl, triethylsilyl, triisopropylsilyl, C₁₋₆ alkoxy or R²C(=O)-;
each of Y₁, Y₂, Y₃, Y₄, and Y₅ is independently H, 9-fluorenylmethoxycarbonyl or R³C(=O)-;
alkoxy, phenyl, halogen-substituted phenyl, C₁₋₆ alkylphenyl or benzyl; or
each of R² and R³ is independently C₁₋₄ alkyl, halogenated C₁₋₆ alkyl, C₁₋₄ alkoxy, phenyl, halogen-substituted phenyl, C₁₋₄ alkylphenyl, or benzyl; or
each R² and R³ is independently methyl, ethyl, n-propyl, isopropyl, trifluoromethyl, difluoromethyl, monofluoromethyl, trichloromethyl, dichloromethyl, monochloromethyl, 2,2,2-trichloroethyl, methoxy, ethoxy, 1-propoxy, 2-propoxy, 1-butoxy, 2-methyl-1-propoxy, 2-butoxy, tert-butoxy, phenyl, halogen-substituted phenyl, C₁₋₃ alkylphenyl or benzyl;
each R¹ is independently H, deuterium or C₁₋₄ alkyl;
each of R^{W1}, R^{W2} and R^{W3} is independently deuterium, =O, hydroxy, nitro, cyano, F, Cl, Br, I, methyl, ethyl, n-propyl, isopropyl, n-butyl, isobutyl, methoxy, ethoxy, 1-propoxy, 2-propoxy, 1-butoxy, 2-methyl-1-propoxy, 2-butoxy, cyclopropyl, cyclobutyl, cyclopentyl, cyclohexyl, C₂₋₄ alkenyl or C₁₋₄ heteroalkyl.

7. The compound according to any one of claims 1 to 6, wherein R is the structure represented by the following Formula (a-1) or Formula (b-1): 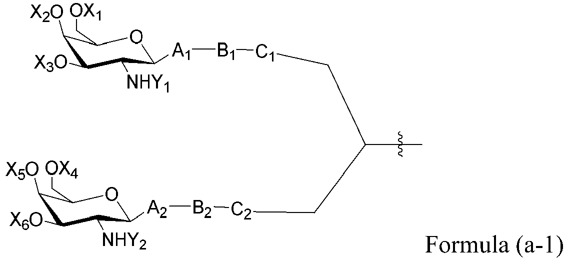 or 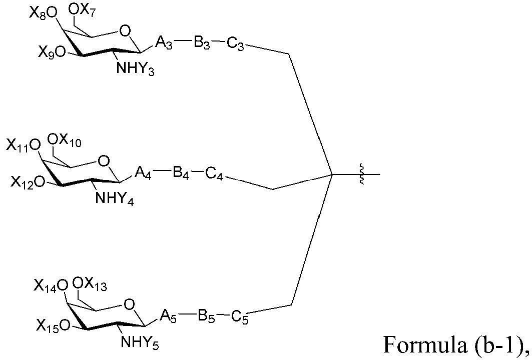 wherein each of X₁, X₂, X₃, X₄, X₅, X₆, X₇, X₈, X₉, X₁₀, X₁₁, X₁₂, X₁₃, X₁₄, X₁₅, Y₁, Y₂, Y₃, Y₄, Y₅, A₁, A₂, A₃, A₄, A₅, B₁, B₂, B₃, B₄, B₅, C₁, C₂, C₃, C₄ and C₅ has the meaning as defined in any one of claims 1-6.

8. The compound according to any one of claims 1-7, having the structure represented by Formula (II), or a stereoisomer, tautomer, or acceptable salt of the compound represented by Formula (II): 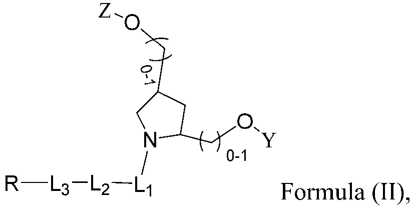 wherein each of Z, Y, L₁, L₂, L₃ and R has the meaning as defined in any one of claims 1-6.

9. The compound according to any one of claims 1to 8, having the structure represented by Formula (III), or a stereoisomer, tautomer, or acceptable salt of the compound represented by Formula (III): 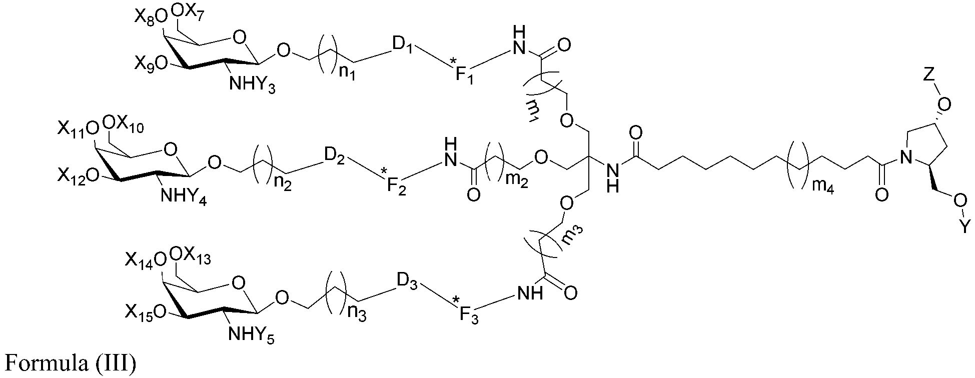 wherein, each of D₁, D₂ and D₃ is independently -C(=O)-*, -C(=O)O-*, -OC(=O)-*, -C(=O)N(R¹)-*, -N(R¹)C(=O)-*, -C(=O)N(R¹)CH₂-* or -N(R¹)C(=O)CH₂-*;
each of F₁, F₂, and F₃ is independently cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl;
wherein each of R¹, X₇, X₈, X₉, X₁₀, X₁₁, X₁₂, X₁₃, X₁₄, X₁₅, Y₃, Y₄, Y₅, Z and Y has the meaning as defined in any one of claims 1 to 6.

10. The compound according to any one of claims 1 to 9, which is one of the following compounds, or a stereoisomer, tautomer, or acceptable salt thereof, 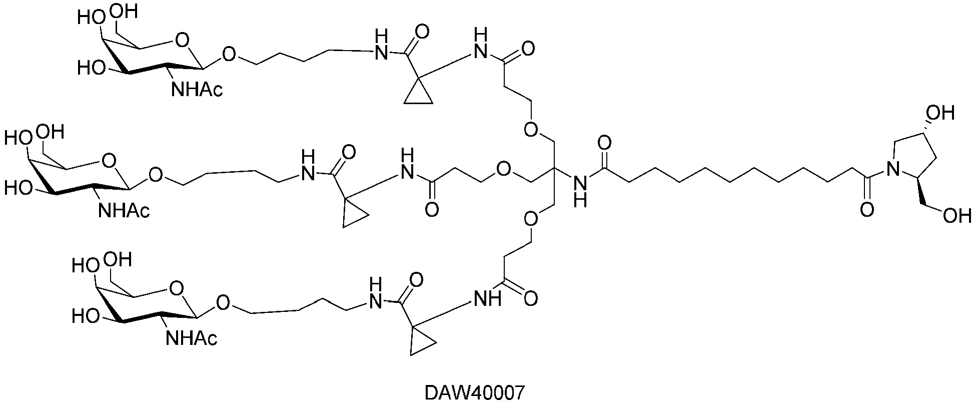 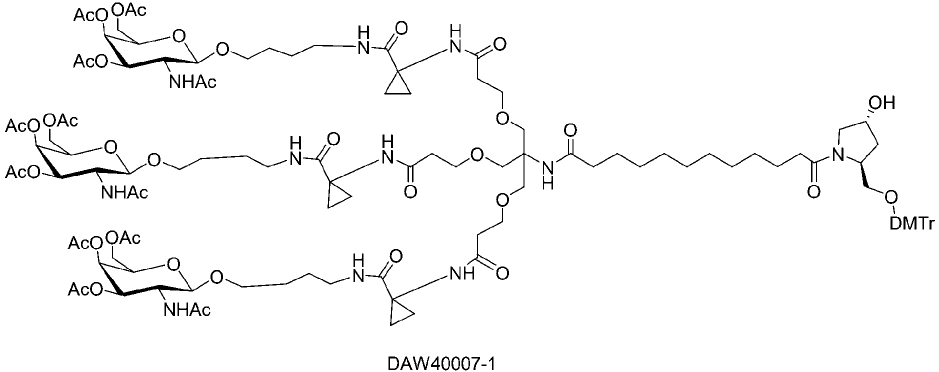 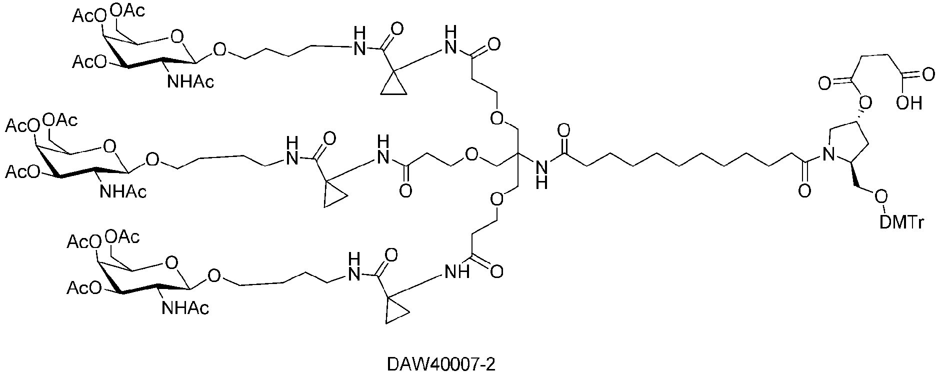 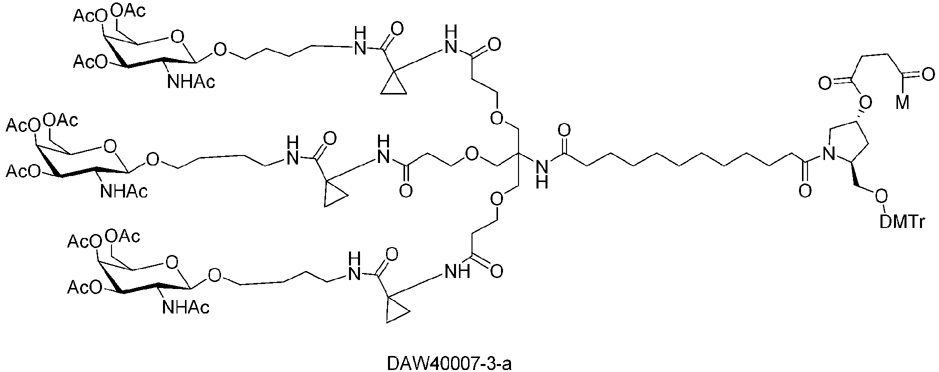 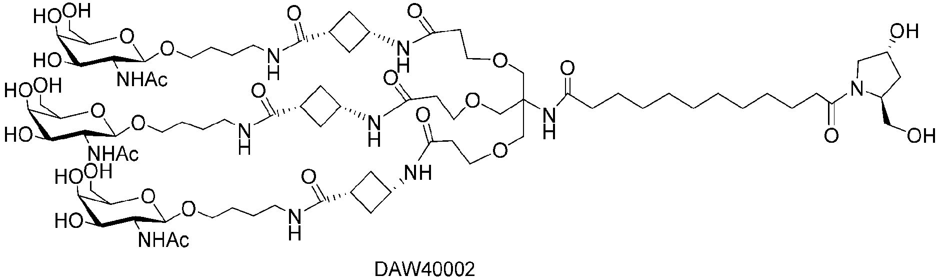 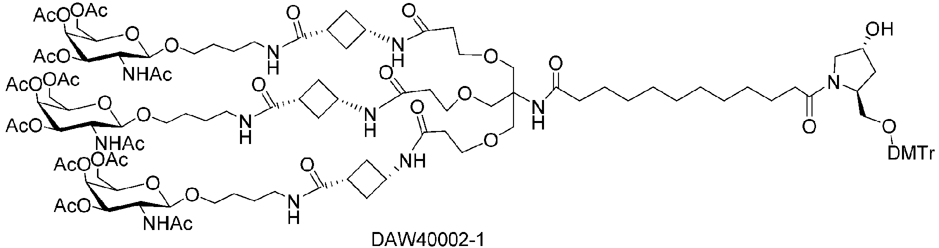 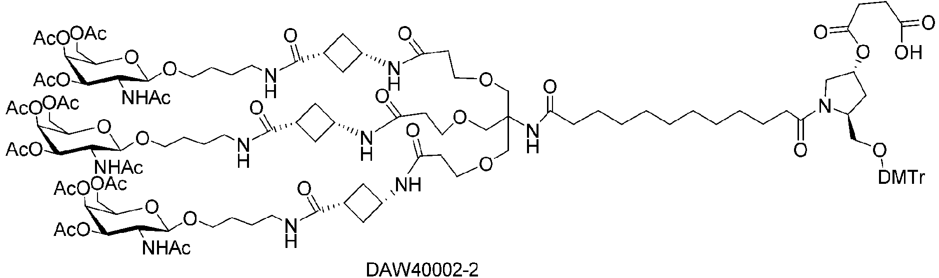 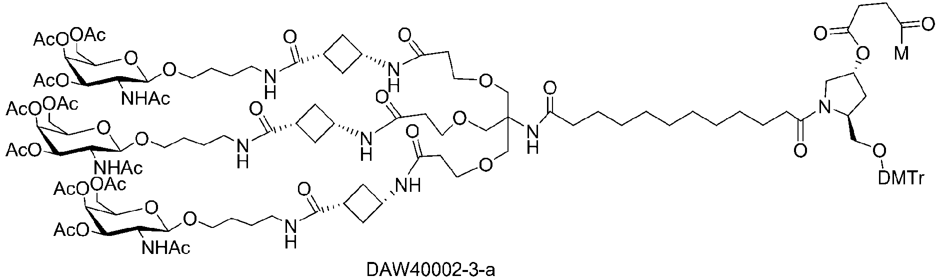 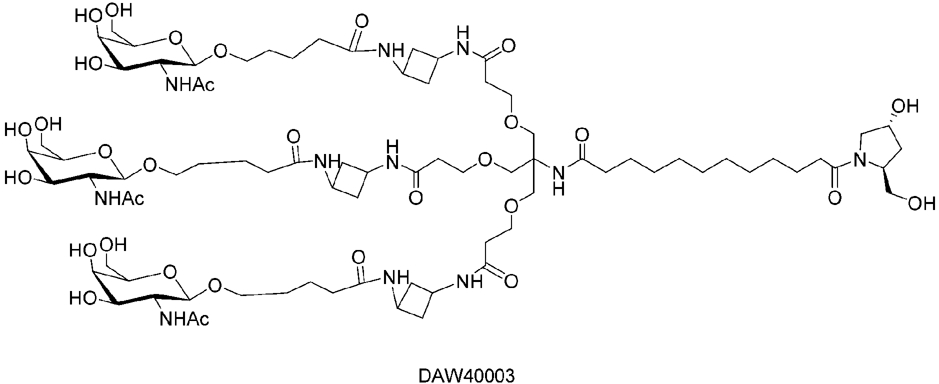 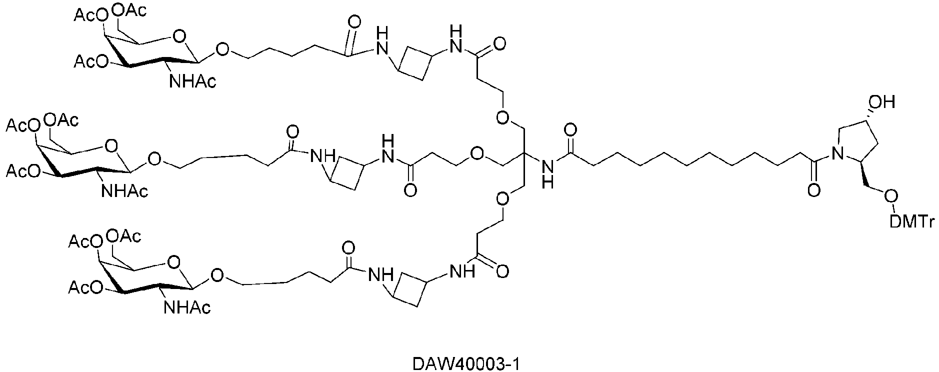 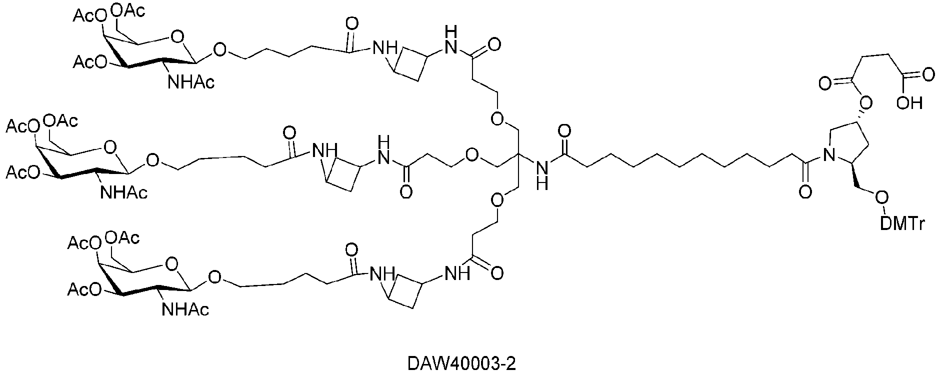 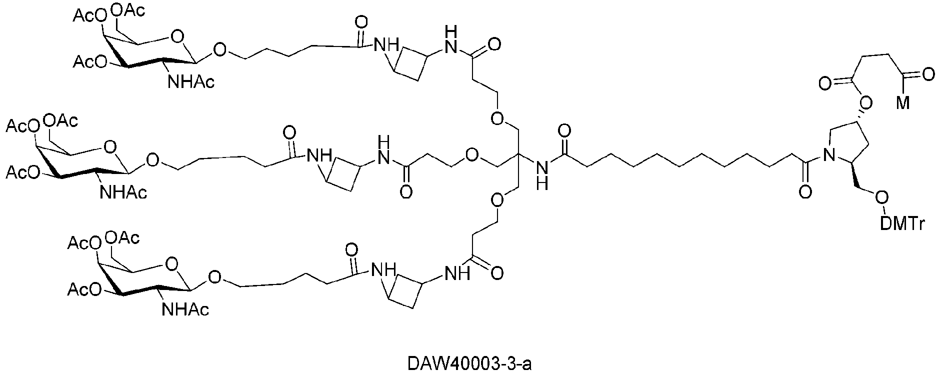 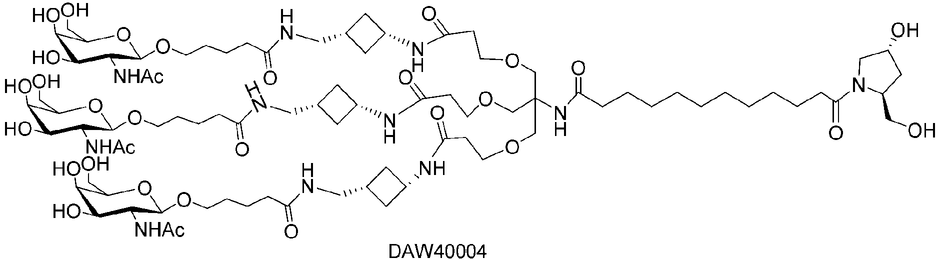 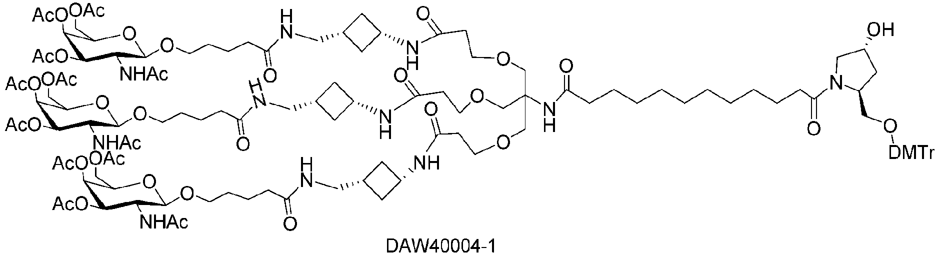 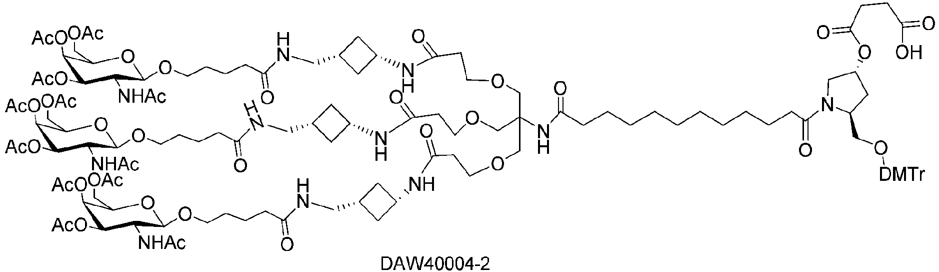 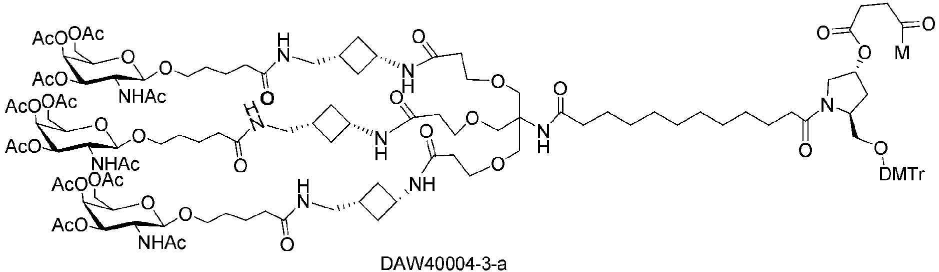 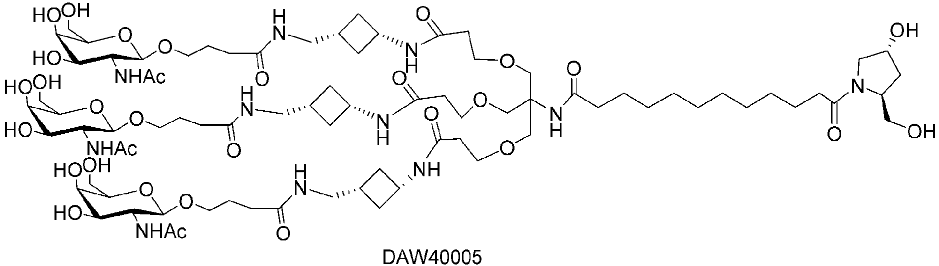 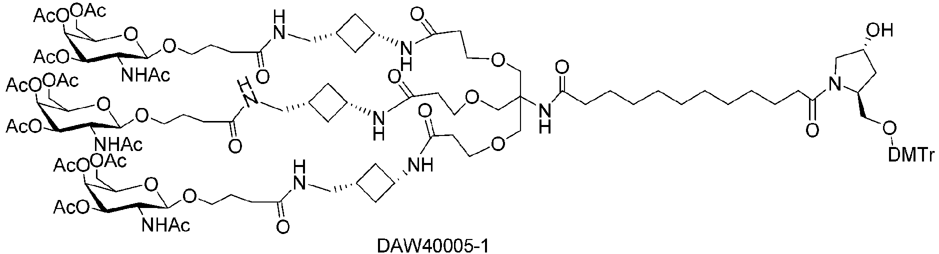 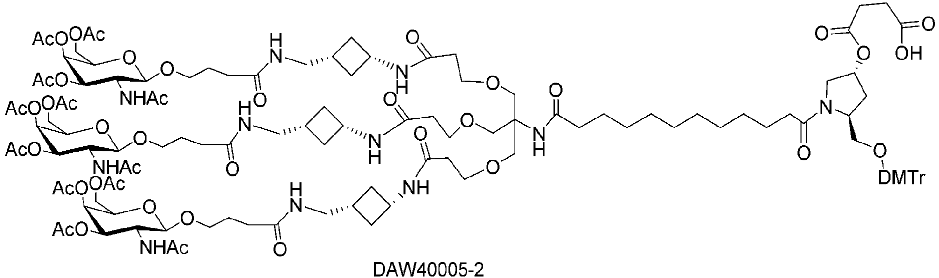 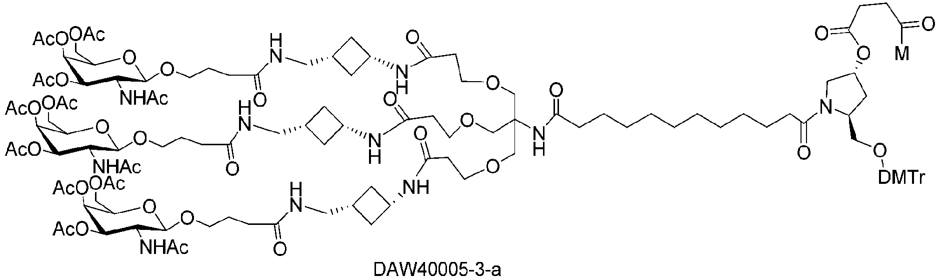 M is a solid support, preferably a hydroxyl- or amino-functionalized solid support, more preferably a resin or CPG, further preferably a macroporous resin or CPG, and even more preferably an aminomethyl resin, a hydroxy resin, or -NHCPG.

11. A conjugated group comprising the structure represented by Formula (I-a), or a stereoisomer, tautomer, or acceptable salt of the structure represented by Formula (I-a), 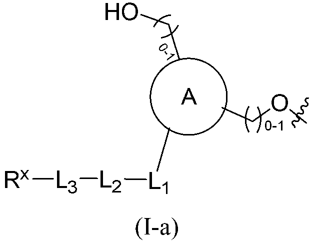 wherein, R^{x} is a structure represented by Formula (c) or Formula (d): 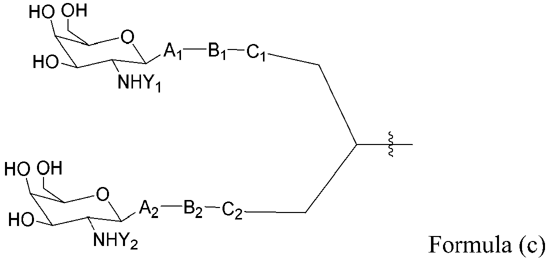 or 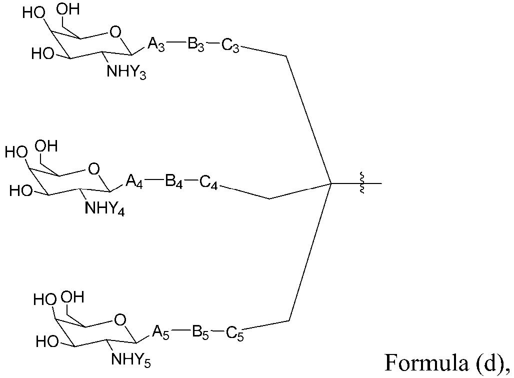 each of ring A, A₁, A₂, A₃, A₄, A₅, B₁, B₂, B₃, B₄, B₅, C₁, C₂, C₃, C₄, C₅, Y₁, Y₂, Y₃, Y₄, Y₅*,* L₁, L₂ and L₃ has the meaning as defined in any one of Claims 1 to 6.

12. The conjugated group according to claim 11, wherein R^{x} is a structure represented by the following Formula (c-1) or Formula (d-1): 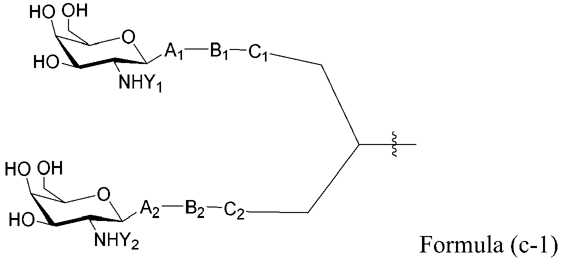 or 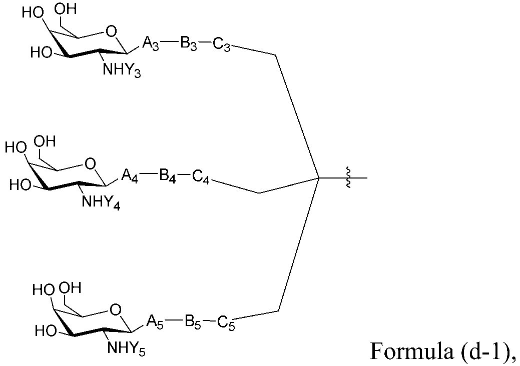 wherein, each of A₁, A₂, A₃, A₄, A₅, B₁, B₂, B₃, B₄, B₅, C₁, C₂, C₃, C₄, C₅, Y₁, Y₂, Y₃, Y₄ and Y₅ has the meaning as defined in any one of Claims 1 to 6.

13. The conjugated group according to claim 11 or 12, having the structure represented by Formula (II-a), or a stereoisomer, tautomer, or acceptable salt of the compound represented by Formula (II-a): 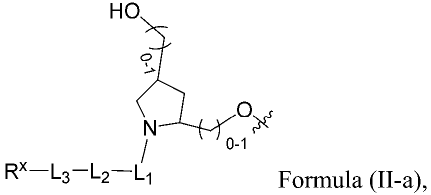 wherein, each of R^{x}, each of L₁, L₂ and L₃ has the meaning as defined in any one of Claims 1 to 6.

14. The conjugated group according to any one of claims 11 to 13, having the structure represented by Formula (III-a), or a stereoisomer, tautomer, or acceptable salt of the compound represented by Formula (III-a): 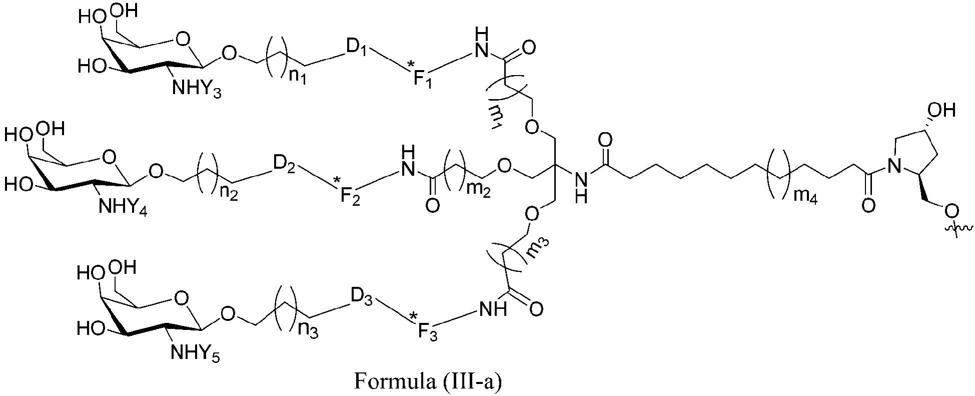 wherein, each of D₁, D₂ and D₃ is independently -C(=O)-*, -C(=O)O-*, -OC(=O)-*, -C(=O)N(R¹)-*, -N(R¹)C(=O)-*, -C(=O)N(R¹)CH₂-* or -N(R¹)C(=O)CH₂-*;
each of F₁, F₂ and F₃ is independently cyclopropyl, cyclobutyl, cyclopentyl or cyclohexyl;
each of n₁, n₂, n₃, m₁, m₂, m₃ and m₄ is independently 0, 1, 2, 3, or 4;
wherein, each of R¹, Y₃, Y₄ and Y₅ has the meaning as defined in any one of Claims 1 to 6.

15. The conjugated group according to any one of claims 11 to 14, which is one of the following substruchtures, or a stereoisomer, tautomer, or acceptable salt thereof,

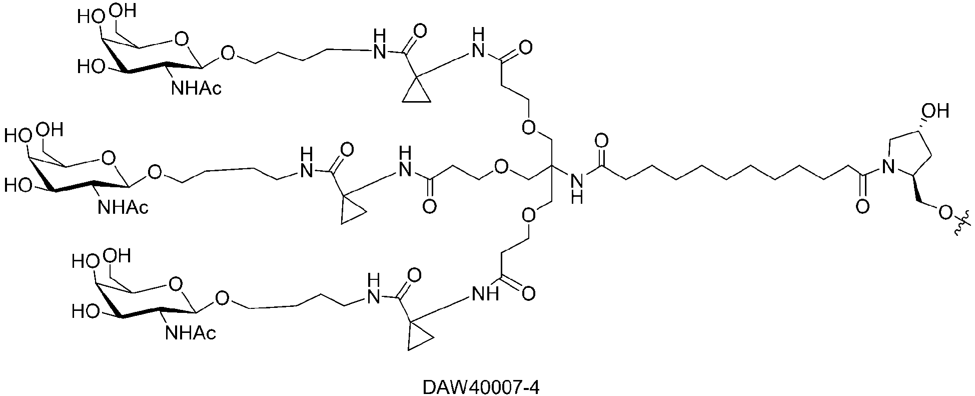

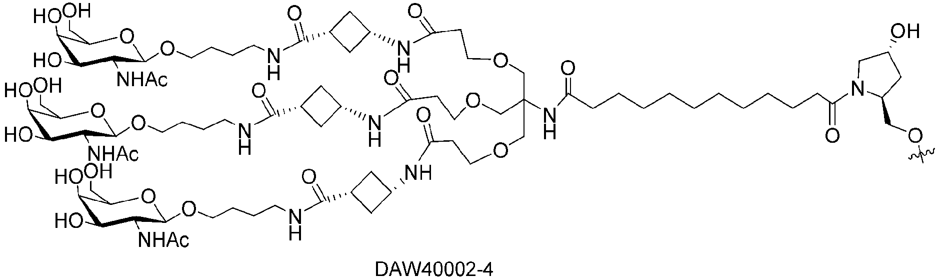

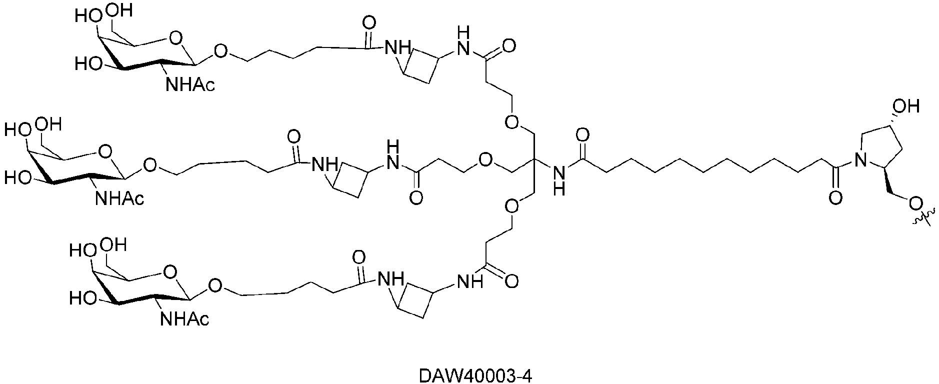

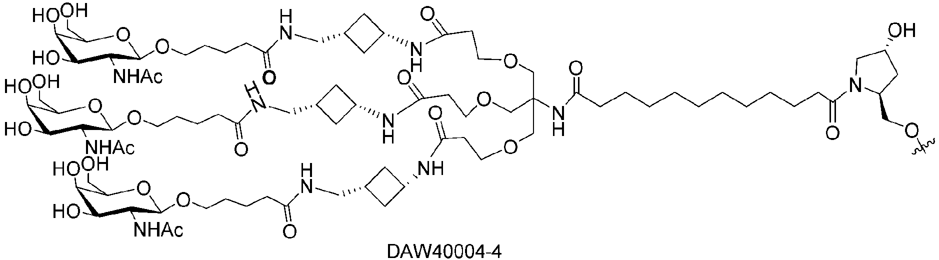

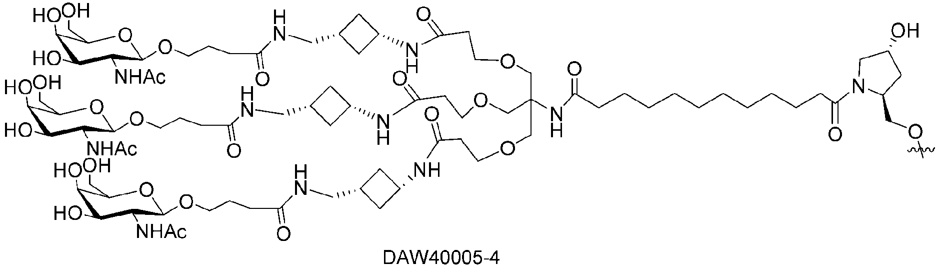

16. A nucleic acid conjugate comprising the conjugated group according to any one of claims 11 to 15 and a nucleic acid, wherein the nucleic acid is a modified or unmodified nucleic acid.

17. The nucleic acid conjugate according to claim 16, wherein the nucleic acid is linked to the conjugated group via a phosphate ester group, a phosphorothioate group or a phosphate group.

18. The nucleic acid conjugate according to claim 16 or 17, wherein the conjugated group is conjugated to the 3' end or the 5' end of the nucleic acid.

19. The nucleic acid conjugate according to any one of claims 16 to 18, wherein the nucleic acid is a single-stranded nucleic acid or a double-stranded nucleic acid, preferably siRNA; optionally, the conjugated group is preferably conjugated to the 3' end or 5' end of the sense strand of the siRNA, more preferably conjugated to the 3' end of the sense strand of the siRNA.

20. An siRNA conjugate, which is one of DAW30223, DAW30062, DAW30219, DAW30221, DAW30222 and DAW30255,
DAW30223:
DAW30223:
Sense Strand (5'-3'): gs-us-g-u-Gf-c-Af-Cf-Uf-u-c-g-c-u-u-c-a-c-a-DAW40002-4 (SEQ ID NO: 2)
Antisense Strand (5'-3'): us-Gfs-u-g-a-Agn-g-Cf-Gf-a-a-g-u-Gf-c-Af-c-a-cs-us-u (SEQ ID NO: 7)
DAW30062:
Sense Strand (5'-3'): gs-us-g-u-Gf-c-Af-Cf-Uf-u-c-g-c-u-u-c-a-c-a-DAW40003-4 (SEQ ID NO: 3)
Antisense Strand (5'-3'): us-Gfs-u-g-a-Agn-g-Cf-Gf-a-a-g-u-Gf-c-Af-c-a-cs-us-u (SEQ ID NO: 7)
DAW30219:
Sense Strand (5'-3'): gs-us-g-u-Gf-c-Af-Cf-Uf-u-c-g-c-u-u-c-a-c-a-DAW40004-4 (SEQ ID NO: 4)
Antisense Strand (5'-3'): us-Gfs-u-g-a-Agn-g-Cf-Gf-a-a-g-u-Gf-c-Af-c-a-cs-us-u (SEQ ID NO: 7)
DAW30221:
Sense Strand (5'-3'): gs-us-g-u-Gf-c-Af-Cf-Uf-u-c-g-c-u- u-c-a-c-a-DAW40005-4 (SEQ ID NO: 5)
Antisense Strand (5'-3'): us-Gfs-u-g-a-Agn-g-Cf-Gf-a-a-g-u-Gf-c-Af-c-a-cs-us-u (SEQ ID NO: 7)
DAW30222:
Sense Strand (5'-3'): gs-us-g-u-Gf-c-Af-Cf-Uf-u-c-g-c-u-u-c-a-c-a-DAW40007-4 (SEQ ID NO: 6)
Antisense Strand (5'-3'): us-Gfs-u-g-a-Agn-g-Cf-Gf-a-a-g-u-Gf-c-Af-c-a-cs-us-u (SEQ ID NO: 7)
DAW30255:
Sense Strand (5'-3'): gs-us-c-a-u-c-Cf-a-Cf-Af-Af-u-g-a-g-a-g-u-a-c-a-DAW40007-4 (SEQ ID NO: 8)
Antisense Strand (5'-3'): us-Gfs-u-a-c-(Tgn)-c-u-c-a-u-u-g-Uf-g-Gf-a-u-g-a-cs-gs-a (SEQ ID NO: 9)
wherein, the conjugate groups DAW40002-4, DAW40003-4, DAW40004-4, DAW40005-4 and DAW40007-4 are each conjugated to the 3' end of the siRNA sense strand through a phosphate ester group, and their structures are shown below:

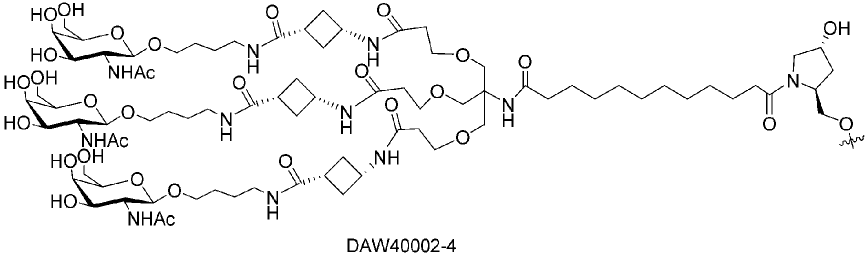

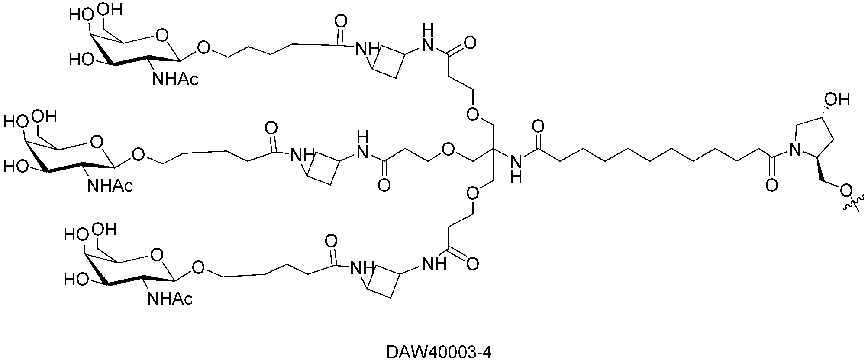

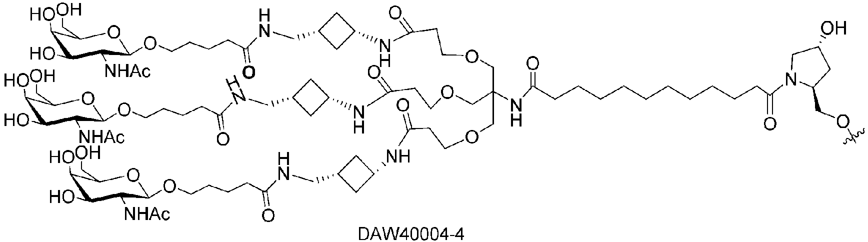

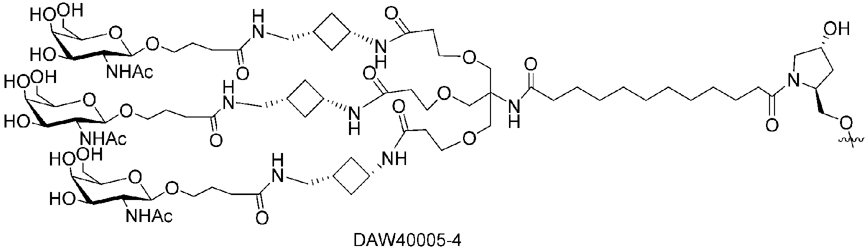

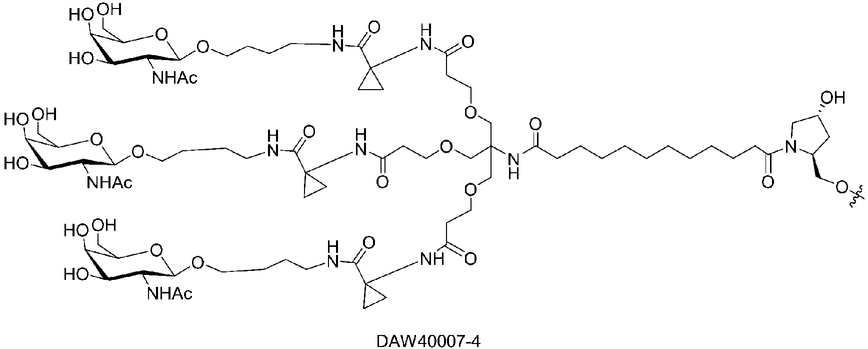

21. A pharmaceutical composition comprising the siRNA conjugate according to claim 20 and a pharmaceutically acceptable carrier.

22. Use of the compound of any one of claims 1 to 10, the conjugated group of any one of claims 11 to 15, or the nucleic acid conjugate of any one of claims 16 to 19 in the preparation of a medicament for treating and/or preventing a pathological condition or disease caused by the expression of a specific gene in hepatocytes.

23. The use according to claim 22, wherein the specific gene is selected from ApoB, ApoC3, AGT, ANGPTL3, PCSK9, LPA, SCD1, FVII, p53, HBV or HCV.

24. Use of DAW30223, DAW30062, DAW30219, DAW30221 and/or DAW30222, or pharmaceutical compositions thereof, in the preparation of a medicament for treating and/or preventing hepatitis B, or use of DAW30255 or a pharmaceutical composition thereof in the preparation of a medicament for treating and/or preventing hypertension.

25. The compound of any one of claims 1-10 or the conjugate group of any one of claims 11-15 for use in delivering a drug for treating and/or preventing a pathological condition or disease caused by the expression of a specific gene in hepatocytes, and the nucleic acid conjugate of claims 16-19 for use in treating and/or preventing a pathological condition or disease caused by the expression of a specific gene in hepatocytes.

26. The compound, conjugated group or nucleic acid conjugate according to claim 25, wherein the specific gene is selected from ApoB, ApoC3, AGT, ANGPTL3, PCSK9, LPA, SCD1, FVII, p53, HBV or HCV.

27. The siRNA drugs DAW30223, DAW30062, DAW30219, DAW30221 and/or DAW30222, or pharmaceutical compositions thereof for use in treating and/or preventing hepatitis B disease, or DAW30255 or a pharmaceutical composition thereof for use in treating and/or preventing hypertension.

28. A method for treating and/or preventing a pathological condition or disease caused by the expression of a specific gene in hepatocytes, the method comprising using the compound of any one of claims 1 to 10 or the conjugated group of any one of claims 11 to 15 as a drug delivery system, or the method comprising administering the nucleic acid conjugate of claims 16 to 19.

29. The method according to claim 28, wherein the specific gene is selected from ApoB, ApoC3, AGT, ANGPTL3, PCSK9, LPA, SCD1, FVII, p53, HBV or HCV.

30. A method for preventing, managing, treating or alleviating hepatitis B disease or hypertension, comprising administering to a patient an effective therapeutic amount of DAW30223, DAW30062, DAW30219, DAW30221 and/or DAW30222, or pharmaceutical compositions thereof; or administering to a patient an effective therapeutic amount of DAW30255 or a pharmaceutical composition thereof.
